# NEW EUROPEAN PATENT SPECIFICATION

(11) **EP 2 776 129 B2**
(45) Date of publication and mention of the opposition decision: **17.06.2020**
(45) Mention of the grant of the patent: 29.03.2017
(21) Application number: 12791926.4
(22) Date of filing: 12.11.2012
(51) Int. Cl.: A61P 5/20, A61K 38/08, A61P 13/12, A61P 19/00

(54) **COMPOSITIONS FOR USE IN THE TREATMENT OF CHRONIC KIDNEY DISEASE-MINERAL BONE DISORDER CHARACTERIZED BY SOFT TISSUE CALCIFICATION**
ZUSAMMENSETZUNGEN ZUR VERWENDUNG BEI DER BEHANDLUNG VON CHRONISCHE RENALE OSTEODYSTROPHIE GEKENNZEICHNET DURCH VERKALKUNG DER WEICHTEILE
COMPOSITIONS POUR LEUR UTILISATION DANS LE TRAITEMENT DES TROUBLES MINÉRAUX ET OSSEUX DE LA MALADIE RÉNALE CHRONIQUE CARACTÉRISÉE PAR UNE CALCIFICATION DES TISSUS MOUS

(30) Priority: 10.11.2011 US 201161558389 P
(43) Date of publication of application: 17.09.2014
(73) Proprietor: Kai Pharmaceuticals, Inc., South San Francisco, CA 94080 (US)
(72) Inventor: WALTER, Sarah, Redwood City, CA 94061 (US); BELL, Gregory, Tiburon, CA 94920 (US); TOMLINSON, James, E., Burlingame, CA 94010 (US)
(74) Representative: Dörries, Hans Ulrich
(86) International application number: PCT/US2012/064717
(87) International publication number: WO 2013/071262

(56) References cited:
- US-A1- 2011 028 394
- SVÁRA F.: "Chronic kidney disease-mineral and bone disorder (CKD-MBD): a new term for a complex approach.", J REN CARE, vol. 35, no. 1, March 2009 (2009-03), pages 3-6, XP002692460,
- MUHAMMAD ZIAD SOUQIYYEH, FAISSAL ABDULRAHEEM SHAHEEN: "Survey of attitudes of physicians toward the current evaluation and treatment of chronic kidney disease-mineral and bone disorder (CKD-MBD)", SAUDI JOURNAL FOR KIDNEY DISEASES AND TRANSPLANTATION, vol. 21, no. 1, 2010, pages 93-101, XP002692461,
- KATHERINE WESSELING-PERRY ET AL: "Phosphate binders, vitamin D and calcimimetics in the management of chronic kidney disease-mineral bone disorders (CKD-MBD) in children", PEDIATRIC NEPHROLOGY, 1 February 2013 (2013-02-01), XP055053936, ISSN: 0931-041X, DOI: 10.1007/s00467-012-2381-8
- BRIESE SONIA ET AL: "Arterial and cardiac disease in young adults with childhood-onset end-stage renal disease-impact of calcium and vitamin D therapy.", NEPHROLOGY, DIALYSIS, TRANSPLANTATION : OFFICIAL PUBLICATION OF THE EUROPEAN DIALYSIS AND TRANSPLANT ASSOCIATION - EUROPEAN RENAL ASSOCIATION JUL 2006, vol. 21, no. 7, July 2006 (2006-07), pages 1906-1914, ISSN: 0931-0509
- NOONAN WILLIAM ET AL: "Differential effects of vitamin D receptor activators on aortic calcification and pulse wave velocity in uraemic rats", NEPHROLOGY DIALYSIS TRANSPLANTATION, vol. 23, no. 12, December 2008 (2008-12), pages 3824-3830, ISSN: 0931-0509
- Klaus Olgaard, Ewa Lewin And Justin Silver: "Calcimimetics, vitamin D and ADVANCE in the management of CKD-MBD", NEPHROLOGY DIALYSIS TRANSPLANTATION., OXFORD UNIVERSITY PRESS., GB, vol. 26, no. 4, 1 April 2011 (2011-04-01), pages 1117-1119, XP055448194, GB ISSN: 0931-0509, DOI: 10.1093/ndt/gfq862
- P. Raggi, et al: "The ADVANCE study: a randomized study to evaluate the effects of cinacalcet plus low-dose vitamin D on vascular calcification in patients on hemodialysis", NEPHROLOGY DIALYSIS TRANSPLANTATION., OXFORD UNIVERSITY PRESS., GB, vol. 26, no. 4, 1 April 2011 (2011-04-01), pages 1327-1339, XP055448200, GB ISSN: 0931-0509, DOI: 10.1093/ndt/gfq725
- Takehisa Kawata, Nobuo Nagano, Masaki Obi, Sonoe Miyata, Chika Koyama, Nami Kobayashi, Sachiko Wakita, Michihito Wada: "Cinacalcet suppresses calcification of the aorta and heart in uremic rats", Kidney international, Nature Publishing Group, New York, vol. 74, no. 10, 2 November 2008 (2008-11-02), pages 1270-1277, XP055448187, New York ISSN: 1523-1755, DOI: 10.1038/ki.2008.407
- M J Aladren Regidor: "Cinacalcet reduces vascular and soft tissue calcification in secondary hyperparathyroidism (SHPT) in hemodialysis patients", CLINICAL NEPHROLOGY, DUSTRI VERLAG, NUENCHEN-DEISENHOFEN, DE, vol. 71, no. 2, 1 February 2009 (2009-02-01), pages 207-213, XP055448208, DE ISSN: 0301-0430, DOI: 10.5414/CNP71207
- "Press release regarding promising results of KAI-4169 for the treatment of CKD-MBD", , July 2011 (2011-07), Retrieved from the Internet: URL:https://www.businesswire.com/news/home /20110607006164/en/KAI-Pharmaceuticals-Ann ounces-Promising-Results-KAI-4169-Program
- Baruch et al -Abstract
- Poster by Baruch et al. presented at the ENDO2011 in Boston on June 5, 2011
- "Press release regarding a poster presentation of KAI-4169 Phase 2 clinical results at the American Soc. of Nephrology's Kidney Week 2011", , Retrieved from the Internet: URL:https://www.businesswire.com/news/home /20111111005115/en/KAI-Pharmaceuticals-Ann ounces-Late-Breaker-Poster-Presentation
- "LB-P03147", J Am Soc Nephrol, vol. 22, 2011,
- "FR-P01222", J Am Soc Nephrol, vol. 22, 2011,
- Poster P01222 submitted by the patentee in the US
- "SAO014", Nephrology Dialysis Transplantation, vol. 27, no. Supp 2, ii36-ii3, May 2012 (2012-05), DOI: 10.1093/ndt/gfs193

## Description

### CROSS-REFERENCE TO RELATED APPLICATIONS

This application claims the benefit of U.S. Provisional Application No. 61/558,389, filed November 10, 2011.

### REFERENCE TO SEQUENCE LISTING

A Sequence Listing is being submitted electronically via EFS in the form of a text file, created November 12, 2012, and named "632008022WO00.txt" (85,400 bytes).

### TECHNICAL FIELD

The present disclosure relates to calcimimetics, pharmaceutical compositions comprising CaSR agonists and methods for their use. The invention is defined in claims 1-3.

### BACKGROUND

Calcium homeostasis is the mechanism by which the body maintains adequate calcium levels. The process is highly regulated, and involves a complex interplay between calcium absorption, transport, storage in bones, deposition in other tissues, and excretion. PTH is a regulator of serum calcium levels, and functions to increase the concentration of calcium in the blood by enhancing the release of calcium from bone through the process of bone resorption; increasing reabsorption of calcium from the renal tubules; and enhancing calcium absorption in the intestine by increasing the production of 1,25-(OH)₂ vitamin D, the active form of vitamin D. PTH also stimulates phosphorus excretion from the kidney, and increases release from bone.

PTH secretion is regulated by the calcium sensing receptor (CaSR), a G-protein coupled receptor expressed on the cell surface of parathyroid cells, which detects small fluctuations in the concentration of extracellular calcium ion (Ca²⁺) and responds by altering the secretion of PTH. Activation of the CaSR by Ca²⁺ inhibits PTH secretion within seconds to minutes, and this process may be modulated by protein kinase C (PKC) phosphorylation of the receptor. The CaSR is also expressed on osteoblasts and in the kidney, where it regulates renal Ca²⁺excretion. In addition,

PTH regulates phosphorus homeostasis. PTH stimulates the parathyroid hormone receptor 1 (PTHR1) on both apical (brush border membrane) and basolateral membranes. PTHR1 stimulation leads to an increase in urinary excretion of phosphate (Pi) as a consequence of reduction by internalization of the renal Na+/phosphate (NaPi-IIa) co-transporter on the brush border membrane.

PTH is also involved in the regulation of osteoblasts and osteoclasts in bone. PTH increases serum Ca₂₊ by increasing bone resorption and renal absorption of calcium. PTH stimulates osteoblasts to produce RANK ligand (RANKL), which binds to the RANK receptor and activates the osteoclasts, leading to an increase in bone resorption and an increase in serum Ca₂₊. Osteoprotegerin (OPG) is a decoy receptor for RANKL which blocks bone resorption. Osteoporosis is caused by an imbalance between the processes of bone resorption by osteoclasts and bone formation by osteoblasts.

The human body contains approximately 1 kg of calcium, 99% of which resides in bone. Under normal conditions, circulating calcium ion (Ca²⁺) is tightly maintained at a level of about 8 to 10 mg/dL (i.e., 2.25-2.5 mmol/L; -600 mg). Approximately 1 g of elemental calcium (Ca²⁺) is ingested daily. Of this amount, approximately 200 mg/day is absorbed, and 800 mg/day is excreted. In addition, approximately 500 mg/day is released by bone resorption or is deposited into bone. About 10 g of Ca₂₊ is filtered through the kidney per day, with about 200 mg appearing in the urine, and the remainder being reabsorbed.

Hypercalcemia is an elevated calcium level in the blood. Acute hypercalcemia can result in gastrointestinal (anorexia, nausea, vomiting); renal (polyuria, polydipsia), neuro-muscular (depression, confusion, stupor, coma) and cardiac (bradycardia, first degree atrio-ventricular) symptoms. Chronic hypercalcemia is also associated with gastrointestinal (dyspepsia, constipation, pancreatitis); renal (nephrolithiasis, nephrocalcinosis), neuro-muscular (weakness) and cardiac (hypertension block, digitalis sensitivity) symptoms. Abnormal heart rhythms can result, and EKG findings of a short QT interval and a widened T wave suggest hypercalcemia. Hypercalcemia may be asymptomatic, with symptoms more commonly occurring at high calcium levels (12.0 mg/dL or 3 mmol/l). Severe hypercalcemia (above 15-16 mg/dL or 3.75-4 mmol/l) is considered a medical emergency: at these levels, coma and cardiac arrest can result.

Hypercalcemia is frequently caused by hyperparathyroidism, leading to excess bone resorption and elevated levels of serum calcium. In primary sporadic hyperparathyroidism, PTH is overproduced by a single parathyroid adenoma; less commonly, multiple adenomas or diffuse parathyroid gland hyperplasia may be causative. Increased PTH secretion leads to a net increase in bone resorption, with release of Ca₂₊ and phosphate (Pi). PTH also enhances renal reabsorption of Ca₂₊ and inhibits reabsorption of phosphate (Pi), resulting in a net increase in serum calcium and a decrease in phosphate.

Secondary hyperparathyroidism (SHPT) occurs when a decrease in the serum Ca₂₊ level stimulates PTH secretion. One cause of secondary hyperparathyroidism is chronic renal insufficiency (also referred to as chronic kidney disease or CKD), such as that in renal polycystic disease or chronic pyelonephritis, or chronic renal failure, such as that in hemodialysis patients (also referred to as end stage renal disease or ESRD). Excess PTH may be produced in response to hypocalcemia resulting from low calcium intake, GI disorders, renal insufficiency, vitamin D deficiency, and renal hypercalciuria. Tertiary hyperparathyroidism may occur after a long period of secondary hyperparathyroidism and hypercalcemia.

CKD is characterized by a progressive loss of renal function. The National Kidney Foundation's Kidney Disease Outcomes Quality Initiative (NKF KDOQI™) has defined CKD as either kidney damage or glomerular filtration rate (GFR) <60 mL/min/1.73 m² persisting for 3 months or more. Kidney damage may manifest as pathologic abnormalities or markers of kidney damage, including abnormalities in blood or urine tests or imaging studies.

Two of the most common causes of CKD are hypertension and diabetes mellitus (Type 1 and Type 2). Other causes of CKD include glomerulonephritis, pyelonephritis, and polycystic disease. In addition, the use of ibuprofen and acetaminophen over a long period of time can result in analgesic neuropathy, another cause of CKD.

The KDOQI guideline divides CKD patients into five stages based on the level of estimated GFR and the presence or absence of urinary protein. Stage 1 patients have kidney damage and normal or high GFR (≥90 mL/min/1.73 m²). Stage 2 patients have kidney damage with a mild decrease in their GFR (60-89 mL/min/1.73 m²). Stage 3 patients have moderately decreased GFR (30-59 mL/min/1.73 m²). Stage 4 patients have severely decreased GFR (15-29 mL/min/1.73 m²). Stage 5 patients have kidney failure (GFR ≤ 15 mL/min/1.73 m²).

Patients in Stage 1 or 2 typically do not have any symptoms that indicate the kidneys are damaged and their condition often goes undiagnosed. However, as kidney function continues to decline, excess amounts of urea and other nitrogenous waste products normally excreted by the kidney begin to build up in the blood leading to a condition referred to as uremia. Patients in Stage 3 are more likely to experience complications of kidney failure, such as hypertension, anemia and/or early bone loss. A patient in Stage 4 has advanced kidney damage and cardiovascular complications are more likely. Kidney failure, also referred to as endstage renal disease (ESRD), is reached in Stage 5 CKD and is followed by renal replacement therapy with the treatment options of dialysis or a kidney transplantation.

Chronic kidney disease-mineral and bone disorder (CKD-MBD) is a complex disorder associated with CKD and ESRD in which the impairment of blood and bone mineral homeostatis (calcium and phosphorus) and vitamin D metabolism [1,25(OH)₂D] lead to excessive PTH levels. These changes begin early in CKD in conjunction with the significant loss of renal function that occurs in Stage 3 and Stage 4 CKD patients, and gradually worsens as CKD progresses to ESRD. Elevated PTH levels further exacerbate the mineral imbalances (particularly calcium and phosphorus), and are linked to pathological effects in a variety of organ systems including osteodystrophy, vascular calcification, left ventricular hypertrophy and increased risk for cardiovascular events, which are the leading cause of morbidity and mortality in these patients (∼66% 5-year mortality).

Malignancy is a common cause of non-PTH mediated hypercalcemia. Hypercalcemia of malignancy, is an uncommon but severe complication of cancer, affecting between 10% and 20% of cancer patients, and may occur with both solid tumors and leukemia. The condition has an abrupt onset and has a very poor prognosis, with a median survival of only six weeks. Growth factors (GF) regulate the production of parathyroid hormone-related protein (PTHrP) in tumor cells. Tumor cells may be stimulated by autocrine GF to increase production of PTHrP, leading to enhanced bone resorption. Tumor cells metastatic to bone may also secrete PTHrP, which can resorb bone and release additional GF which in turn act in a paracrine manner to further enhance PTHrP production.

Cinacalcet HCl (Sensipar®), an orally-administered small molecule calcimimetic was approved in the United States and Europe for the treatment of SHPT in CKD patients on dialysis and for the treatment of hypercalcemia in patients with parathyroid carcinoma. Peptide calcimimetics also have been described (U.S. Patent Publication Nos. 2011/0028394 and 2009/0023652).

Described herein are methods for administering calcimimetics (sometimes referred to as CaSR agonists) to subjects in need thereof. In some embodiments, the calcimimetics are administered according to dosing regimes that provide stable and long-term efficacy in the reduction of serum calcium levels in patients in need thereof.

### BRIEF SUMMARY

In one aspect, the invention provides a composition comprising a therapeutically effective amount of the calcimimetic Ac-c(C)arrrar-NH₂ (SEQ ID NO:3) or a pharmaceutically acceptable salt thereof for use in the treatment of chronic kidney disease-mineral bone disorder (CKD-MBD) characterized by soft tissue calcification in a subject, wherein the therapeutically effective amount of the calcimimetic reduces the amount of soft tissue calcification in the subject.

The present disclosure provides a method for reducing hyperplasia of the parathyroid gland in a subject, comprising administering a therapeutically effective dose of a calcimimetic to the subject. Typically, the subject has a disease, disorder or condition characterized by elevated PTH levels or hypercalcemia. In some embodiments, the subject has secondary hyperparathyroidism. In other embodiments, the subject does not have secondary hyperparathyroidism. In some embodiments, the subject has CKD. In other embodiments of the disclosure, the subject does not have CKD. In the disclosure, the calcimimetic is a peptide. The calcimimetic is one that provides prolonged suppression of PTH.

The present disclosure provides a method for reducing soft tissue calcification in a subject, comprising administering a therapeutically effective dose of a calcimimetic to the subject. Typically, the subject has a disease, disorder or condition characterized by hypercalcemia. In some embodiments, the subject has secondary hyperparathyroidism. In other embodiments, the subject does not have secondary hyperparathyroidism. The subject has CKD characterized by soft tissue calcification. In other embodiments of the disclosure, the subject does not have CKD. In one embodiment, the vascular calcification is arterial wall calcification. In some embodiments the vascular calcification is aortic calcification. In some embodiments, the soft tissue calcification is renal parenchymal calcification or vascular calcification. In some embodiments of the disclosure, the calcimimeticisa peptide. The calcimimeticis one that provides prolonged suppression of PTH.

The present disclosure provides a method for slowing the decline in renal function in a subject, comprising administering a therapeutically effective dose of a calcimimetic to the subject. The present disclosure also provides a method for preserving or improving renal function in a subject, comprising administering a therapeutically effective dose of a calcimimetic to the subject. Typically, the subject has a disease, disorder or condition characterized by an elevated serum creatinine level. In some embodiments, the subject has secondary hyperparathyroidism. In other embodiments, the subject does not have secondary hyperparathyroidism. The subject has CKD characterized by soft tissue calcification. In the disclosure, the subject does not have CKD. The calcimimetic is a peptide of SEQ ID NO: 3. The calcimimetic is one that provides prolonged suppression of PTH.

The present disclosure provides a method for increasing or preserving parathyroid gland responsiveness to normal physiologic control in a subject, comprising administering a therapeutically effective dose of a calcimimetic to the subject. Typically, the subject has a disease, disorder or condition characterized by decreased parathyroid gland responsiveness. Parathyroid gland receptors are increased or preserved. The parathyroid gland receptor is CaSR. The parathyroid gland receptor is FGFR1. The parathyroid gland receptor is a Vitamin D receptor. In some embodiments, the subject has secondary hyperparathyroidism. In other embodiments, the subject does not have secondary hyperparathyroidism. The subject has CKD characterized by soft tissue calcification. In other embodiments of the disclosure, the subject does not have CKD. In the disclosure, the calcimimetic is a peptide. The calcimimetic is one that provides prolonged suppression of PTH.

The present disclosure provides a method for slowing progression of chronic kidney disease in a subject, comprising administering a therapeutically effective dose of a calcimimetic to the subject. The present disclosure provides a method for preserving or reversing the progression of chronic kidney disease in a subject, comprising administering a therapeutically effective dose of a calcimimetic to the subject. In some embodiments, the subject has secondary hyperparathyroidism. In other embodiments, the subject does not have secondary hyperparathyroidism. In the disclosure, the calcimimetic is a peptide. The calcimimetic is one that provides prolonged suppression of PTH.

In the disclosure, the method for providing prolonged PTH suppression in a subject is provided, comprising administering to the subject a therapeutically effective amount of a calcimimetic.

In the disclosure, PTH suppression continues more than 16 hours after the last dose of the calcimimetic. In another embodiment, the PTH suppression continues more than 48 hours after the last dose of the calcimimetic.

In the disclosure, the method comprises providing a first dose of the calcimimetic with activity to decrease PTH in a subject, the first dose of the calcimimetic effective, relative to serum PTH concentration prior to administration, and providing a subsequent dose of the calcimimetic 48 to 96 hours, 48 to 76 hours or 40 to 65 hours after providing the first dose. In another embodiment, the first dose is effective to decrease serum PTH concentration by at least about 40% within 30 minutes after administration. In still another embodiment, the first dose is effective to maintain a reduced serum PTH concentration for at least 40 hours after administration.

In the disclosure, the administering comprises administering parenterally. In another embodiment, the administering comprises administering every 3 days.

In the disclosure, the administering is effective to maintain a reduced serum PTH concentration for at least 72 hours after administration.

In the disclosure, the providing a subsequent dose comprises providing the subsequent dose administered parenterally.

In the disclosure, the providing a subsequent dose comprises providing a subsequent dose about 3 days after providing the first dose.

In the disclosure, the therapeutically effective dose of the calcimimetic is about 0.5-10 mg/kg, about 1-8 mg/kg, about 2-7 mg/kg, about 3-5 mg/kg or about 1-5 mg/kg. In another embodiment, the dose of the compound is about 0.5 mg/kg, 1.0 mg/kg, 2.0 mg/kg, 3.0 mg/kg, 4.0 mg/kg, 5.0 mg/kg, 6.0 mg/kg, 7.0 mg/kg, 8.0 mg/kg, 9.0 mg/kg or 10.0 mg/kg.

The present disclosure provides a dosing regimen for administration of a calcimimetic to a subject. The dosing regimen comprises providing a first dose of the calcimimetic and providing a subsequent dose of the calcimimetic 24 to 96 hours after providing the first dose. The subject has secondary hyperparathyroidism. The subject does not have secondary hyperparathyroidism. In the disclosure, the calcimimetic is a peptide. The calcimimetic is one that provides prolonged suppression of PTH.

In the disclosure, the first dose of the calcimimetic is effective to decrease PTH in a subject, wherein the decrease is relative to serum PTH concentration prior to administration of the first dose. In one embodiment, serum PTH concentration is decreased by at least about 25%, about 30%, about 35%, about 40%, about 45%, about 50%, or about 55% within about 20 minutes (min), 25 min, 30 min, 35 min, or 40 min after administration of the calcimimetic. In another embodiment, the first dose is effective to decrease serum PTH concentration by at about 20%-50%, 25%-50%, about 25%-40%, about 30%-50%, about 35%-50% or about 30%-45% within about 20 minutes (min), 25 min, 30 min, 35 min, or 40 min after administration of the calcimimetic.

In the disclosure, the first dose is an amount effective to maintain a reduced serum PTH concentration for at least about 24 h, 30 h, 35 h, 40 h, 45 h, 48 h, 50 h or 55 h after administration.

In disclosure, the first dose of the calcimimetic is about 0.5-10 mg/kg, about 1-8 mg/kg, about 2-7 mg/kg, about 3-5 mg/kg or about 1-5 mg/kg, In another embodiment, the dose of the compound is about 0.5 mg/kg, 1.0 mg/kg, 2.0 mg/kg, 3.0 mg/kg, 4.0 mg/kg, 5.0 mg/kg, 6.0 mg/kg, 7.0 mg/kg, 8.0 mg/kg, 9.0 mg/kg or 10.0 mg/kg,

The first dose of the calcimimetic may be administered parenterally, intravenously or transdermally.

The first dose of the calcimimetic may be administered prior to hemodialysis. The first dose may be administered about 60 min, about 30 min, about 15 min or about 5 min prior to hemodialysis. The first dose may be administered about 1-5 min, about 1-10 min, about 5-15 min, about 15-10 min or about 30-60 min prior to hemodialysis.

The first dose of the calcimimetic may be administered during hemodialysis.

The first dose of the calcimimetic may be administered after the completion of hemodialysis. In another embodiment, the first dose is administered about 60 min, about 30 min, about 15 min or about 5 min after the completion of hemodialysis. The first dose may be administered about 1-5 min, about 1-10 min, about 5-15 min, about 15-10 min, about 30-60 min, about 0.5-1 hour (h), about 1-2 h, about 1-3 h, about 2-3 h, or about 3-4 h after the completion of hemodialysis.

The subsequent dose of the calcimimetic about 24 h to 48 h, 24 h to 36 h, 36 h to 48 h, 36 h to 72 h, 38 h to 72 h, or 48 h to 96 h, after providing the first dose.

The subsequent dose may be administered every 2 days, 3 days, or 4 days.

The first dose and/or subsequent dose of the calcimimetic may be administered parenterally, intravenously or transdermally.

The treatment regimen may comprise administering the calcimimetic in combination with a second therapeutic agent.

The second therapeutic agent may be vitamin D, a vitamin D analog or cinacalcet hydrochloride.

The calcimimetic does not compete with cinacalcet for binding to the calcium sensing receptor.

The contiguous sequence of amino acid residues is a contiguous sequence of L-amino acid residues, a contiguous sequence of D-amino acid residues, a contiguous sequence of a mixture of L-amino acid residues and D-amino acid residues, or a mixture of amino acid residues and non-natural amino acid residues.

The contiguous sequence of amino acid residues may be linked to a compound to facilitate transport across a cell membrane. In another embodiment, the contiguous sequence of amino acid residues is linked to a compound that enhances delivery of the sequence into or across one or more layers of tissue.

The contiguous sequence of amino acid residues may be contained within a sequence of amino acid residues from 8-50 amino acid residues, 8-40 amino acid residues, 8-30 amino acid residues or 8-20 amino acid residues in length. The contiguous sequence of amino acid residues may be contained within a sequence of amino acid residues from 8-19 amino acid residues, 8-18 amino acid residues, 8-17 amino acid residues, 8-16 amino acid residues, 8-15 amino acid residues, 8-14 amino acid residues, 8-13 amino acid residues, 8-12 amino acid residues, 8-11 amino acid residues, 8-10 amino acid residues, or 8-9 amino acid residues in length.

The therapeutically effective dose of the calcimimetic may be about 0.5-10 mg/kg, about 1-8 mg/kg, about 2-7 mg/kg, about 3-5 mg/kg or about 1-5 mg/kg, In another embodiment, the dose of the compound is about 0.5 mg/kg, 1.0 mg/kg, 2.0 mg/kg, 3.0 mg/kg, 4.0 mg/kg, 5.0 mg/kg, 6.0 mg/kg, 7.0 mg/kg, 8.0 mg/kg, 9.0 mg/kg or 10.0 mg/kg.

The calcimimetic may be administered parenterally. The calcimimetic may be administered transdermally or subcutaneously.

### BRIEF DESCRIPTION OF THE DRAWINGS

FIG. 1 is a graph showing PTH level (% change from pre-dose baseline) at 6, 16 and 48 hours after the last dose in 5/6 Nx rats treated with Ac-c(C)arrrar-NH₂ (SEQ ID NO:3) at a dose of 1 mg/kg (IV), cinacalcet at a dose of 10 mg/kg (PO) or saline (IV).
FIGS. 2A-B are micrographs of tissue sections that have been stained using BrdU. The sections are of parathyroid gland tissue obtained from a 5/6 Nx rat treated with Ac-c(C)arrrar-NH₂ (SEQ ID NO:3) (FIG. 2A) and from an untreated control (FIG. 2B).
FIG. 2C is a graph providing quantitative results of the BrdU staining comparing the number of BrdU positive cells in the tissue sections obtained from the treated and untreated 5/6 Nx rats. FIG. 2D is a graph showing the normalized parathyroid gland weight from treated and untreated 5/6 Nx rats.
FIGS. 3A-B are micrographs of tissue sections that have been stained for calcium using the von Kossa method. The sections are of kidney tissue obtained from a 5/6 Nx rat treated with Ac-c(C)arrrar-NH₂ (SEQ ID NO:3) (FIG. 3A) and a control 5/6 Nx rat given vehicle (FIG 3B).
FIG 3C is a graph providing quantitative results of calcification of aorta and kidney sections obtain from a 5/6 Nx rats treated with Ac-c(C)arrrar-NH₂ (SEQ ID NO:3) (FIG. 2A) and from an untreated control as measured by atomic emission spectroscopy.
FIG. 4 is a graph showing percent change in serum creatinine (sCr) levels in 5/6 Nx rats treated with Ac-c(C)arrrar-NH₂ (SEQ ID NO:3) at a dose of 0.3, 1 or 3 mg/kg (SC) or vehicle.
FIG. 5A-C each include micrographs of tissue sections that have been stained for CaSR, FGFR1 and VDR, respectfully, as well as a graph providing quantitative results (% of total area). The sections are of parathyroid tissue obtained from normal rats and from 5/6 Nx rats treated with Ac-c(C)arrrar-NH₂ (SEQ ID NO:3) at a dose of 3 mg/kg (SC) or vehicle.
FIG. 6A is a schematic summarizing a dosing regimen. FIG. 6B is a graph showing plasma PTH level (pg/mL) after a 1-week washout period in 5/6 Nx rats treated with Ac-c(C)arrrar-NH₂ (SEQ ID NO:3) at a dose of 0.3, 1 and 3 mg/kg (SC) according to the dosing regimen summarized in FIG. 6A.
FIG. 7 is a graph showing PTH level (pg/mL) pre-dose, and after 2 and 4 weeks of treatment in rats with adenine-induced chronic renal failure treated with Ac-c(C)rrarar-NH₂ (SEQ ID NO:28) at a dose of 0.3 and 1 mg/kg (SC) or vehicle (SC) compared to that of normal rats (no adenine).
FIG. 8 is a graph showing SCr (mg/dL) after 4 weeks of treatment in rats with adenine-induced chronic renal failure treated with Ac-c(C)rrarar-NH₂ (SEQ ID NO:28) at a dose of 0.3 and 1 mg/kg (SC) or vehicle (SC) compared to that of normal rats (no adenine).
FIG. 9 is a graph showing serum phosphorus (P) (mg/dL) after 4 weeks of treatment in rats with adenine-induced chronic renal failure treated with Ac-c(C)rrarar-NH₂ (SEQ ID NO:28) at a dose of 0.3 and 1 mg/kg (SC) or vehicle (SC) compared to that of normal rats (no adenine).
FIG. 10 is a graph showing total Ca (mg/dL) after 4 weeks of treatment in rats with adenine-induced chronic renal failure treated with Ac-c(C)rrarar-NH₂ (SEQ ID NO:28) at a dose of 0.3 and 1 mg/kg (SC) or vehicle (SC) compared to that of normal rats (no adenine).
FIG. 11 is a graph showing the product of Ca and P after 4 weeks of treatment in rats with adenine-induced chronic renal failure treated with Ac-c(C)rrarar-NH₂ (SEQ ID NO:28) at a dose of 0.3 and 1 mg/kg (SC) or vehicle (SC) compared to that of normal rats (no adenine).
FIG. 12 is a graph showing normalized parathyroid gland weight (mg/kg body weight) after 4 weeks of treatment in rats with adenine-induced chronic renal failure treated with Ac-c(C)rrarar-NH₂ (SEQ ID NO:28) at a dose of 0.3 and 1 mg/kg (SC) or vehicle (SC) compared to that of normal rats (no adenine)PTG weight.
FIG. 13 is a graph showing the von Kossa score (aortic calcification) after 4 weeks of treatment in rats with adenine-induced chronic renal failure treated with Ac-c(C)rrarar-NH₂ (SEQ ID NO:28) at a dose of 0.3 and 1 mg/kg (SC) or vehicle (SC) compared to that of normal rats (no adenine). A von Kossa score of 0 indication no detected calcification; 1: 0-20% calcification; 2: 20-40% calcification; 3: 40-60% calcification; 4: 60-80% calcification; 5: >80% calcification.
FIG. 14 is a graph of Ac-c(C)arrrar-NH₂ (SEQ ID NO:3) levels (ng/ml) as a function of time (hours) in CKD-BMD subjects with SHPT receiving hemodialysis who received Ac-c(C)arrrar-NH₂(SEQ ID NO:3) as a single IV bolus (5, 10, 20, 40 or 60 mg).
FIG. 15 is a graph of percent change from baseline in serum iPTH as a function of time (hours) in CKD-BMD subjects with SHPT receiving hemodialysis who received Ac-c(C)arrrar-NH₂ (SEQ ID NO:3) as a single IV bolus (5, 10, 20, 40 or 60 mg) or placebo.
FIG. 16 is a graph of the corrected calcium (cCa) (mg/dL) as a function of time (hours) in CKD-BMD subjects with SHPT receiving hemodialysis who received Ac-c(C)arrrar-NH₂(SEQ ID NO:3) as a single IV bolus (5, 10, 20, 40 or 60 mg) or placebo.
FIG. 17 is a graph of serum iPTH, as percent of the baseline pre-dose value, as a function of time, wherein serum samples were taken immediately prior to the subject receiving their next 10 mg dose of Ac-c(C)arrrar-NH₂(SEQ ID NO:3) (during the "drug trough").
FIG. 18 is a graph of the mean percent change of iPTH, as percent of the baseline pre-dose value, through a four-week period of treatment with 10 mg of Ac-c(C)arrrar-NH₂ (SEQ ID NO:3).
FIG. 19 is a graph of the amount of serum iPTH in samples taken from subjects administered 10 mg Ac-c(C)arrrar-NH₂(SEQ ID NO:3) over a 4-week period followed by a 4-week follow-up period with no administration of Ac-c(C)arrrar-NH₂ (SEQ ID NO:3).

The present subject matter may be understood more readily by reference to the following detailed description of the preferred embodiments and the examples included herein.

### DETAILED DESCRIPTION

### I. Definitions

Within this disclosure unless otherwise stated, definitions of the terms and illustration of the techniques of this application may be found in any of several well-known references such as: Sambrook, J., et al., Molecular Cloning: A Laboratory Manual, Cold Spring Harbor Laboratory Press (1989); Goeddel, D., ed., Gene Expression Technology, Methods in Enzymology, 185, Academic Press, San Diego, CA (1991); "Guide to Protein Purification" in Deutshcer, M.P., ed., Methods in Enzymology, Academic Press, San Diego, CA (1989); Innis, et al., PCR Protocols: A Guide to Methods and Applications, Academic Press, San Diego, CA (1990); Freshney, R.I., Culture of Animal Cells: A Manual of Basic Technique, 2nd Ed., Alan Liss, Inc. New York, NY (1987); Murray, E.J., ed., Gene Transfer and Expression Protocols, pp. 109- 128, The Humana Press Inc., Clifton, NJ and Lewin, B., Genes VI, Oxford University Press, New York (1997).

As used herein, the singular form "a", "an", and "the" include plural references unless indicated otherwise. For example, "a" modulator peptide includes one of more modulator peptides.

As used herein a compound has "activity to decrease parathyroid hormone level" or "PTH-lowering activity" when the compound, upon administration to a subject, lowers plasma parathyroid hormone (PTH) relative to the plasma PTH concentration prior to administration of the compound. In one embodiment, the decrease in PTH level is at least 20%, 30%, 40%, 50%, 60%, 70%, 80%, 90% or which will inhibit, decrease or reverse development of a pathological condition. Treatment of diseases and disorders herein is intended to also include therapeutic administration of a compound of the disclosure (or a pharmaceutical salt, derivative or prodrug thereof) or a pharmaceutical composition containing said compound to a subject (*i.e.*, an animal, for example a mammal, such as a human) believed to be in need thereof. Treatment also encompasses administration of the compound or pharmaceutical composition to subjects not having been diagnosed as having a need thereof, *i.e.*, prophylactic administration to the subject. Generally, the subject is initially diagnosed by a licensed physician and/or authorized medical practitioner, and a regimen for prophylactic and/or therapeutic treatment via administration of the compound(s) or compositions of the disclosure is suggested, recommended or prescribed.

As used herein, the term "transdermal" means that in the methods of treatment described herein a therapeutically effective amount of a calcimimetic agent is applied to skin to deliver the compound to systemic circulation and thus achieve a desired therapeutic effect.

As used herein, "amino acid" refers to natural and non-natural amino acids. The twenty naturally occurring amino acids (L-isomers) are designated by the three letter code with the prefix "L-" (except for glycine which is achiral) or by the one letter code in upper-case: alanine ("L-Ala" or "A"), arginine ("L-Arg" or "R"), asparagine ("L-Asn" or "N"), aspartic acid ("L-Asp" or "D"), cysteine ("L-Cys" or "C"), glutamine ("L-Gln" or "Q"), glutamic acid ("L-Glu" or "E"), glycine ("Gly" or "G"), histidine ("L-His" or "H"), isoleucine ("L-Ile" or "I"), leucine ("L-Leu" or "L"), lysine ("L-Lys" or "K"), methionine ("L-Met" or "M"), phenylalanine ("L-Phe" or "F"), proline ("L-Pro" or "P"), serine ("L-Ser" or "S"), threonine ("L-Thr" or "T"), tryptophan ("L-Trp" or "W"), tyrosine ("L-Tyr" or "Y") and valine ("L-Val" or "V"). L-norleucine and L-norvaline may be represented as (NLeu) and (NVal), respectively. The nineteen naturally occurring amino acids that are chiral have a corresponding D-isomer which is designated by the three letter code with the prefix "D-" or by the lower-case one letter code: alanine ("D-Ala" or "a"), arginine ("D-Arg" or "r"), asparagine ("D-Asn" or "a"), aspartic acid ("D-Asp" or "d"), cysteine ("D-Cys" or "c"), glutamine ("D-Gln" or "q"), glutamic acid ("D-Glu" or "e"), histidine ("D-His" or "h"), isoleucine ("D-Ile" or "i"), leucine ("D-Leu" or "I"), lysine ("D-Lys" or "k"), methionine ("DMet" or "m"), phenylalanine ("D-Phe" or "f"), proline ("D-Pro" or "p"), serine ("D-Ser" or "s"), threonine ("D-Thr" or "t"), tryptophan ("D-Trp" or "w"), tyrosine ("D-Tyr" or "y") and valine ("D-Val" or "v"). D-norleucine and D-norvaline may be represented as (dNLeu) and (dNVal), respectively. Although "amino acid residue" is often used in reference to a monomeric subunit of a peptide, which will inhibit, decrease or reverse development of a pathological condition. Treatment of diseases and disorders herein is intended to also include therapeutic administration of a compound of the invention (or a pharmaceutical salt, derivative or prodrug thereof) or a pharmaceutical composition containing said compound to a subject (i.e., an animal, for example a mammal, such as a human) believed to be in need thereof. Treatment also encompasses administration of the compound or pharmaceutical composition to subjects not having been diagnosed as having a need thereof, i.e., prophylactic administration to the subject. Generally, the subject is initially diagnosed by a licensed physician and/or authorized medical practitioner, and a regimen for prophylactic and/or therapeutic treatment via administration of the compound(s) or compositions of the invention is suggested, recommended or prescribed.

As used herein, the term "transdermal" means that in the methods of treatment described herein a therapeutically effective amount of a calcimimetic agent is applied to skin to deliver the compound to systemic circulation and thus achieve a desired therapeutic effect.

As used herein, "amino acid" refers to natural and non-natural amino acids. The twenty naturally occurring amino acids (L-isomers) are designated by the three letter code with the prefix "L-" (except for glycine which is achiral) or by the one letter code in upper-case: alanine ("L-Ala" or "A"), arginine ("L-Arg" or "R"), asparagine ("L-Asn" or "N"), aspartic acid ("L-Asp" or "D"), cysteine ("L-Cys" or "C"), glutamine ("L-Gln" or "Q"), glutamic acid ("L-Glu" or "E"), glycine ("Gly" or "G"), histidine ("L-His" or "H"), isoleucine ("L-Ile" or "I"), leucine ("L-Leu" or "L"), lysine ("L-Lys" or "K"), methionine ("L-Met" or "M"), phenylalanine ("L-Phe" or "F"), proline ("L-Pro" or "P"), serine ("L-Ser" or "S"), threonine ("L-Thr" or "T"), tryptophan ("L-Trp" or "W"), tyrosine ("L-Tyr" or "Y") and valine ("L-Val" or "V"). L-norleucine and L-norvaline may be represented as (NLeu) and (NVal), respectively. The nineteen naturally occurring amino acids that are chiral have a corresponding D-isomer which is designated by the three letter code with the prefix "D-" or by the lower-case one letter code: alanine ("D-Ala" or "a"), arginine ("D-Arg" or "r"), asparagine ("D-Asn" or "a"), aspartic acid ("D-Asp" or "d"), cysteine ("D-Cys" or "c"), glutamine ("D-Gln" or "q"), glutamic acid ("D-Glu" or "e"), histidine ("D-His" or "h"), isoleucine ("D-Ile" or "i"), leucine ("D-Leu" or "I"), lysine ("D-Lys" or "k"), methionine ("DMet" or "m"), phenylalanine ("D-Phe" or "f"), proline ("D-Pro" or "p"), serine ("D-Ser" or "s"), threonine ("D-Thr" or "t"), tryptophan ("D-Trp" or "w"), tyrosine ("D-Tyr" or "y") and valine ("D-Val" or "v"). D-norleucine and D-norvaline may be represented as (dNLeu) and (dNVal), respectively. Although "amino acid residue" is often used in reference to a monomeric subunit of a peptide, polypeptide or protein, and "amino acid" is often used in reference to a free molecule, usage of these terms in the art overlaps and varies. The term "amino acid" and "amino acid residue" are used interchangeably and may refer to a free molecule or a monomeric subunit of a peptide, polypeptide or protein, depending on context.

To determine the percent "homology" or percent "identity" of two amino acid sequences, the sequences are aligned for optimal comparison purposes (e.g., gaps can be introduced in the sequence of one polypeptide for optimal alignment with the other polypeptide). The amino acid residues at corresponding amino acid positions are then compared. When a position in one sequence is occupied by the same amino acid residue as the corresponding position in the other sequence, then the molecules are identical at that position. As used herein amino acid or nucleic acid "homology" is equivalent to amino acid or nucleic acid "identity". Accordingly, the percent sequence identity between the two sequences is a function of the number of identical positions shared by the sequences (i.e., percent sequence identity = numbers of identical positions/total numbers of positions x 100). Percent sequence identity between two polypeptide sequences can be determined using the Vector NTI software package (Invitrogen Corporation, 5791 Van Allen Way, Carlsbad, CA 92008). A gap opening penalty of 10 and a gap extension penalty of 0.1 are used for determining the percent identity of two polypeptides. All other parameters are set at the default settings.

A "cationic amino acid" intends an amino acid residue that has a net positive charge at physiologic pH (7.4), as is the case, for example, in the amino acid residues where the side chain, or "R group", contains an amine functional group or other functional group that can accept a proton to become positively charged at physiologic pH, such as a guanidine or imidazole moiety. Cationic amino acid residues include arginine, lysine, histidine, 2,3-diaminopropionic acid (Dap), 2,4-diaminobutyric acid (Dab), ornithine, and homoarginine.

A "cationic subunit" intends a subunit that has a net positive charge at physiologic pH (7.4).

A "non-cationic amino acid" intends an amino acid residue that has no charge or a net negative charge at physiologic pH (7.4), as is the case, for example, in the amino acid residues where the side chain, or "R group", is neutral (neutral polar and neutral non-polar) and acidic. Non-cationic amino acids include those residues with an R group that is a hydrocarbon alkyl or aromatic moiety (e.g., valine, alanine, leucine, isoleucine, phenylalanine); a neutral, polar R group (asparagine, cysteine, glutamine, serine, threonine, tryptophan, tyrosine); or a neutral, non-polar R group (glycine, methionine, proline, valine, isoleucine). Non-cationic amino acids with an acidic R group include aspartic acid and glutamic acid.

As used herein, "conservative amino acid substitutions" are substitutions which do not result in a significant change in the activity or tertiary structure of a selected polypeptide or protein. Such substitutions typically involve replacing a selected amino acid residue with a different amino acid residue having similar physico-chemical properties. Groupings of amino acids and amino acid residues by physico-chemical properties are known to those of skill in the art. For example, among the naturally occurring amino acids, families of amino acid residues having similar side chains have been defined in the art, and include basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

A "subunit" intends a monomeric unit that is joined to more than one other monomeric unit to form a polymeric compound, where a subunit is the shortest repeating pattern of elements in the polymeric compound. Exemplary subunits are amino acids, which when linked form a polymer compound such as those referred to in the art as a peptide, a polypeptide or a protein.

As used herein, "chemical cross-linking" refers to covalent bonding of two or more molecules.

A peptide or peptide fragment is "derived from" a parent peptide or polypeptide if it has an amino acid sequence that is identical or homologous to at least a contiguous sequence of five amino acid residues, more preferably eight amino acid residues, of the parent peptide or polypeptide.

As used herein, the term "hyperparathyroidism" refers to primary, secondary and tertiary hyperparathyroidism, unless otherwise indicated.

The term "intradermal" intends that in the methods of treatment described herein a therapeutically effective amount of a calcimimetic compound is applied to skin to deliver the compound to layers of skin beneath the stratum corneum, and thus achieve a desired therapeutic effect.

As used herein, an "isolated" or "purified" polypeptide or biologically active portion thereof is free of some of the cellular material when produced by recombinant DNA techniques, or chemical precursors or other chemicals when chemically synthesized. The language "substantially free of cellular material" includes preparations of polypeptides in which the polypeptide is separated from some of the cellular components of the cells in which it is naturally or recombinantly produced. When the polypeptide or biologically active portion thereof is recombinantly produced, it is also preferably substantially free of culture medium, i.e., culture medium represents less than about 20%, more preferably less than about 10%, and most preferably less than about 5% of the volume of the polypeptide preparation. The language "substantially free of chemical precursors or other chemicals" includes preparations of polypeptides in which the polypeptide is separated from chemical precursors or other chemicals that are involved in the synthesis of the polypeptide. In one embodiment, the language "substantially free of chemical precursors or other chemicals" includes preparations of a polypeptide having less than about 30% (by dry weight) of chemical precursors or other chemicals, preferably less than about 20% chemical precursors or other chemicals, more preferably less than about 15% chemical precursors or other chemicals, still more preferably less than about 10% chemical precursors or other chemicals, and most preferably less than about 5% chemical precursors or other chemicals. In preferred embodiments, isolated polypeptides, or biologically active portions thereof, lack contaminating polypeptides from the same organism from which the domain polypeptide is derived.

As used herein, "macromolecule" refers to a molecule, such as a peptide, polypeptide, protein or nucleic acid, that typically has a molecular weight greater than about 900 Daltons.

A "polymer" refers to a linear chain of two or more identical or non-identical subunits joined by covalent bonds.

As used herein, "peptide" and "polypeptide" refer to any polymer made up of a chain of amino acid residues linked by peptide bonds, regardless of its size. Although "protein" is often used in reference to relatively large polypeptides, and "peptide" is often used in reference to small polypeptides, usage of these terms in the art overlaps and varies. Thus, for simplicity, the term "peptide" will be used herein, although in some cases the art may refer to the same polymer as a "polypeptide." Unless otherwise indicated, the sequence for a peptide is given in the order from the amino terminus to the carboxyl terminus.

A "thiol-containing group" or "thiol-containing moiety" as used herein intends a functional group comprising a sulfur-hydrogen bond (-SH), and that is capable of reacting with another thiol under physiologic conditions to form a disulfide bond. A thiol that is capable of forming a disulfide bond with another thiol is referred to herein as a "reactive thiol." In a preferred embodiment the thiol-containing group is less than 6 atoms away from the backbone of the compound. In a more preferred embodiment, the thiol-containing group has the structure (-SH-CH₂-CH₂-C(O)-O-)-.

As used herein, "small molecule" refers to a molecule other than a macromolecule, such as an organic molecule, and typically has a molecular weight of less than 1000 Daltons.

### II. Methods for Therapeutic Use of Calcimimetics

In one aspect of the disclosure, calcimimetics as described herein are administered to subjects in need thereof to treat and/or ameliorate symptoms associated with hypercalcemia. Primary hyperparathyroidism and malignancy account for about 90% of cases of hypercalcemia. Other diseases associated with hypercalcemia include but are not limited to abnormal parathyroid gland function, primary hyperparathyroidism, solitary parathyroid adenoma, primary parathyroid hyperplasia, parathyroid carcinoma, multiple endocrine neoplasia (MEN), familial isolated hyperparathyroidism, familial hypocalciuric hypercalcaemia/familial benign hypercalcaemia, malignancy, solid tumor with metastasis (e.g. breast cancer or classically squamous cell carcinoma, which can be PTHrPmediated), solid tumor with humoral mediation of hypercalcaemia (e.g. lung cancer (most commonly non-small cell lung cancer), or kidney cancer, phaeochromocytoma), haematologic malignancy (multiple myeloma, lymphoma, leukaemia), sarcoidosis and other granulomatous diseases, vitamin-D metabolic disorders, hypervitaminosis D (vitamin D intoxication), elevated 1,25(OH)2D levels, idiopathic hypercalcaemia of infancy, rebound hypercalcaemia after rhabdomyolysis, disorders related to high bone turnover rates, hyperthyroidism, Paget's disease of the bone, renal failure, severe secondary hyperparathyroidism, milk-alkali syndrome. Hypercalcemia can also result from lithium use, thiazide use, vitamin A intoxication and aluminium intoxication, as well as prolonged immobilization.

In another aspect of the disclosure, calcimimetics as described herein are used to inhibit, decrease or reduce progression of kidney damage, vascular calcification, or parathyroid hyperplasia in a subject in need thereof.

In one embodiment, the calcimimetic is administered as an intravenous (IV) product to be administered three-times weekly for the treatment of CKD-MBD in endstage renal disease (ESRD) patients receiving hemodialysis. In another embodiment, the calcimimetic is administered via a daily transdermal patch (e.g., for a subject classified as having Stage 3 or Stage 4 CKD).

In one aspect of the disclosure, calcimimetics as described herein are administered to subjects at risk of, or diagnosed with, early stage chronic kidney disease (CKD) and/or chronic kidney disease-mineral bone disorder (CKD-MBD). The subjects may be undergoing dialysis, or the subject may be pre-dialysis. In some embodiments, the subject may be classified as having Stage 1, Stage 2, Stage 3, Stage 4 or Stage 5 CKD.

In another aspect of the disclosure, the compositions described herein are used to treat a subject exhibiting, or at risk of developing, soft tissue calcification, a well-recognized and common complication of chronic kidney disease. Such an individual can have, or be at risk of developing, for example, vascular calcification associated with conditions such as atherosclerosis, stenosis, restenosis, renal failure, diabetes, prosthesis implantation, tissue injury or age-related vascular disease. In another aspect of the disclosure, the compositions described herein are used to treat a subject exhibiting, or at risk of developing, elevated serum creatinine levels. In another aspect of the disclosure, the compositions described herein are used to treat a subject exhibiting, or at risk of developing, parathyroid gland hypertrophy. In another aspect of the disclosure, the compositions described herein are used to treat a subject exhibiting, or at risk of developing, elevated phosphorus levels. In another aspect of the disclosure, the compositions described herein are used to treat a subject exhibiting, or at risk of developing, reduced PTH receptor expression. The prognostic and clinical indications of these conditions are known in the art. An individual treated by a method of the disclosure can have a systemic mineral imbalance associated with, for example, diabetes, chronic kidney disease, renal failure, kidney transplantation or kidney dialysis.

### III. Therapeutic Efficacy of the Calcimimetics as Described Herein

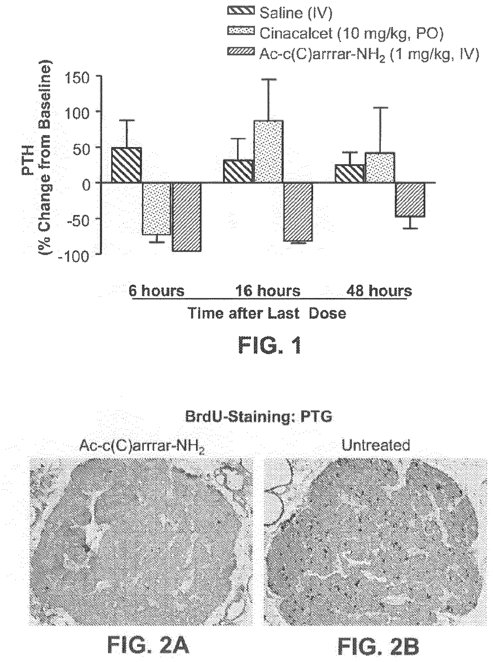

Use of calcimimetics to reduce PTH was assessed using a rat model of CKD, the 5/6 nephrectomy (5/6 Nx; Charles River Laboratories, Wilmingham, MA). The animals were treated with Ac-c(C)arrrar-NH₂ (SEQ ID NO:3), (1 mg/kg, IV) or with cinacalcet (10 mg/kg, PO). The animals were dosed daily for 28 days. Serum PTH levels were measured 6 hours, 16 hours and 48 hours after the last dosing. Animals treated with Ac-c(C)arrrar-NH₂ (SEQ ID NO:3)had prolonged reductions in PTH following chronic daily dosing (see Example 1A, FIG. 1). Animals treated with cinacalcet showed an increase in serum PTH at 16 hours and 48 hours after dosing, whereas animals treated with Ac-c(C)arrrar-NH₂ (SEQ ID NO:3)showed a decrease greater than 50% lower than baseline at 6 hours and 16 hours after the last dosing and showed a decrease between 25% and 50% lower than baseline PTH 48 hours after dosing. These results support the conclusion that calcimimetics effectively may be used as therapeutic agents for diseases and conditions associated with increased PTH, including without limitation CKD, secondary hyperparathyroidism and primary parathyroidism.

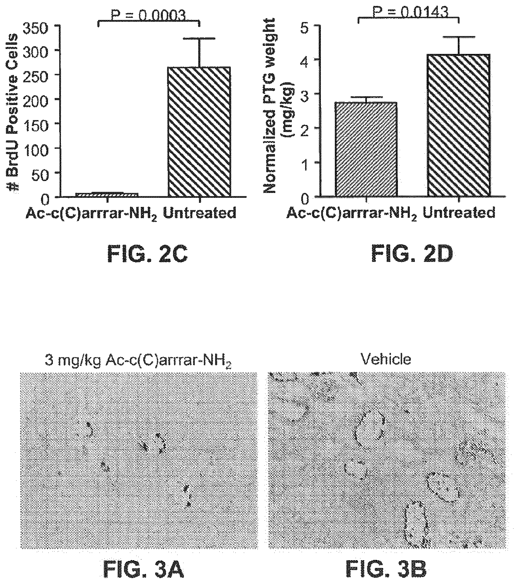

The 5/6 Nx rat model was used in another study to determine the effects of a calcimimetic on parathyroid gland proliferation. Animals treated with Ac-c(C)arrrar-NH₂ (SEQ ID NO:3) (3 mg/kg, SC) had a dramatic reduction in parathyroid gland hyperplasia (Example 1 B, FIGS. 2A-D). These results support the conclusion that treatment with a calcimimetic is effective in reducing parathyroid gland proliferation.

Another study done using the 5/6 Nx rat model to examine the effect of Ac-c(C)arrrar-NH₂ (SEQ ID NO:3) on ectopic calcification (i.e., inappropriate biomineralization occurring in soft tissues). CKD patients have a high incidence of kidney calcification. Also, patients with CKD on dialysis have a 2- to 5-fold more coronary artery calcification than age-matched individuals with angiographically proven coronary artery disease. Calcification is correlated with atherosclerotic plaque burden and increased risk of myocardial infarction, increased ischemic episodes in peripheral vascular disease and increased risk of dissection following angioplasty.

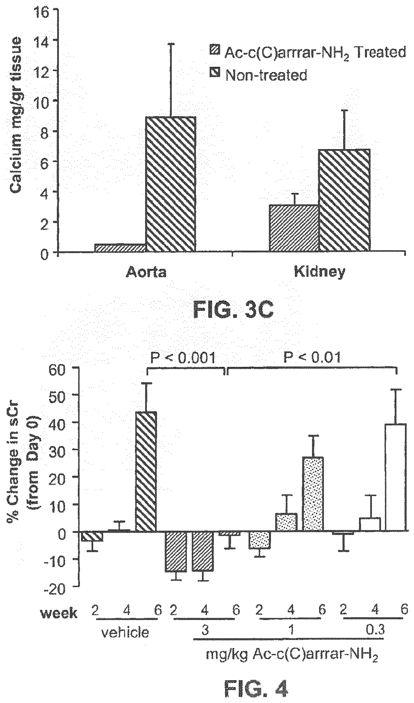

The effects of calcimimetic administration on ectopic calcification were studied by administering Ac-c(C)arrrar-NH₂ (SEQ ID NO:3)to 5/6 Nx rats (Example 1C). Animals treated for 6 weeks with 3 mg/kg Ac-c(C)arrrar-NH₂ (SEQ ID NO:3) were found to have reduced kidney calcification as compared to untreated 5/6 Nx rats (FIGS. 3A-B). 5/6 Nx rats treated for 6 weeks with 3 mg/kg Ac-c(C)arrrar-NH₂ (SEQ ID NO:3) had less calcification of aortic and kidney tissue than did the untreated counterparts (FIG. 3C). The results lead to the conclusion that administration of a calcimimetic is effective in reducing soft tissue calcification.

Measurement of serum creatinine (sCr) provides a means for assessing the progression of kidney failure, and whether or not a pharmaceutical agent is able slow its progression. Higher levels of creatinine indicate a falling glomerular filtration rate and a resultant decreased capability of the kidneys to excrete waste products. Serum creatinine levels were monitored in 5/6 Nx rats who had been treated with 0.3 mg/kg, 1 mg/kg, 3 mg/kg and a vehicle control over a six week period of 3 times weekly dosing (Example 1C). Animals treated with 3 mg/kg Ac-c(C)arrrar-NH₂ (SEQ ID NO:3) experienced a decrease in creatinine levels in serum while animals treated with 0.3 mg/kg and 1 mg/kg had decreased levels of serum creatinine in week 2 and an increase in creatinine levels which was less than that observed in the vehicle-treated animals. The data show that elevation of serum creatinine is suppressed in a dose-dependent manner over several weeks and supports the conclusion that administration of a calcimimetic was effective in slowing progression of the kidney failure.

Use of calcimimetics to reduce PTH also was assessed using Ac-c(C)rrarar-NH₂ (SEQ ID NO:28) in an adenine-induced rat model of chronic renal failure. The results, were consistent with those described above (see Example 2)

A clinical trial was designed to study the effects of Ac-c(C)arrrar-NH₂(SEQ ID NO:3) in subjects receiving hemodialysis three-times weekly. The trial was a double-blind, randomize placebo-controlled, multicenter study. The results, described in Example 3, show that a calcimimetic, as described herein, can decrease PTH secretion and synthesis, simultaneously lowering serum PTH, phosphorous and calcium, thereby improving all three major biochemical abnormalities of CKD-MBD.

A study of the pharmacokinetic profile of Ac-c(C)arrrar-NH₂ (SEQ ID NO:3) shows the calcimimetic to have a relatively low clearance rate of approximately 2 L/h, and the total plasma exposure to the peptide was proportional to the administered dose (Example 3A). These data show a prolonged effect of the calcimimetic, supporting dosing of the hemodialysis patients at a frequency of 2-3 times per week, or possibly 2-4 times per week. In a preferred embodiment, the calcimimetic is Ac-c(C)arrrar-NH₂ (SEQ ID NO:3).

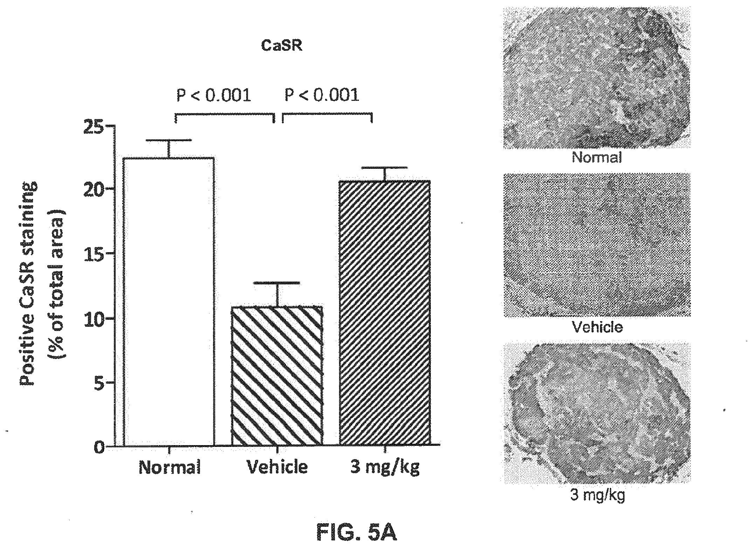

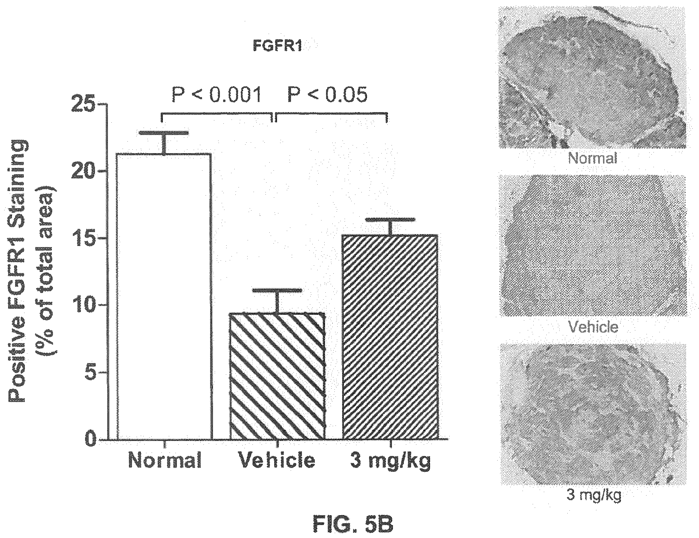

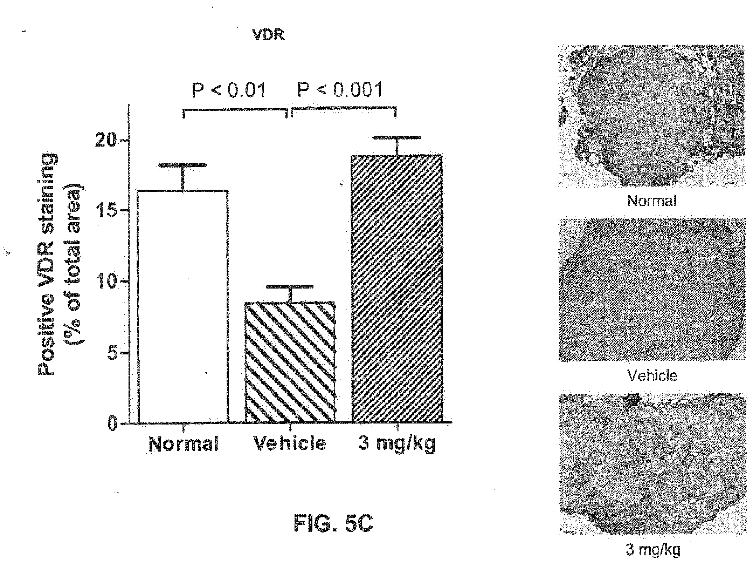

The efficacy of the calcimimetic to reduce serum iPTH and calcium levels was also observed in this trial. The subjects were administered a single IV bolus of Ac-c(C)arrrar-NH₂ (SEQ ID NO:3)2-4 hours after completion of hemodialysis and blood from the subjects was analyzed for changes in serum iPTH and corrected calcium levels (Example 2B). The doses administered were 5 mg, 10 mg, 20 mg, 40 mg or 60 mg. As shown in FIG. 5, there was a dose-dependent decrease in iPTH which occurred within 30 minutes of dosing. The data support administration of a calcimimetic as described herein to a hemodialysis patient, as the iPTH suppression effected by the peptide was sustained through the interdialytic period at doses greater than or equal to about 20 mg, or at doses ranging from about 20 mg to about 100 mg. Such doses may provide a decrease in serum iPTH within about 5 min - 45 min after dosing, or about 10 min - 30 min after dosing, or with about an hour after dosing, for example, with an IV bolus, or a peritoneal injection.

The administered calcimimetic was also effective in reducing serum corrected calcium levels as described in Example 2B. The doses of Ac-c(C)arrrar-NH₂ (SEQ ID NO:3), ranging from about 5 mg to about 60 mg resulted in a reduction in serum corrected calcium of about 10% to 14%. No subject had a corrected calcium level below 7.5 mg/dL at any time during the study.

Accordingly, a calcimimetic which modulates the CaSR activity is effective in treating subjects suffering from excessive levels of PTH or calcium in the blood or in reversing the effects of a loss of calcium homeostasis in such patients, for example, patients diagnosed with hypercalcemia.

The clinical study further provided safety data which showed that administration of the calcimimetic was very well-tolerated, with no reports of diarrhea, nausea or vomiting (Example 2C). Thus, administration of a therapeutically effective amount of a calcimimetic as described herein may cause fewer side effects or less frequent adverse events as compared to the number and frequency of those associated with a therapeutically effective amount of an oral calcimimetic.

The calcimimetics as described herein have a disease modifying effect such that the therapeutic effects of the calcimimetic lasts for several weeks after drug treatment is stopped.

The calcimimetic is effective in preventing the loss of the key receptors in the parathyroid gland, including the CaSR, the vitamin D receptor and the FGF-23 co-receptor (Example 1 E). After administration of Ac-c(C)arrrar-NH₂ (SEQ ID NO:3) to 5/6 Nx rats for 6 weeks at a dose of 3 mg/kg, the parathyroid glands were sectioned and stained to observe the expression levels of the CaSR, the vitamin D receptor and the FGF-23 co-receptor. In each case, administration of Ac-c(C)arrrar-NH₂ (SEQ ID NO:3) resulted in an increased expression of the receptor relative to the expression seen in glands from rats treated with a vehicle control. These data support the conclusion that treatment with a calcimimetic, as described herein, can improve parathyroid gland responsiveness by maintaining and/or increasing expression of one or more receptors of the parathyroid gland, and thereby potentially modify the course of the disease.

In a further investigation, 5/6 Nx rats were treated with 4 doses of 3 mg/kg Ac-c(C)arrrar-NH₂ (SEQ ID NO:3)over a one-week period, followed by a 1 week washout period (drug holiday) (Example 3B). The treated rats experienced a 50% reduction in baseline PTH as measured after the one-week washout period and as compared to baseline PTH prior to administration of the first dose of Ac-c(C)arrrar-NH₂ (SEQ ID NO:3). Administration of a therapeutically effective amount of the calcimimetic results in a 25% to 75% reduction, or a 40% to 50% reduction in serum PTH in a subject undergoing hemodialysis as compared to serum PTH prior to a first dose of the calcimimetic, and wherein the reduction is measured 1 to 7 days, 3 to 8 days or 5 to 10 days after administration of the last dose of the calcimimetic.

The disease modifying effects described above were also observed in a clinical trial in which hemodialysis patients were treated with 10 mg Ac-c(C)arrrar-NH₂ (SEQ ID NO:3), 3 times per week, for 4 weeks. Serum PTH levels were measured at the drug trough which occurs immediately before the patient receives their next dose of Ac-c(C)arrrar-NH₂ (SEQ ID NO:3). As discussed in Example 4, patients treated with Ac-c(C)arrrar-NH₂ (SEQ ID NO:3)experienced about a 50% reduction in baseline PTH as measured on Day 27 as compared to the baseline PTH measured prior to the first dose of Ac-c(C)arrrar-NH₂ (SEQ ID NO:3). The data further support that Ac-c(C)arrrar-NH₂ (SEQ ID NO:3) has a disease modifying effect in the hemodialysis patients.

A subject suffering from kidney damage, vascular calcification, parathyroid hyperplasia, hypercalcemia and/or hyperparathyroidism may be treated using the described calcimimetics.

Untreated SHPT patients with moderately severe hyperparathyroidism often have baseline circulating intact PTH levels >300 pg/ml, and levels that can exceed 600 pg/mL. In a preferred embodiment, the decrease in PTH levels is measured as a decrease in intact PTH below pretreatment baseline levels. The desired decrease in PTH is to bring the plasma PTH levels into generally recognized guidelines established by the National Kidney Foundation or other experts in the treatment of kidney disorders and renal insufficiency.

In another aspect of the disclosure, means for treating hyperparathyroidism, hypercalcemia and/or bone disease are provided, comprising administering a therapeutically effective amount of a described compound. In another embodiment, the subject can be treated with a described compound in combination with one or more other therapeutically effective agents.

The described compound may be administered in an amount effective to reduce PTH or PTH effect. In some embodiments, the reduction in plasma PTH is at least 1%, 2%, 3%, 4%, 5%, 6%, 7%, 8%, 9%, 10%, 11 %, 12%, 13%, 14%, 15%, 16%, 17%, 18%, 19%, 20%, 25% or 30% below pretreatment baseline levels for at least 10 hours post administration of the described compound. In specific embodiments, the reduction in plasma PTH is at least 20% at 10 hours post administration. In preferred embodiments, the reduction in plasma PTH is 15 to 40%, preferably 20 to 50%, more preferably 30 to 70% below pretreatment baseline levels for at least 48 hours post administration of the described compound.

The described compound is administered in an amount effective to decrease serum calcium or calcium effect. The reduction in serum calcium may be at least 1 %, 2%, 3%, 4%, 5%, 6%, 7%, 8%, 9%, 10%, 11%, 12%, 13%, 14%, 15%, 16%, 17%, 18%, 19%, 20% or 25% below pretreatment levels for at least 10 hours post administration of the compound. The reduction in serum calcium may be at least 5% at 10 hours post administration. The reduction in serum calcium may be 5 to 10%, preferably 5 to 20% below pretreatment levels for at least 48 hours post administration of the described compound.

Based on the relationship between serum calcium, bone metabolism and PTH, it is thought that the described compounds are beneficial for the treatment of various forms of bone disease and/or hypercalcemia in addition to hyperparathyroidism. The described compounds may have advantages compared to current therapeutic agents, because they may be administered parenterally and may not be associated with gastrointestinal adverse effects, are not metabolized by cytochrome P450 and may result in more effective reductions in plasma PTH and calcium.

As discussed above, the described methods may be used with the compound alone or in combination with one or more other therapeutically effective agents. Such other therapeutically effective agents include, but are not limited to, treatment with antiresorptive bisphosphonate agents, such as alendronate and risedronate; integrin blockers, such as 〈αᵥβ₃ antagonists; conjugated estrogens used in hormone replacement therapy, such as Prempro^{™}, Premarin^{™} and Endometrion^{™}; selective estrogen receptor modulators (SERMs), such as raloxifene, droloxifene, CP-336,156 (Pfizer) and lasofoxifene; cathespin K inhibitors; vitamin D therapy; vitamin D analogs, such as Zemplar^{™} (paricalcitol); Calcijex® (calcitriol), Hectorol® (doxercalciferol), One-Alpha® (alfacalcidol) and the analogs in development from Cytochroma known as CTA-018, CTAP201 and CTAP101; other calcimimetics such as Sensipar® (cinacalcet); inhibitors of type II sodium-dependent phosphate transporter family, SLC34 (including the two renal isoforms NaPi-Ila and NaPi-Ilc, and the intestinal NaPi-Ilb transporter); phosphatonins (including FGF-23, sFRP4, MEPE or FGF-7); low dose PTH treatment (with or without estrogen); calcitonin; inhibitors of RANK ligand; antibodies against RANK ligand, osteoprotegrin; adensosine antagonists; and ATP proton pump inhibitors.

A described compound may be administered at a dose sufficient to decrease both PTH and serum calcium levels.A described compound is administered at a dose sufficient to decrease PTH without significantly affecting serum calcium levels. A described compound is administered at a dose sufficient to increase PTH without significantly affecting serum calcium levels.

### IV. Calcium Sensing Receptor Agonists

The methods described herein comprise administration of a calcimimetic to a subject. Such agonists are described in U.S. Patent Nos. 6,011,068 and 6,031,003 and U.S. Patent Publication Nos. 2011/0028394 and 2009/0023652.

In one embodiment of the disclosure, the calcimimetic is cinacalcet hydrochloride.

Herein described is a calcimimetic which comprises a peptide comprising the formula: X₁ - X₂- X₃- X₄- X₅- X₆- X₇ wherein X₁ is a subunit comprising a thiol-containing group; X₅ is a cationic subunit; X₆ is a non-cationic subunit; X₇ is a cationic a subunit; and at least one, preferably two, of X₂, X₃ and X₄ is/are independently a cationic subunit.

In the disclosure, the calcimimetic is a compound comprising the sequence carrrar (SEQ ID NO:2). In another embodiment of the disclosure, the calcimimetic is a conjugate comprised of the peptide carrrar (SEQ ID NO:2), where the peptide is conjugated at its N-terminal residue to a Cys residue. Although the invention may be described in terms of certain preferred embodiments, such as SEQ ID NO:3, it will be within the understanding of one of skill in the art that the disclosure also applies to other calcimimetics, including the compounds and conjugates described in U.S. Patent Nos. 6,011,068 and 6,031,003 and U.S. Patent Publication Nos. 2011/0028394 and 2009/0023652. Likewise, although the invention may be described in terms of certain preferred embodiments, such as hemodialysis, it will be within the understanding of one of skill in the art that the disclosure also applies to other forms of dialysis, such as peritoneal dialysis, and other approaches, such as quotidian hemodialysis.

In the disclosure, the thiol-containing conjugate group has both an N-terminal cap and a C-terminal cap. In the disclosure, the thiol-containing conjugating group is itself a peptide comprising the amino acid sequence of SEQ ID NO:161. In the disclosure, the thiol-containing conjugating group and the peptide are the same (i.e., the conjugate is a dimer).

In the disclosure, compounds are in the form of a conjugate, where the thiol-containing subunit in position X₁ is linked through a disulfide linkage to an L-Cys residue. These compounds have, for example, the following structure:

In the notation used herein, the compound that is linked to the thiol-containing moiety in the X₁ subunit is identified parenthetically, where in these exemplary conjugates the compound L-Cys is indicated (C) is linked to the thiol-containing moiety in the X₁ subunit: Ac-c(C)arrrar-NH₂(SEQ ID NO:3) and Ac-c(Ac-C)arrrar-NH₂ (SEQ ID NO:141).

When the described agonists are administered as pharmaceuticals, to humans and animals, they can be given alone or as a pharmaceutical composition containing, for example, 0.1 to 99% (more preferably, 10 to 30%) of active ingredient in combination with a pharmaceutically acceptable carrier. The pharmaceutical composition may contain 0.2-25%, preferably 0.5-5% or 0.5-2%, of active ingredient. These compounds may be administered to humans and other animals for therapy by any suitable route of administration, including, e.g., oral, subcutaneous injection, subcutaneous depot, intravenous injection, intravenous or subcutaneous infusion

These agonists may be administered to humans and other animals for therapy by any suitable route of administration.

Peptide calcimimetics have been described previously (U.S. Patent Publication Nos. 2011/0028394 and 2009/0023652. One exemplary peptide calcimimetic is referred to herein as Ac-c(C)arrrar-NH₂ (SEQ ID NO:3) and has the structure:

Additional structures are provided in Table 1 below.

**Table 1**

| SEQ ID NO. | Compound Structure |
|---|---|
| SEQ ID NO:2 | carrrar |
| SEQ ID NO:3 | Ac-c(C)arrrar-NH₂ |
| SEQ ID NO:4 | Ac-crrrr-NH₂ |
| SEQ ID NO:5 | Ac-crrrrr-NH₂ |
| SEQ ID NO:6 | Ac-crrrrrr-NH₂ |
| SEQ ID NO:7 | Ac-crrrrrrr-NH₂ |
| SEQ ID NO:8 | Ac-carrrrr-NH₂ |
| SEQ ID NO:9 | Ac-crarrrr-NH₂ |
| SEQ ID NO:10 | Ac-crrarrr-NH₂ |
| SEQ ID NO:11 | Ac-crrrarr-NH₂ |
| SEQ ID NO:12 | Ac-crrrrar-NH₂ |
| SEQ ID NO:13 | Ac-crrrrra-NH₂ |
| SEQ ID NO:14 | Ac-crrarra-NH₂ |
| SEQ ID NO:15 | Ac-cararrr-NH₂ |
| SEQ ID NO:16 | Ac-carrarr-NH₂ |
| SEQ ID NO:17 | Ac-crraarr-NH₂ |
| SEQ ID NO:18 | Ac-crararr-NH₂ |
| SEQ ID NO:19 | Ac-carrrra-NH₂ |
| SEQ ID NO:20 | Ac-crarrra-NH₂ |
| SEQ ID NO:21 | Ac-crrraar-NH₂ |
| SEQ ID NO:22 | Ac-caarrrr-NH₂ |
| SEQ ID NO:23 | Ac-crarrar-NH₂ |
| SEQ ID NO:24 | Ac-craarrr-NH₂ |
| SEQ ID NO:25 | Ac-crrarar-NH₂ |
| SEQ ID NO:26 | Ac-carrrar-NH₂ |
| SEQ ID NO:27 | Ac-c(C)arrrar-NH₂ |
| SEQ ID NO:28 | Ac-c(C)rrarar-NH₂ |
| SEQ ID NO:29 | Ac-arrrar-NH₂ |
| SEQ ID NO:30 | Ac-bAla-crrrrrr-NH₂ |
| SEQ ID NO:31 | Mpa-rrrrrr-NH₂ |
| SEQ ID NO:32 | Ac-dHcy-rrrrrr-NH₂ |
| SEQ ID NO:33 | Ac-dPen-rrrrrr-NH₂ |
| SEQ ID NO:34 | Ac-C(C)arrrar-NH₂ |
| SEQ ID NO:35 | Ac-c(C)Arrrar-NH₂ |
| SEQ ID NO:36 | Ac-c(C)aRrrar-NH₂ |
| SEQ ID NO:37 | Ac-c(C)arRrar-NH₂ |
| SEQ ID NO:38 | Ac-c(C)arrRar-NH₂ |
| SEQ ID NO:39 | Ac-c(C)arrrAr-NH₂ |
| SEQ ID NO:40 | Ac-c(C)arrraR-NH₂ |
| SEQ ID NO:41 | Ac-crrrrrrrr-NH₂ |
| SEQ ID NO:42 | Ac-cGrrrGr-NH₂ |
| SEQ ID NO:43 | Ac-cArrrAr-NH₂ |
| SEQ ID NO:44 | Ac-CaRrRaR-NH₂ |
| SEQ ID NO:45 | CHDAPIGYD |
| SEQ ID NO:46 | CPDYHDAGI |
| SEQ ID NO:47 | Ac-CYGRKKRRQRRR-NH₂ |
| | |
| | |
| SEQ ID NO:48 | Ac-YGRKKRRQRRR-NH₂ |
| SEQ ID NO:49 | Ac-caraarrr-NH₂ |
| SEQ ID NO:50 | Ac-cygrkkrrqrrr-NH₂ |
| SEQ ID NO:51 | H₂N-crrrrrr-NH₂ |
| | |
| SEQ ID NO:52 | Ac-carrrar-NH₂ |
| | |
| SEQ ID NO:53 | Ac-c(GS)rrrrrr-NH₂ |
| SEQ ID NO:54 | GS-crrrrrr |
| SEQ ID NO:55 | Ac-c(Ac-C)arrrar-NH₂ |
| SEQ ID NO:56 | Ac-c(Mpa)arrrar-NH₂ |
| SEQ ID NO:57 | Ac-c(PEG2-C)arrrar-NH₂ |
| SEQ ID NO:58 | Ac-c(PEG5-C)rrrrrr-NH₂ |
| SEQ ID NO:59 | Ac-c(PEG2-C)rrrrrr-NH₂ |
| SEQ ID NO:60 | c(C)arrrar-NH₂ |
| SEQ ID NO:61 | Ac-bAla-c(C)arrrar-NH₂ |
| SEQ ID NO:62 | bAla-c(C)arrrar |
| SEQ ID NO:63 | Ac-cGrrrGr |
| SEQ ID NO:64 | Ac-cArrrAr |
| SEQ ID NO:65 | Ac-cvrrrvr-NH₂ |
| SEQ ID NO:66 | Ac-cvrrrvr |
| SEQ ID NO:67 | Ac-Crrrrrr-NH₂ |
| SEQ ID NO:68 | Ac-carrrer-NH₂ |
| SEQ ID NO:69 | Ac-cerrrar-NH₂ |
| SEQ ID NO:70 | Ac-carrrak-NH₂ |
| SEQ ID NO:71 | Ac-qrrrar-NH₂ |
| SEQ ID NO:72 | Ac-cakrrar-NH₂ |
| SEQ ID NO:73 | Ac-carkrar-NH₂ |
| SEQ ID NO:74 | Ac-carrrar-OH |
| SEQ ID NO:75 | Ac-CARRRAR-NH₂ |
| SEQ ID NO:76 | Ac-caarrrrrr-NH₂ |
| SEQ ID NO:77 | Ac-caaarrrrrr-NH₂ |
| SEQ ID NO:78 | Ac-carararar-NH₂ |
| SEQ ID NO:79 | Ac-carrrarar-NH₂ |
| SEQ ID NO:80 | crrrrrr-NH₂ |
| SEQ ID NO:32 | Ac-dHcy rrrrrr-NH₂ |
| SEQ ID NO:81 | Ac-c(Benzoyl)rrrrrr-NH₂ |
| SEQ ID NO:82 | Ac-c(acetyl)rrrrrr-NH₂ |
| SEQ ID NO:83 | Ac-carrrfr-NH₂ |
| SEQ ID NO:84 | Ac-carrrir-NH₂ |
| SEQ ID NO:85 | Ac-carrrir-NH₂ |
| SEQ ID NO:68 | Ac-carrrer-NH₂ |
| SEQ ID NO:87 | Ac-carrrvr-NH₂ |
| SEQ ID NO:88 | Ac-carrrpr-NH₂ |
| SEQ ID NO:89 | Ac-carrrhr-NH₂ |
| SEQ ID NO:90 | Ac-carrrqr-NH₂ |
| SEQ ID NO:91 | Ac-carrrtr-NH₂ |
| SEQ ID NO:92 | Ac-carrrsr-NH₂ |
| SEQ ID NO:93 | Ac-carrrGr-NH₂ |
| SEQ ID NO:94 | Ac-cerrrar-NH₂ |
| SEQ ID NO:95 | Ac-cGrrrar-NH₂ |
| SEQ ID NO:96 | Ac-cirrrar-NH₂ |
| SEQ ID NO:97 | Ac-cprrrar-NH₂ |
| SEQ ID NO:98 | Ac-clrrrar-NH₂ |
| SEQ ID NO:99 | Ac-cqrrrar-NH₂ |
| SEQ ID NO:100 | Ac-ctrrrar-NH₂ |
| SEQ ID NO:101 | Ac-cvrrrar-NH₂ |
| SEQ ID NO:102 | Ac-csrrrar-NH₂ |
| SEQ ID NO:103 | Ac-chrrrar-NH₂ |
| SEQ ID NO:104 | Ac-cfrrrar-NH₂ |
| SEQ ID NO:105 | Ac-crrGrar-NH₂ |
| SEQ ID NO:106 | Ac-crrprar-NH₂ |
| SEQ ID NO:107 | Ac-crrerar-NH₂ |
| SEQ ID NO:108 | Ac-crrtrar-NH₂ |
| SEQ ID NO:109 | Ac-crrhrar-NH₂ |
| SEQ ID NO:110 | Ac-crrfrar-NH₂ |
| SEQ ID NO:111 | Ac-crrsrar-NH₂ |
| SEQ ID NO:112 | Ac-crrqrar-NH₂ |
| SEQ ID NO:113 | Ac-crrvrar-NH₂ |
| SEQ ID NO:114 | Ac-crrlrar-NH₂ |
| SEQ ID NO:115 | Ac-crrirar-NH₂ |
| SEQ ID NO:116 | Ac-crr-Sar-rar-NH₂ |
| SEQ ID NO:117 | Ac-carrr-Sar-r-NH₂ |
| SEQ ID NO:118 | Ac-c-Nma-rrr-Nma-r-NH₂ |
| SEQ ID NO:119 | Ac-crrar-Nma-r-NH₂ |
| SEQ ID NO:120 | Ac-c-Aib-rrr-Aib-r-NH₂ |
| SEQ ID NO:121 | Ac-crr-Nma-rar-NH₂ |
| SEQ ID NO:122 | Ac-carrr-Nma-r-NH₂ |
| SEQ ID NO:123 | Ac-c-Aib-rrrar-NH₂ |
| SEQ ID NO:124 | Ac-carrr-Aib-r-NH₂ |
| SEQ ID NO:125 | Ac-c-Sar-rrr-Sar-r-NH₂ |
| SEQ ID NO:126 | Ac-crrar-Sar-r-NH₂ |
| SEQ ID NO:127 | Ac-c-Nma-rrrar-NH₂ |
| SEQ ID NO:128 | Ac-c-Sar-rrrar-NH₂ |
| SEQ ID NO:129 | Ac-carrr-Nle-r-NH₂ |
| SEQ ID NO:130 | Ac-c-dNle-rrr-dNle-r-NH₂ |
| SEQ ID NO:131 | Ac-carrr-dNva-r-NH₂ |
| SEQ ID NO:132 | Ac-c-d Nva-rrr-d Nva-r-N H₂ |
| SEQ ID NO:133 | Ac-crrar-dNle-r-NH₂ |
| SEQ ID NO:134 | Ac-c-dNle-rrrar-NH₂ |
| SEQ ID NO:135 | Ac-crrar-dNva-r-NH₂ |
| SEQ ID NO:136 | Ac-c-dNva-rrrar-NH₂ |
| SEQ ID NO:137 | Ac-crr-dNva-rar-NH₂ |
| SEQ ID NO:138 | Ac-crr-dNle-rar-NH₂ |
| SEQ ID NO:139 | Ac-c(dHcy)arrrar-NH₂ |
| SEQ ID NO:140 | Ac-c(Mpa)arrrar-NH₂ |
| SEQ ID NO:141 | Ac-c(Ac-C)arrrar-NH₂ |
| SEQ ID NO:142 | Ac-c(c)arrrar-NH₂ |
| SEQ ID NO:143 | Ac-c(C-PEG20)rrrrrr-NH₂ |
| SEQ ID NO:144 | Ac-c(C-PEG40)rrrrrr-NH₂ |
| SEQ ID NO:145 | CEEEEEE |
| | |
| | |
| | |
| | |
| SEQ ID NO:146 | Ac-crrrraa-NH₂ |
| SEQ ID NO:147 | Ac-cakkkak-NH₂ |
| SEQ ID NO:148 | Ac-cararar-NH₂ |
| SEQ ID NO:149 | Ac-crrarGr-NH₂ |
| SEQ ID NO:150 | Ac-crrarqr-NH₂ |
| SQ ID NO:151 | Ac-crrarhr-NH₂ |
| SEQ ID NO:152 | Ac-crrarir-NH₂ |
| SEQ ID NO:153 | Ac-ca(DAP)rrar-NH₂ |
| SEQ ID NO:154 | Ac-ca(dHar)(dHar)(dHar)ar-NH₂ |
| SEQ ID NO:162 | CRRR |
| SEQ ID NO:163 | CRRRR |
| SEQ ID NO:164 | CRRRRRRR |
| SEQ ID NO:165 | CRRRRRRRR |
| SEQ ID NO:165 | CRRRRRRRR |
| SEQ ID NO:166 | CRRRRRRRRR |
| SEQ ID NO:167 | CRRRRRRRRRR |
| SEQ ID NO:168 | CRRRRRRRRRRR |
| SEQ ID NO:169 | CRRRRRRRRRRRR |
| SEQ ID NO:170 | Ac-c(Ac-C)rrarar-NH₂ |
| GS = oxidized glutathione; dHcy = D-homocysteine; Mpa = Mercaptopropionic acid; PEG = polyethylene glycol. | |

### V. Formulations

A pharmaceutical composition comprising a described compound and at least one pharmaceutically acceptable excipient or carrier is provided. Methods of preparing such pharmaceutical compositions typically comprise the step of bringing into association a described compound with a carrier and, optionally, one or more accessory ingredients. The described compounds and/or pharmaceutical compositions comprising same may be formulated into pharmaceutically-acceptable dosage forms by conventional methods known to those of skill in the art. Typically, formulations are prepared by uniformly and intimately bringing into association a described compound with liquid carriers, or finely divided solid carriers, or both, and then, if necessary, shaping the product.

Pharmaceutical compositions herein described aresuitable for parenteral administration comprise one or more described compounds in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain sugars, alcohols, amino acids, antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These pharmaceutical compositions may also contain adjuvants such as preservatives, wetting agents, emulsifying agents and dispersing agents. Prevention of the action of microorganisms upon the described compounds may be ensured by the inclusion of various antibacterial and antifungal agents, for example, paraben, chlorobutanol, phenol sorbic acid, and the like. It may also be desirable to include agents to control tonicity, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

For example, a described compound may be delivered to a human in a form of solution that is made by reconstituting a solid form of the drug with liquid. This solution may be further diluted with infusion fluid such as water for injection, 0.9% sodium chloride injection, 5% dextrose injection and lactated ringer's injection. It is preferred that the reconstituted and diluted solutions be used within 4-6 hours for delivery of maximum potency. Alternatively, a described compound may be delivered to a human in a form of tablet or capsule.

Injectable depot forms are made by forming microencapsulated matrices of the described compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissue.

When the described compounds are administered as pharmaceuticals, to humans and animals, they can be given alone or as a pharmaceutical composition containing, for example, 0.1 to 99% (more preferably, 10 to 30%) of active ingredient in combination with a pharmaceutically acceptable carrier. In other embodiments, the pharmaceutical composition may contain 0.2-25%, preferably 0.5-5% or 0.5-2%, of active ingredient. These compounds may be administered to humans and other animals for therapy by any suitable route of administration, including, e.g., subcutaneous injection, subcutaneous depot, intravenous injection, intravenous or subcutaneous infusion. These compounds may be administered rapidly (within < 1 minute) as a bolus or more slowly over an extended period of time (over several minutes, hours or days). These compounds may be delivered daily or over multiple days, continuously or intermittently. In one embodiment, the compounds may be administered transdermally (e.g., using a patch, microneedles, micropores, ointment, microjet or nanojet).

Regardless of the route of administration selected, the described compounds, which may be used in a suitable hydrated form, and/or the pharmaceutical compositions, are formulated into pharmaceutically-acceptable dosage forms by conventional methods known to those of skill in the art.

Actual dosage levels of the active ingredients in the pharmaceutical compositions may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular described compound employed, or the ester, salt or amide thereof, the route of administration, the time of administration, the rate of excretion or metabolism of the particular compound being employed, the rate and extent of absorption, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the described compounds employed in the pharmaceutical composition at levels lower than that required in order to achieve the desired therapeutic effect and gradually increase the dosage until the desired effect is achieved.

In general, a suitable daily dose of a described compound will be that amount of the compound which is the lowest dose effective to produce a therapeutic effect. Such an effective dose will generally depend upon the factors described above. Generally, intravenous, intramuscular, transdermal, intracerebroventricular and subcutaneous doses of the described compounds for a patient, when used for the indicated effects, will range from about 1 µg to about 5 mg per kilogram of body weight per hour. The dose may range from about 5 µg to about 2.5 mg per kilogram of body weight per hour. In further embodiments, the dose will range from about 5 µg to about 1 mg per kilogram of body weight per hour.

If desired, the effective daily dose of a described compound may be administered as two, three, four, five, six or more sub-doses administered separately at appropriate intervals throughout the day, optionally, in unit dosage forms. In one embodiment, the described compound is administered as one dose per day. In further embodiments, the compound is administered continuously, as through intravenous or other routes. The compound may be administered less frequently than daily, such as every 2-3 days. In still other embodiments of the disclosure, the compound is administered in conjunction with dialysis treatment, weekly or less frequently.

The subject receiving this treatment is any animal in need, including primates, in particular humans, and other mammals such as equines, cattle, swine and sheep; and poultry and pets in general.

The described compounds may be administered as such or in admixtures with pharmaceutically acceptable carriers and can also be administered in conjunction with antimicrobial agents such as penicillins, cephalosporins, aminoglycosides and glycopeptides. Conjunctive therapy thus includes sequential, simultaneous and separate administration of the active compound in a way that the therapeutic effects of the first administered one is not entirely disappeared when the subsequent is administered. Routes of Administration for Disclosed Compounds

These compounds may be administered to humans and other animals for therapy by any suitable route of administration. As used herein, the term "route" of administration is intended to include, but is not limited to subcutaneous injection, subcutaneous depot, intravenous injection, intravenous or subcutaneous infusion, intraocular injection, intradermal injection, intramuscular injection, intraperitoneal injection, intratracheal administration, intraadiposal administration, intraarticular administration, intrathecal administration, epidural administration, inhalation, intranasal administration, sublingual administration, buccal administration, rectal administration, vaginal administration, intracisternal administration and topical administration, transdermal administration, or administration via local delivery (for example by catheter or stent).

Transdermal drug delivery to the body is a desirable and convenient method for systemic delivery of biologically active substances to a subject, and in particular for delivery of substances that have poor oral bioavailability, such as proteins and peptides. The transdermal route of delivery has been particularly successful with small (e.g., less than about 1,000 Daltons) lipophilic compounds, such as scopolamine and nicotine, that can penetrate the stratum corneum outer layer of the skin, which serves as an effective barrier to entry of substances into the body. Below the stratum corneum is the viable epidermis, which contains no blood vessels, but has some nerves. Deeper still is the dermis, which contains blood vessels, lymphatics and nerves. Drugs that cross the stratum corneum barrier can generally diffuse to the capillaries in the dermis for absorption and systemic distribution.

Technological advances in transdermal delivery have focused on addressing the need in the art to deliver hydrophilic, high molecular weight compounds, such as proteins and peptides, across the skin. One approach involves disruption of the stratum corneum using chemical or physical methods to reduce the barrier posed by the stratum corneum. Skin microporation technology, which involves the creation of micron dimension transport pathways (micropores) in the skin (in particular, the micropores in the stratum corneum) using a minimally invasive technique, is a more recent approach. Techniques to create micropores in the skin (stratum corneum) include thermal microporation or ablation, microneedle arrays, phonophoresis, laser ablation and radiofrequency ablation (Prausnitz and Langer (2008) Nat. Biotechnology 11:1261-68; Arora et al., Int. J. Pharmaceutics, 364:227 (2008); Nanda et al., Current Drug Delivery, 3:233 (2006); Meidan et al. American J. Therapeutics, 11:312 (2004)).

As noted above, PTH secretion is regulated by the CaSR which is expressed on the cell surface of parathyroid cells. Thus, in order to activate the CaSR, the agent or compound must be delivered to the parathyroid cell. Transdermal delivery of calcimimetic agents must achieve delivery across the stratum corneum and provide systemic exposure to reach the parathyroid cell. To date, the art has not demonstrated whether a calcimimetic compound can be delivered transdermally in an amount sufficient for therapeutic benefit and in particular in an amount sufficient for decreasing PTH and/or the treatment, attenuation, lessening and/or relief hypercalcemia.

In addition to calcimimetics, 1,25-(OH)₂ vitamin D₃ analogs are the most commonly used treatments for patients with hyperparathyroidism associated with chronic kidney disease and end stage renal disease. Vitamin D analogs act by facilitating intestinal absorption of dietary calcium, and reduce PTH levels by inhibiting PTH synthesis and secretion. While intravenous and oral delivery of vitamin D has been used therapeutically, to date, the art has not demonstrated whether vitamin D analogs, such as ZEMPLAR^{™} (paricalcitol), CALCIJEX® (calcitriol), ONE-ALPHA® (alfacalcidol) and HECTOROL® (doxercalciferol) can be delivered transdermally in an amount sufficient for therapeutic benefit and in particular in an amount sufficient for decreasing parathyroid hormone (PTH). In addition, the art has not demonstrated whether the co-administration by transdermal delivery of a calcimimetic agent in combination with a vitamin D analog (either as separate formulations or as a co-formulation) in amounts sufficient for therapeutic benefit, and in particular in amounts sufficient for decreasing PTH and provide effective treatment for patients suffering from hyperparathyroidism.

The calcimimetic agents may be administered across the stratum corneum, and/or other layers of the epidermis, for local or systemic delivery, for decreasing parathyroid hormone (PTH) and/or treating hypercalcemia. In one embodiment, the calcimimetic agent is delivered via microporation. Any one of a number of techniques for microporation is contemplated, and several are briefly described.

Microporation can be achieved by mechanical means and/or external driving forces, to breach the stratum corneum to deliver the calcimimetic agents described herein through the surface of the skin and into the underlying skin layers and/or the bloodstream.

The microporation technique is ablation of the stratum corneum in a specific region of the skin using a pulsed laser light of wavelength, pulse length, pulse energy, pulse number, and pulse repetition rate sufficient to ablate the stratum corneum without significantly damaging the underlying epidermis. The calcimimetic agent is then applied to the region of ablation. Another laser ablation microporation technique, referred to as laser-induced stress waves (LISW), involves broadband, unipolar and compressible waves generated by high-power pulsed lasers. The LISWs interact with tissues to disrupt the lipids in the stratum corneum, creating intercellular channels transiently within the stratum corneum. These channel, or micropores, in the stratum corneum permit entry of the calcimimetic agent.

Sonophoresis or phonophoresis is another microporation technique that uses ultrasound energy. Ultrasound is a sound wave possessing frequencies above 20 KHz. Ultrasound can be applied either continuously or pulsed, and applied at various frequency and intensity ranges (Nanda et al., Current Drug Delivery, 3:233 (2006)).

Another microporation technique involves the use of a microneedle array. The array of microneedles when applied to a skin region on a subject pierce the stratum corneum and do not penetrate to a depth that significantly stimulates nerves or punctures capillaries. The patient, thus, feels no or minimal discomfort or pain upon application of the microneedle array for generation of micropores through which the calcimimetic agent is delivered.

Microneedle arrays comprised of hollow or solid microneedles are contemplated, where the calcimimetic agent can be coated on the external surface of the needles or dispensed from the interior of hollow needles. Examples of microneedle arrays are described, for example, in Nanda et al., Current Drug Delivery, 3:233 (2006) and Meidan et al. American J. Therapeutics, 11:312 (2004). First generation microneedle arrays were comprised of solid, silicon microneedles that were externally coated with a therapeutic agent. When the microarray of needles was pressed against the skin and removed after about 10 seconds, the permeation of the agent on the needles into the body was readily achieved. Second generation microneedle arrays were comprised of microneedles of solid or hollow silicon, polycarbonate, titanium or other suitable polymer and coated or filled with a solution of the therapeutic compound. Newer generations of microneedle arrays are prepared from biodegradable polymers, where the tips of the needles coated with a therapeutic agent remain in the stratum corneum and slowly dissolve.

The microneedles can be constructed from a variety of materials, including metals, ceramics, semiconductors, organics, polymers, and composites. Exemplary materials of construction include pharmaceutical grade stainless steel, gold, titanium, nickel, iron, tin, chromium, copper, palladium, platinum, alloys of these or other metals, silicon, silicon dioxide, and polymers. Representative biodegradable polymers include polymers of hydroxy acids such as lactic acid and glycolic acid polylactide, polyglycolide, polylactide-co-glycolide, and copolymers with poly(ethylene glycol), polyanhydrides, poly(ortho)esters, polyurethanes, poly(butyric acid), poly(valeric acid), and poly(lactideco- caprolactone). Representative non-biodegradable polymers include polycarbonate, polyester, and polyacrylamides.

The microneedles can have straight or tapered shafts. In one embodiment, the diameter of the microneedle is greatest at the base end of the microneedle and tapers to a point at the end distal the base. The microneedle can also be fabricated to have a shaft that includes both a straight (untapered) portion and a tapered portion. The needles may also not have a tapered end at all, i.e. they may simply be cylinders with blunt or flat tips. A hollow microneedle that has a substantially uniform diameter, but which does not taper to a point, is referred to herein as a "microtube." As used herein, the term "microneedle" includes both microtubes and tapered needles unless otherwise indicated.

Electroporation is another technique for creating micropores in the skin. This approach uses the application of microsecond or millisecond long high-voltage electrical pulses to created transient, permeable pores within the stratum corneum.

Other microporation techniques include use of radio waves to create microchannels in the skin. Thermal ablation is yet another approach to achieve delivery of larger molecular weight compounds transdermally.

Applicants have discovered that low doses of calcimimetic agents may be therapeutically administered over an extended period of time to treat SHPT. This markedly differs from current dose requirements of other calcimimetics (e.g., cinacalcet hydrochloride).

### VI. Combination Therapy

As described above, the methods of use may be used alone or in combination with other approaches for the treatment of hypercalcemia and/or bone disease. Such other approaches include, but are not limited to, treatment with agents such as bisphosphonate agents, integrin blockers, hormone replacement therapy, selective estrogen receptor modulators, cathepsin K inhibitors, vitamin D therapy, vitamin D analogs, such as ZEMPLAR^{™}(paricalcitol), CALCIJEX® (calcitriol), ONE-ALPHA® (alfacalcidol) and HECTOROL® (doxercalciferol), anti-inflammatory agents, low dose PTH therapy (with or without estrogen), calcimimetics, phosphate binders, calcitonin, inhibitors of RANK ligand, antibodies against RANK ligand, osteoprotegrin, adensosine antagonists and ATP proton pump inhibitors.

A combination therapy may use vitamin D or a vitamin D analog in combination with a calcimimetic agent. Vitamin D aids in the absorption of calcium and functions to maintain normal blood levels of calcium and phosphorous. PTH works to enhance calcium absorption in the intestine by increasing the production of 1,25-(OH)₂ vitamin D, the active form of vitamin D. PTH also stimulates phosphorus excretion from the kidney, and increases release from bone.

As discussed above, secondary hyperparathyroidism is characterized by an elevation in parathyroid hormone (PTH) associated with inadequate levels of active vitamin D hormone. Vitamin D or a vitamin D analog may be used to reduce elevated PTH levels in treatment of secondary hyperparathyroidism. In one embodiment, the invention includes a pharmaceutical composition comprising a calcimimetic agent and a vitamin D analog.

Herein described is a pharmaceutical composition comprising a calcimimetic agent and ZEMPLAR^{™}(paricalcitol). Paricalcitol is a synthetic analog of calcitriol, the metabolically active form of vitamin D. The recommended initial dose of Zemplar is based on baseline intact parathyroid hormone (iPTH) levels. If the baseline iPTH level is less than or equal to 500 pg/mL, the daily dose is 1 µg and the "three times a week" dose (to be administered not more than every other day) is 2 µg. If the baseline iPTH is greater than 500 pg/mL, the daily dose is 2 µg, and the "three times a week" does (to be administered not more than every other day) is 4 µg. Thereafter, dosing must be individualized and based on serum plasma iPTH levels, with monitoring of serum calcium and serum phosphorus. Paricalcitol is described in U.S. Patent No. 5,246,925 and U.S. Patent No. 5,587,497.

Herein described is a pharmaceutical composition comprising a calcimimetic agent and CALCIJEX® (calcitriol). Calcitriol is the metabolically active form of vitamin D. The recommended initial dosage for CALCIJEX® (oral) is 0.25 µ/day. This amount may be increased by 0.25 µg/day at 4- to 8-wk intervals. Normal or only slightly reduced calcium levels may respond to dosages of 0.25 µg every other day. For patients on dialysis, the recommended initial dose for CALCIJEX® (IV) is 0.02 µg/kg (1 to 2 µg) 3 times/week, every other day. This amount may be increased by 0.5 to 1 µg, every 2 to 4 wk. Calcitriol is described in U.S. Patent No. 6,051,567 and U.S. Patent No. 6,265,392 and U.S. Patent No. 6,274,169.

A pharmaceutical composition comprising a calcimimetic agent and HECTOROL® (doxercalciferol) may be provided. Doxercalciferol is a synthetic analog of vitamin D that undergoes metabolic activation *in vivo* to form 1 α, 25-dihydroxyvitamin D₂, a naturally occurring, biologically active form of vitamin D. The recommended initial dose of HECTOROL® is 10 µg administered three times weekly at dialysis (approximately every other day). The initial dose should be adjusted, as needed, in order to lower blood iPTH into the range of 150 to 300 pg/mL. The dose may be increased at 8-week intervals by 2.5 µg if iPTH is not lowered by 50% and fails to reach target range. The maximum recommended dose of HECTOROL is 20 µg administered three times a week at dialysis for a total of 60 µg per week. Doxercalciferol is described in U.S. Patent No. 5,602,116 and U.S. Patent No. 5,861,386 and U.S. Patent No. 5,869,473 and U.S. Patent No. 6,903,083.

The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder (for example, an inventive compound may be administered concurrently with another agent used to treat the same disorder), or they may achieve different effects (e.g., control of any adverse effects). As used herein, additional therapeutic agents that are normally administered to treat or prevent a particular disease, or condition, are known as "appropriate for the disease, or condition, being treated".

A combination treatment as defined herein may be achieved by way of the simultaneous, sequential or separate administration of the individual components of said treatment.

The compounds or pharmaceutically acceptable compositions thereof may also be incorporated into compositions for coating implantable medical devices, bioerodible polymers, implantable pump, and suppositories. Accordingly, in another aspect, a composition for coating an implantable device comprising a described compound as described generally above is contemplated, and a carrier suitable for coating the implantable device. In still another aspect, included is an implantable device coated with a composition comprising a compound as described generally above, and a carrier suitable for coating said implantable device.

Suitable coatings and the general preparation of coated implantable devices are described in U.S. Pat. Nos. 6,099,562; 5,886,026; and 5,304,121. The coatings are typically biocompatible polymeric materials such as a hydrogel polymer, polymethyldisiloxane, polycaprolactone, polyethylene glycol, polylactic acid, ethylene vinyl acetate, and mixtures thereof. The coatings may optionally be further covered by a suitable topcoat of fluorosilicone, polysaccarides, polyethylene glycol, phospholipids or combinations thereof to impart controlled release characteristics in the composition.

### VII. Potential Clinical Markers for Determining Treatment Efficacy

Determination of the effectiveness of a described method of treatment may be determined by a variety of methods.

Normal levels of serum calcium are in the range of 8.8mg/dL to 10.4 mg/dL (2.2 mmol/L to 2.6 mmol/L). In certain cases, the efficacy of treatment may be determined by measurement of serum and urinary markers related to calcium, including but not limited to, total and ionized serum calcium, albumin, plasma PTH, PTHrP, phosphate, vitamin D, and magnesium.

In other cases, efficacy may be determined by measurement of bone mineral density (BMD), or by measurement of biochemical markers for bone formation and/or bone resorption in serum or urine. Potential bone formation markers include, but are not limited to, total alkaline phosphatase, bone alkaline phosphatase, osteocalcin, undercarboxylated osteocalcin, C-terminal procollagen type I propeptide, and N-terminal procollagen type I propeptide. Potential bone resorption markers include, but are not limited, hydroxyproline, hydroxylysine, glycosyl-galactosyl hydroxylysine, galactosyl hydroxylysine, pyridinoline, deoxypyridinoline, N-terminal crosslinking telopeptide of type I collagen, C-terminal crosslinking telopeptide of type I collagen, C-terminal crosslinking telopeptide of type I collagen generated by MMPs, bone sialoprotein, acid phosphatase and tartrate-resistant acid phosphatase.

In other cases, efficacy may be determined by the percent reduction in PTH relative to a pre-dosing (baseline) level and/or by achieving a desirable PTH level as generally accepted as being beneficial to patients (for example, guidelines established by the National Kidney Foundation). Still in other cases, efficacy may be determined by measurement of the reduction in parathyroid gland hyperplasia associated with a hyperparathyroidism disease.

It is expected that when a described treatment is administered to a subject in need thereof, the treatment will produce an effect, as measured by, for example, one or more of: total serum calcium, ionized serum calcium, total blood calcium, ionized blood calcium, albumin, plasma PTH, blood PTH, PTHrP, phosphate, vitamin D, magnesium, bone mineral density (BMD), total alkaline phosphatase, bone alkaline phosphatase, osteocalcin, under carboxylated osteocalcin, C-terminal procollagen type I propeptide, N-terminal procollagen type I propeptide, hydroxyproline, hydroxylysine, glycosyl-galactosyl hydroxylysine, galactosyl hydroxylysine, pyridinoline, deoxypyridinoline, N-terminal crosslinking telopeptide of type I collagen, C-terminal crosslinking telopeptide of type I collagen, C-terminal crosslinking telopeptide of type I collagen generated by MMPs, bone sialoprotein, acid phosphatase and tartrate-resistant acid phosphatase. Effects include prophylactic treatment as well as treatment of existing disease.

A biologically effective molecule may be operably linked to a described peptide with a covalent bond or a non-covalent interaction. In specific embodiments, the operably linked biologically effective molecules can alter the pharmacokinetics of the described compounds by virtue of conferring properties to the compound as part of a linked molecule. Some of the properties that the biologically effective molecules can confer on the described compounds include, but are not limited to: delivery of a compound to a discrete location within the body; concentrating the activity of a compound at a desired location in the body and reducing its effects elsewhere; reducing side effects of treatment with a compound; changing the permeability of a compound; changing the bioavailability or the rate of delivery to the body of a compound; changing the length of the effect of treatment with a compound; altering the *in vitro* chemical stability of the compound; altering the *in vivo* stability of the compound, half-life, clearance, absorption, distribution and/or excretion; altering the rate of the onset and the decay of the effects of a compound; providing a permissive action by allowing a compound to have an effect.

In a further aspect, the described compound may be conjugated to polyethylene glycol (PEG). The selected PEG may be of any convenient molecular weight, and may be linear or branched, and may be optionally conjugated through a linker. The average molecular weight of PEG will preferably range from about 2 kiloDalton (kDa) to about 100 kDa, more preferably from about 5 kDa to about 40 kDa. Alternatively, the PEG moiety used can be 2, 3, 4, 5, 6, 7, 8, 9, 10, 11, 12, 13, 14, 15, 16, 17, 18, 19, or 20kDa.

The described compounds may be conjugated to PEG through a suitable amino acid residue located at any position on the compounds. The described compounds may optionally contain an additional amino acid residue to which PEG is conjugated, including for example, an additional amine-containing residue, such as lysine.

PEGylated peptides are known in the art to increase serum half-life of conjugated peptide. A variety of methods are known in the art for the formation of PEGylated peptides. For example, the PEG moiety can be linked to the amino terminus, the carboxy terminus or through a side chain of the claimed peptide, optionally through the presence of a linking group. In other embodiments, the PEG moiety may be linked to the sulfur of a thiol-containing amino acid, such as cysteine, or may be coupled to the sidechain of an amine-containing amino acid, such as lysine.

The PEG groups will generally be attached to the described compound by acylation or alkylation through a reactive group on the PEG moiety (e.g., an aldehyde, amine, oxime, hydrazine thiol, ester, or carboxylic acid group) to a reactive group on the described compound (e.g., an aldehyde, amine, oxime, hydrazine, ester, acid or thiol group), which may be located at the amino terminus, carboxy terminus, or a sidechain position of the described compound. One approach for preparation of PEGylation of synthetic peptides consists of combining through a conjugate linkage in solution, a peptide and a PEG moiety, each bearing a functional group that is mutually reactive towards the other. Peptides can be easily prepared using conventional solution or solid phase synthesis techniques. Conjugation of the peptide and PEG is typically done in aqueous phase and may be monitored by reverse phase HPLC. The PEGylated peptides can be readily purified and characterized, using standard techniques known to one of skill in the art.

One or more individual subunits of the described compounds may also be modified with various derivatizing agents known to react with specific side chains or terminal residues. For example, lysinyl residues and amino terminal residues may be reacted with succinic anhydride or other similar carboxylic acid anhydrides which reverses the charge on the lysinyl or amino residue. Other suitable reagents include, e.g., imidoesters such as methyl picolinimidate; pyridoxal; pyridoxal phosphate; chloroborohydride; trinitrobenzenesulfonic acid; O-methylisourea; 2,4,-pentanedione; and transaminase-catalyzed reaction with glyoxalate. Arginyl residues may be modified by reaction with conventional agents such as phenylglyoxal, 2,3-butanedione, 1,2- cyclohexanedione, and ninhydrin.

In addition, the described compounds may be modified to include non-cationic residues that provide immunogenic residues useful for the development of antibodies for bioanalytical ELISA measurements, as well as to evaluate immunogenicity. For example, the described compounds may be modified by incorporation of tyrosine and/or glycine residues. Specific modifications of tyrosyl residues are of particular interest for introducing spectral labels into tyrosyl residues. Non-limiting examples include reaction with aromatic diazonium compounds or tetranitromethane. Most commonly, Nacetylimidazole and tetranitromethane are used to form O-acetyl tyrosyl and 3-nitro derivatives, respectively.

### VII. Kits Comprising the Disclosed Compounds

Herein described are kits for carrying out the therapeutic regimens of the invention. Such kits comprise therapeutically effective amounts of the described compounds having activity as a CaSR modulator, in pharmaceutically acceptable form, alone or in combination with other agents, in pharmaceutically acceptable form. Preferred pharmaceutical forms include the described compounds in combination with sterile saline, dextrose solution, buffered solution, sterile water, or other pharmaceutically acceptable sterile fluid. Alternatively the composition may include an antimicrobial or bacteriostatic agent. Alternatively, the composition may be lyophilized or desiccated. In this instance, the kit may further comprise a pharmaceutically acceptable solution, preferably sterile, to form a solution for injection purposes. The kit may further comprise a needle or syringe, preferably packaged in sterile form, for injecting the composition. In other embodiments, the kit further comprises an instruction means for administering the composition to a subject. The instruction means can be a written insert, an audiotape, an audiovisual tape, or any other means of instructing the administration of the composition to a subject.

The kit may comprise (i) a first container containing a described compound having activity as a CaSR modulator; and (ii) instruction means for use. In another embodiment the kit comprises (i) a first container containing a compound as described herein, and (ii) a second container containing a pharmaceutically acceptable vehicle for dilution or reconstitution.

The kit may comprise (i) a first container containing a described compound having activity as a CaSR modulator; (ii) a second container containing an anticalcemic agent; and (iii) instruction means for use.

The anticalcemic agent may be an agent selected from the group consisting of bisphosphonate agents, hormone replacement therapeutic agents, vitamin D therapy, vitamin D analogs, such as ZEMPLAR_{™} (paricalcitol); CALCIJEX® (calcitriol), ONE-ALPHA_{®(alfacalcidol)} and HECTOROL® (doxercalciferol), low dose PTH (with or without estrogen), and calcitonin.

In related aspects, herein described are articles of manufacture that comprise the contents of the kits described above. For instance, herein described is provides an article of manufacture comprising an effective amount of a described peptide, alone or in combination with other agents, and instruction means indicating use for treating diseases described herein.

### EXAMPLES

The following examples are offered to illustrate but not to limit the compounds and methods described herein. Various modifications may be made by the skilled person without departing from the true spirit and scope of the subject matter described herein.

### Example 1

### Disease Progression in Ac-c(C)arrrar-NH₂-Treated Animals

The therapeutic efficacy of Ac-c(C)arrrar-NH₂ (SEQ ID NO:3) was assessed using the 5/6 nephrectomy (Nx) rat model of renal insufficiency. The 5/6 Nx male rats were obtained from Charles River Laboratories (Wilmington, MA). These rats have undergone surgical removal of one kidney and 2/3 of the other kidney and were fed a high-phosphate diet. All experimental procedures with animals were performed according to IACUC guidelines. Statistical analysis was performed using one-way ANOVA with Bonferroni post test. All p-values are nominal.

### A. PTH Suppression

Male 5/6 Nx rats were dosed daily for 28 days with Ac-c(C)arrrar-NH₂ (SEQ ID NO:3) by IV injection at a dose of 1mg/kg (IV), saline (IV), or cinacalcet at a dose of 10 mg/kg (PO). Tail vein blood samples were taken periodically for measurement of PTH. PTH was measured using the Immutopics BioActive Intact ELISA (Cat #60-2700; Immutopics, San Clemente, CA).

After 4 weeks, the mean baseline PTH levels ranged from 413-498 pg/mL (FIG. 1). Measurements taken 6 hours after the last dosing show that PTH levels were reduced in the animals treated with Ac-c(C)arrrar-NH₂ (SEQ ID NO:3)and in the animals treated with cinacalcet. Measurements taken at 16 hours and 48 hours after the last dosing show that PTH levels had increased in the animals treated with cinacalcet, but PTH levels remained suppressed in the animals treated with Ac-c(C)arrrar-NH₂ (SEQ ID NO:3).

### B. Parathyroid Gland Hyperplasia

Male 5/6 Nx rats were dosed thrice-weekly for 6 weeks with Ac-c(C)arrrar-NH₂ (SEQ ID NO:3) at a dose of 3 mg/kg (SC). Control animals were untreated. At sacrifice, tissue samples were removed for immunohistochemistry (IHC) analysis to measure the extent of parathyroid gland hyperplasia.

Parathyroid glands from the animals were analyzed using Bromodeoxyuridine (5-bromo-2'-deoxyuridine, BrdU).

Parathyroid glands from animals treated with Ac-c(C)arrrar-NH₂ (SEQ ID NO:3) had fewer BrdU-positive cells relative to untreated controls (FIGS. 2A-C), indicating reduced parathyroid gland proliferation in the treated animals.

In addition, parathyroid gland weight was reduced in animals treated with Ac-c(C)arrrar-NH₂ (SEQ ID NO:3) relative to untreated controls (FIG. 2D).

### C. Ectopic Calcification

Male 5/6 Nx rats were dosed thrice-weekly for 6 weeks with Ac-c(C)arrrar-NH₂ (SEQ ID NO:3) at a dose of 0.3, 1 or 3 mg/kg (SC), or with a vehicle control (SC). At sacrifice, sections of remaining kidney were analyzed by IHC for calcium staining using the von Kossa method. As shown in FIGS. 3A-B, kidney sections from the animals treated with Ac-c(C)arrrar-NH₂ (SEQ ID NO:3)(3 mg/kg) had less calcification than kidney sections from the control animals.

Male 5/6 Nx rats were dosed thrice-weekly for 6 weeks with Ac-c(C)arrrar-NH₂ (SEQ ID NO:3) at a dose of 3 mg/kg (SC). Control animals were untreated. At sacrifice, aortic and kidney tissue samples were removed and analyzed using atomic emission spectroscopy. As shown in FIG. 3C, aorta and kidney from animals treated with Ac-c(C)arrrar-NH₂ (SEQ ID NO:3) had substantially less calcium content than untreated animals.

### D. Renal Function

Male 5/6 Nx rats were dosed thrice-weekly for 6 weeks with Ac-c(C)arrrar-NH₂ (SEQ ID NO:3) at a dose of 0.3, 1 or 3 mg/kg (SC), or with a vehicle control (SC). Tail vein blood samples were taken periodically to measure serum creatinine, a marker of kidney function, using the QuantiChrom™ kit (DICT-500; BioAssay Systems, Hayward, CA).

The mean baseline creatinine level (pretreatment) ranged from about 1.06 mg/dL to about 1.09 mg/dL. Over the 6 weeks of the study, animals receiving vehicle showed a large increase in serum creatinine (FIG. 4). By contrast, the elevation in serum creatinine was suppressed in a dose-dependent manner over the six-week study in the animals treated with Ac-c(C)arrrar-NH₂ (SEQ ID NO:3).

### E. PTH Receptor Expression

Male Nx rats were dosed thrice-weekly for 6 weeks with Ac-c(C)arrrar-NH₂ (SEQ ID NO:3) at a dose of 3 mg/kg (SC). Age-matched normal rats were used as controls. At sacrifice, parathyroid glands from the 5/6 Nx rats and from the controls were removed and tissue sections were stained using antibodies to detect the CaSR, Vitamin D receptor and FGFR1 proteins.

Higher levels of CaSR (FIG. 5A), FGFR1 (FIG. 5B) and Vitamin D receptor (FIG. 5C) were seen in the parathyroid glands of animals treated with Ac-c(C)arrrar-NH₂ (SEQ ID NO:3) than in the parathyroid glands of normal controls.

### F. Baseline PTH After Washout

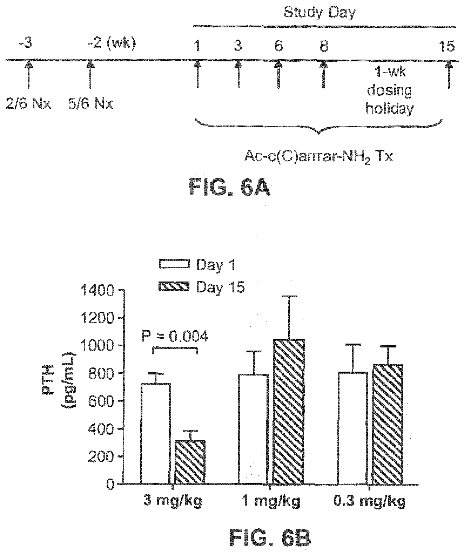

Male Nx rats were treated for 1 week with Ac-c(C)arrrar-NH₂ (SEQ ID NO:3), at a dose of 1 or 3 mg/kg (SC) on days 1, 3, 6 and 8 (FIG 6A). The dose on day 8 was followed by a 1 week washout.

As shown in FIG. 6B, rats receiving 3 mg/kg of Ac-c(C)arrrar-NH₂ (SEQ ID NO:3) had a 50% reduction in baseline PTH as measured after the 1-week washout.

### Example 2

### Disease Progression in Ac-c(C)rrarar-NH₂-Treated Animals

The therapeutic efficacy Ac-c(C)rrarar-NH₂, (SEQ ID NO:28) was assessed using an adenine-induced model of chronic renal failure in rats. The rats were obtained from Charles River Laboratories (Wilmington, MA). The rats were fed a low protein (2.5%), high phosphorus (0.92%) diet containing 0.75% adenine (Teklad Custom Diet). Animals were randomly assigned to receive daily subcutaneous doses of vehicle (10 mM succinic acid, 0.85% NaCl, 0.9% benzyl alcohol, pH 4.5) or Ac-c(C)rrarar-NH₂ (SEQ ID NO:28) at 0.3 or 1 mg/kg (SC) for 4 weeks. A control group was fed the identical high phosphorus diet and tissues without adenine. Treatment was initiated at the start of diet. All experimental procedures with animals were performed according to IACUC guidelines. Statistical analysis was performed using one-way ANOVA with Bonferroni post test. All p-values are nominal.

### A. PTH Suppression

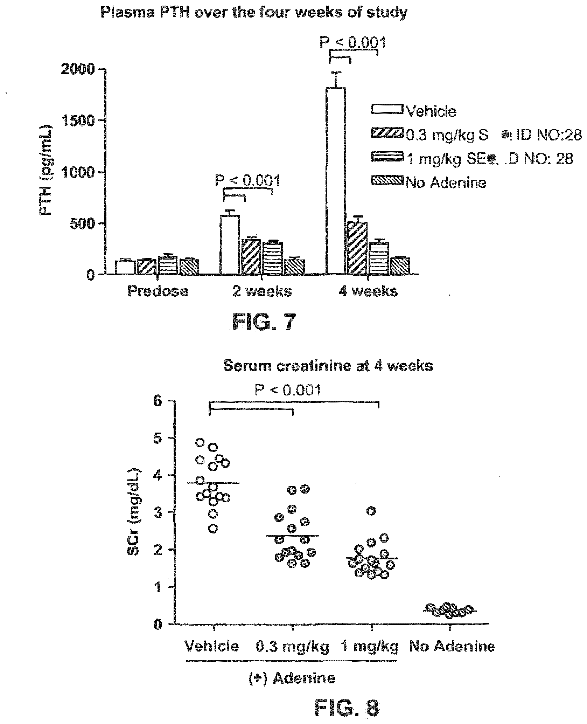

The effects of Ac-c(C)rrarar-NH₂ (SEQ ID NO:28) treatment on PTH levels in uremic rats is shown in FIG. 7. Plasma PTH was measured using the Immunotopics BioActive Intact ELISA (Immutopics, San Clemente, CA). Prior to dosing with Ac-c(C)rrarar-NH₂ (SEQ ID NO:28), all treatment groups had similar plasma PTH levels. The no-adenine-control animals maintained consistent plasma PTH levels over the 4 week study. PTH levels were significantly elevated over the course of the study for vehicle-treated uremic animals. Uremic animals treated with Ac-c(C)rrarar-NH₂ (SEQ ID NO:28) at a dose of 0.3 or 1 mg/kg for 2 and 4 weeks had significantly lower plasma PTH than vehicle-treated animals.

### B. Creatinine Suppression

The effects of Ac-c(C)rrarar-NH₂ (SEQ ID NO:28) treatment on serum creatinine levels are shown in FIG. 8. Serum creatinine was measured using QuantiChrom^{™} kit (BioAssay Systems, Hayward, CA). Serum creatinine levels from vehicle-treated uremic animals were approximately 8-10 fold higher compared to the control group of non-uremic animals at the end of the 4 weeks. Uremic animals treated with Ac-c(C)rrarar-NH₂ (SEQ ID NO:28) at doses of 0.3 and 1 mg/kg had significantly lower serum creatinine levels compared to vehicle-treated uremic rats. Uremic animals treated with Ac-c(C)rrarar-NH₂ (SEQ ID NO:28) at a dose of 1 mg/kg Ac-c(C)rrarar-NH₂ (SEQ ID NO:28) had approximately 50% lower serum creatinine levels compared with vehicle-treated controls.

### C. Phosphorus Suppression

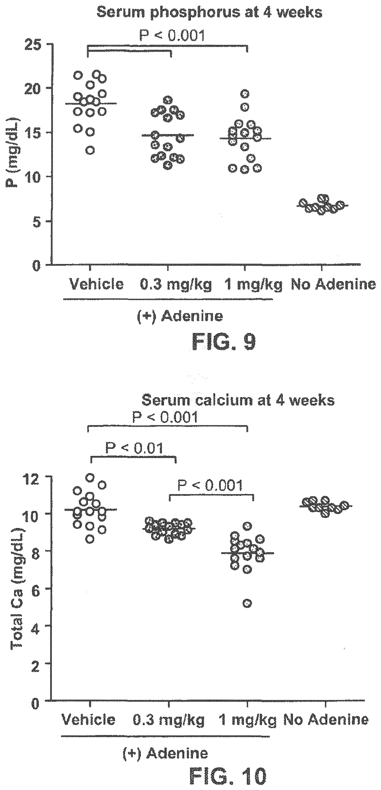

The effects of Ac-c(C)rrarar-NH₂ (SEQ ID NO:28)treatment on serum phosphorus levels are shown in FIG. 9. While serum phosphorus levels increased in uremic animals relative to non-uremic controls, uremic animals treated with Ac-c(C)rrarar-NH₂ (SEQ ID NO:28) at doses of 0.3 and 1 mg/kg for 4 weeks showed a significant reduction in serum phosphorus levels.

### D. Serum Calcium Suppression

The effects of Ac-c(C)rrarar-NH₂ (SEQ ID NO:28) treatment on serum calcium levels are shown in FIG. 10. While serum calcium levels in uremic rats were similar to calcium levels in control rats, uremic animals treated with Ac-c(C)rrarar-NH₂ (SEQ ID NO:28) at doses of 0.3 and 1 mg/kg for 4 weeks had a significant dose-dependent reduction in serum calcium compared to vehicle-treated rats.

### E. Serum Calcium-Phosphorus Suppression

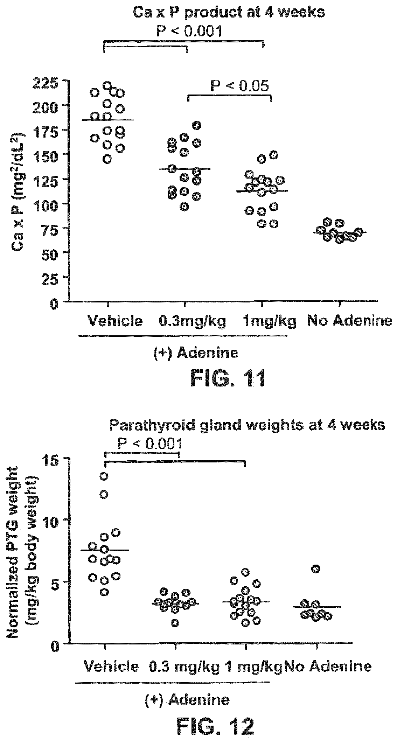

The effects of Ac-c(C)rrarar-NH₂ (SEQ ID NO:28) treatment on serum calcium-phosphorus product levels are shown in FIG. 11. Phosphorus and calcium were determined using a Cobas c-501 analyzer. Uremic animals treated with vehicle for 4 weeks had an approximate 2.5-fold increase in calcium-phosphorus product compared to non-uremic animals. Uremic animals treated with Ac-c(C)rrarar-NH₂ (SEQ ID NO:28) at doses of 0.3 mg/kg or 1 mg/kg for 4 weeks showed an approximate 20% and 40% reduction in serum calcium-phosphorus product, respectively.

### F. Parathyroid Gland Hyperplasia

The effects of Ac-c(C)rrarar-NH₂ (SEQ ID NO:28) treatment on parathyroid gland enlargement was also determined using the adenine-induced rat model of chronic renal failure. After treatment with Ac-c(C)rrarar-NH₂ (SEQ ID NO:28) at doses of 0.3 or 1 mg/kg for 4 weeks, the uremic rats were sacrificed and the parathyroid glands were removed, trimmed and weighed. Parathyroid glands in the groups treated with Ac-c(C)rrarar-NH₂ (SEQ ID NO:28) were significantly reduced in weight compared to the parathyroid glands from the group treated with vehicle. Parathyroid glands in the groups treated with Ac-c(C)rrarar-NH₂ (SEQ ID NO:28) were not significantly different in weight compared to the parathyroid glands from non-uremic animals.

### G. Vascular Calcification

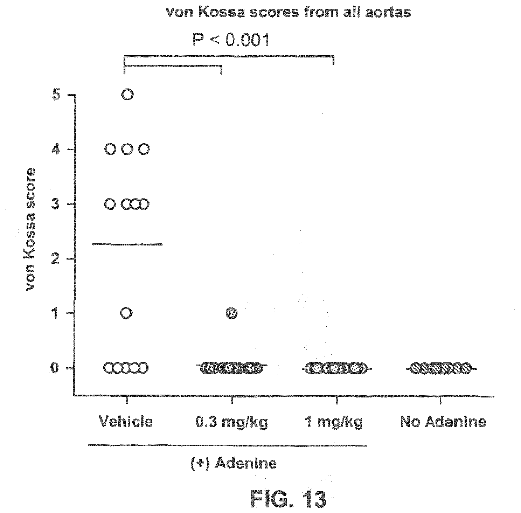

The effects of Ac-c(C)rrarar-NH₂ (SEQ ID NO:28) treatment of vascular calcification was also determined using adenine-induced rat model of chronic renal failure. After treatment with Ac-c(C)rrarar-NH₂ (SEQ ID NO:28) at doses of 0.3 or 1 mg/kg for 4 weeks, the uremic rats were sacrificed and aortic tissue was analyzed. Aortic tissue was processed and stained by Alizarin Red and von Kossa methods to visualize vascular mineralization. Using a scoring system from 0-5, identical sections of von Kossa-stained aorta from all animals were scored in a blinded fashion. Results of the Alizarin Red and Von Kossa staining showed that aortas from uremic rats treated with Ac-c(C)rrarar-NH₂ (SEQ ID NO:28) had little or no mineralization. As illustrated in FIG.13, 67% (10/15) of the uremic animals treated with vehicle demonstrated positive von Kossa staining, indicative of aortic mineralization. Uremic rats treated with Ac-c(C)rrarar-NH₂ (SEQ ID NO:28) demonstrated no appreciable von Kossa staining. Only one animal in the 0.3 mg/kg treatment group showed some minimal mineralization.

### Example 3

### Single Dosing of Ac-c(C)arrrar-NH₂ and Effects on iPTH and Calcium

A clinical study was performed to assess the safety and tolerability of rising single doses of Ac-c(C)arrrar-NH₂ (SEQ ID NO:3), administered by IV bolus to patients diagnosed with CKD-BMD and SHPT, and who were receiving hemodialysis. Data were generated to measure changes in patient serum intact PTH (iPTH) and serum calcium as well as bioavailability of the single dose of Ac-c(C)arrrar-NH₂ (SEQ ID NO:3).

A double-blind, randomized, placebo-controlled, multicenter study in subjects receiving thrice-weekly hemodialysis (HD) was designed and carried out. The major inclusion criteria included hemodialysis for at least 3 months prior to the start of the study, a serum iPTH level greater than 300 pg/mL, a serum cCa (corrected calcium) level greater than or equal to 9.0 mg/dL and receiving of stable doses of active vitamin D or analogs, phosphate binders, and calcium supplements.

Cohorts 1, 2 and 3 were conducted with a two-period crossover design. Each cohort enrolled 4 subjects randomized 1:1 to Ac-c(C)arrrar-NH₂ (SEQ ID NO:3) followed by placebo or placebo followed by Ac-c(C)arrrar-NH₂ (SEQ ID NO:3). The dose levels of Ac-c(C)arrrar-NH₂ (SEQ ID NO:3) for Cohorts 1-3 were 5, 10 and 20 mg. Cohort 4 enrolled 8 subjects who were randomized 1:1 to 40 mg Ac-c(C)arrrar-NH₂ (SEQ ID NO:3)or placebo in a parallel group. Cohort 5 enrolled 8 subjects were randomized 1:1 to 60 mg Ac-c(C)arrrar-NH₂ (SEQ ID NO:3) or placebo in a parallel group.

Subjects were admitted to a clinical research unit following the last HD session of the week and were observed for the 3-day interdialytic period prior to discharge for hemodialysis. Ac-c(C)arrrar-NH₂ (SEQ ID NO:3) or placebo was administered by IV bolus injection 2-4 hours following completion of HD.

Serum iPTH levels were determined using the electrochemiluminescence immunoassay (ECLIA) on the Roche Elecsys® analyzer. Calcium levels were adjusted for albumin <4 g/dL with the following equation: cCa =[measured Ca in mg/dL] + [4-(albumin in g/dL)] x 0.8

Adverse events were captured through 7 days after study drug administration.

### A. Geometric Mean PK Parameters Using Noncompartmental Analysis

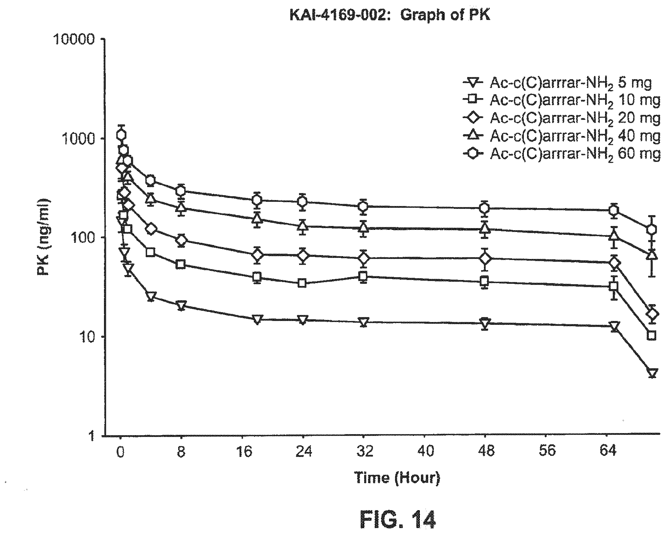

Pharmacokinetic (PK) analysis was performed on samples obtained from the treated subjects to determine bioavailability of the administered Ac-c(C)arrrar-NH₂ (SEQ ID NO:3). The results, presented below in Table 2 and in FIG. 14, show that systemic exposure increased in a dose-related manner over the dose range evaluation. The observed increase in Ac-c(C)arrrar-NH₂ (SEQ ID NO:3) total plasma exposure, as seen in the Cₘₐₓ and AUCₐₗₗ values, was reasonably proportional to the administered dose. The geometric mean terminal elimination half-life (81.7 h to 175 h) appeared to be reasonably comparable across the dose range evaluation. Clearance (CL) and volume of distribution as steady state (Vss) values appear to be dose-independent over the range evaluation. Total clearance of Ac-c(C)arrrar-NH₂ (SEQ ID NO:3) was approximately about 2 L/h. Ac-c(C)arrrar-NH₂ (SEQ ID NO:3) was readily cleared from the central compartment by hemodialysis. Hemodialysis clearance was estimated at about 33 L/h.

**Table 2**

| **Cohort** | **Dose (mg)** | **Cmax (µg/L)** | **AUCall (hr*µg/L)** | **T1/2 (hr)** | **CL (L/hr)** | **Vss (L)** |
|---|---|---|---|---|---|---|
| 1 | 5 | 133 | 1080 | 121 | 1.68 | 278 |
| 2 | 10 | 257 | 2770 | 81.7 | 1.91 | 203 |
| 3 | 20 | 479 | 4680 | 175 | 1.21 | 293 |
| 4 | 40 | 686 | 8890 | NC | NC | NC |
| 5 | 60 | 1080 | 14800 | 115 | 1.45 | 232 |

### B. Effects of Ac-c(C)arrrar-NH₂ on Serum iPTH and Corrected Calcium Levels

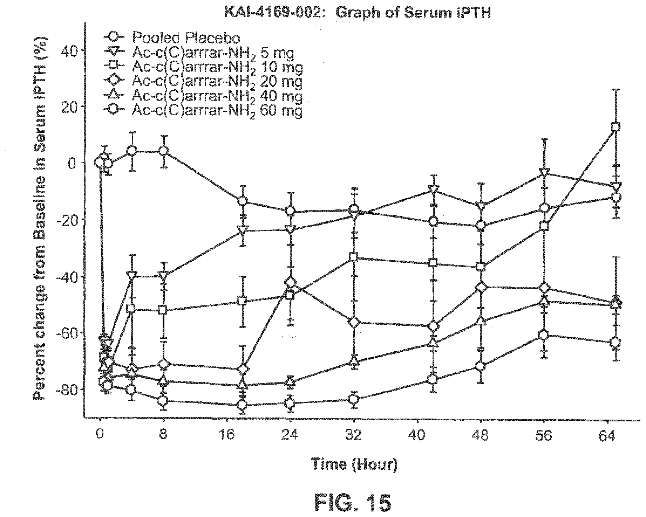

The effects of Ac-c(C)arrrar-NH₂ (SEQ ID NO:3) administration on serum iPTH and corrected calcium levels was also determined in this study. Subjects were administered a single IV bolus of Ac-c(C)arrrar-NH₂ (SEQ ID NO:3)2-4 hours after completion of hemodialysis. Blood from each subject was then analyzed to determine levels of iPTH and corrected calcium. The percent change in iPTH levels after Ac-c(C)arrrar-NH₂ (SEQ ID NO:3) administration is shown in FIG. 15. Treatment with Ac-c(C)arrrar-NH₂ (SEQ ID NO:3) at all doses resulted in dose-dependent decreases in iPTH. The changes occurred within 30 minutes after dosing. The duration of iPTH suppression was also dose-dependent with sustained reductions through the 3-day interdialytic period associated with Ac-c(C)arrrar-NH₂ (SEQ ID NO:3) doses which were greater than or equal to 20 mg.

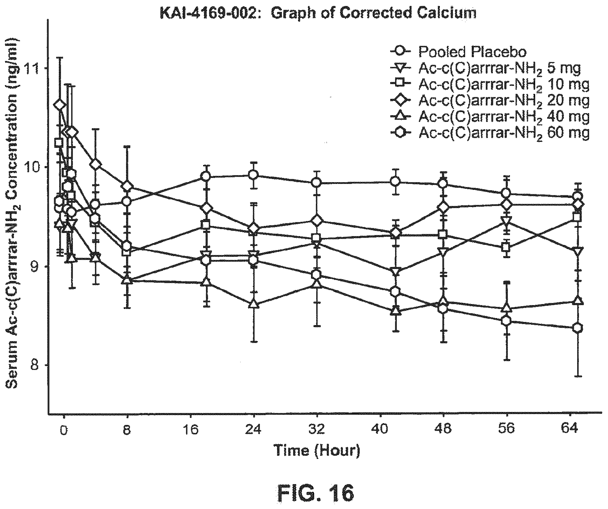

The effect of Ac-c(C)arrrar-NH₂ (SEQ ID NO:3) single dose administration on corrected calcium levels is shown in FIG. 16. The mean corrected calcium in the Ac-c(C)arrrar-NH₂ (SEQ ID NO:3) groups dosed with 10 mg, 20 mg, 40 mg or 60 mg was reduced up to about 10-14% during the observation period, with the largest mean decreases occurring in the 40 mg group. There were no apparent changes over time in mean corrected calcium in the placebo and the Ac-c(C)arrrar-NH₂ (SEQ ID NO:3) 5 mg groups.

### C. Safety Profile for the Administration of Ac-c(C)arrrar-NH₂

Adverse events experienced by each of the subjects was recorded for 7 days after administration of the Ac-c(C)arrrar-NH₂ (SEQ ID NO:3) or placebo. The data are presented in Table 3 below. A single serious adverse event occurred in one subject who had been treated with placebo in the 10 mg dose cohort. The subject discontinued form the study prior to receiving Ac-c(C)arrrar-NH₂ (SEQ ID NO:3). There were no reports of nausea, vomiting or diarrhea.

**Table 3**

| | **Dose Cohort** | | | | | |
|---|---|---|---|---|---|---|
| | Pooled Placebo | 5 mg | 10 mg | 20 mg | 40 mg | 60 mg |
| **Number of subjects dosed** | 20 | 4 | 3 | 4 | 4 | 4 |
| **Number of subjects with ≥ 1 AE** | 2 (10%) | 1 (25%) | 1 (33%) | 1 (25%) | 4 (100%) | 2 (50%) |

| **Adverse Event (Preferred Term)** | | | | | | |
|---|---|---|---|---|---|---|
| **Decreased ionized calcium** | 0 (0%) | 0 (0%) | 1 (33%) | 0 (0%) | 4 (100%) | 2 (50%) |
| **Paresthesias** | 1 (5%) | 1 (25%) | 0(0%) | 0(0%) | 1 (25%) | 0 (0%) |
| **Gastroesophageal reflux** | 0 (0%) | 1 (25%) | 0 (0%) | 0 (0%) | 0 (0%) | 0 (0%) |
| **Toothache** | 0 (0%) | 0 (0%) | 0 (0%) | 1 (25%) | 0 (0%) | 0 (0%) |
| **Anemia** | 1 (5%) | 0 (0%) | 0 (0%) | 0 (0%) | 0 (0%) | 0 (0%) |
| **Congestive heart failure** | 1 (5%) | 0 (0%) | 0 (0%) | 0 (0%) | 0 (0%) | 0 (0%) |
| **Injection site pruritis** | 0 (0%) | 0 (0%) | 0 (0%) | 0 (0%) | 1 (25%) | 0 (0%) |
| **Pneumonia** | 1 (5%) | 0 (0%) | 0 (0%) | 0 (0%) | 0 (0%) | 0 (0%) |

### Example 4

### Repeated Doses of Drug Clinical Trial

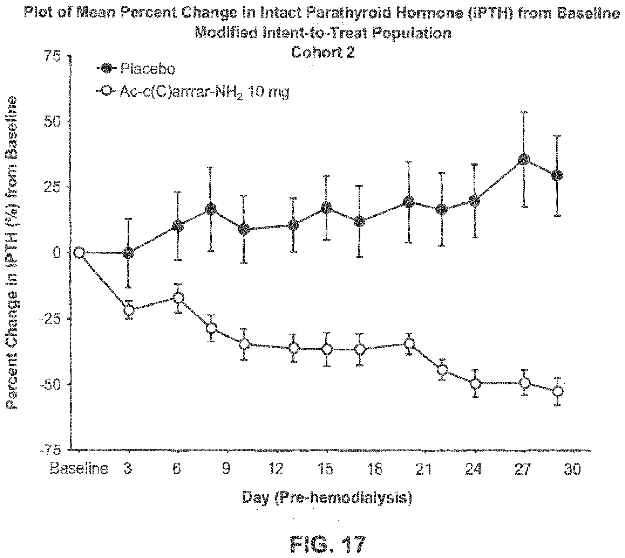

Hemodialysis patients were treated with 10 mg of Ac-c(C)arrrar-NH₂ (SEQ ID NO:3) 3 times per week for 4 weeks. Serum iPTH levels were measured at the time of drug trough, immediately before the subject received their next treatment of Ac-c(C)arrrar-NH₂ (SEQ ID NO:3). The data, presented in FIG. 17, shows that iPTH levels in subjects receiving treatment with Ac-c(C)arrrar-NH₂ (SEQ ID NO:3) steadily decreased over the 4-week period.

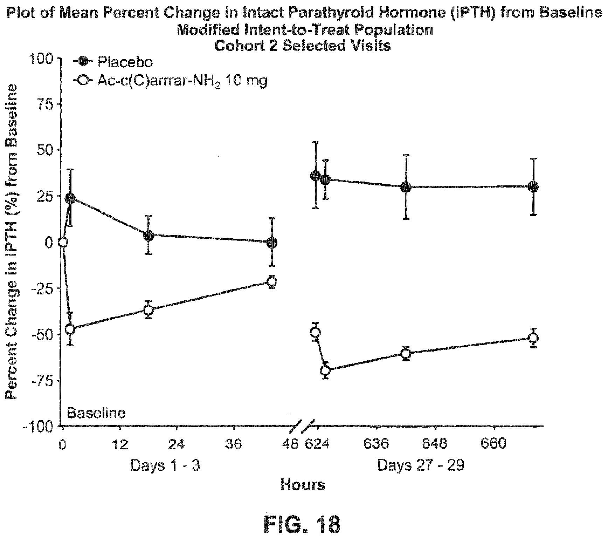

The data were also analyzed with respect to the percent change in serum PTH levels from baseline. As shown in FIG. 18, on the last day of Ac-c(C)arrrar-NH₂ (SEQ ID NO:3) dosing, the baseline level of serum iPTH is about 50% less than the level on the first day of dosing.

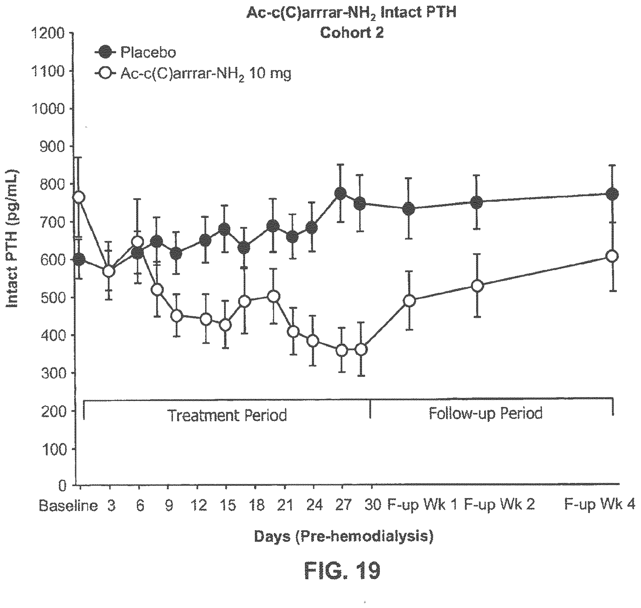

Serum samples from the subjects were further analyzed during a 4-week follow-up period following the final dosing with Ac-c(C)arrrar-NH₂ (SEQ ID NO:3). The data, presented in FIG. 19, show that levels of serum iPTH in subjects during the 4-week treatment period in which 10 mg/kg Ac-c(C)arrrar-NH₂ (SEQ ID NO:3) was administered and during a 4-week follow-up period.

## Claims

1. A composition comprising a therapeutically effective amount of the calcimimetic Ac-c(C)arrrar-NH2 (SEQ ID NO:3) or a pharmaceutically acceptable salt thereof for use in the treatment of chronic kidney disease-mineral bone disorder (CKD-MBD) **characterized by** soft tissue calcification in a subject, wherein the therapeutically effective amount of the calcimimetic reduces the amount of soft tissue calcification in the subject.

2. The composition for use of claim 1, wherein the soft tissue calcification is vascular calcification.

3. The composition for use of claim 1, wherein the composition is administered to CKD-MBD patients in end stage renal disease receiving hemodialysis, and wherein the composition is administered intravenously three times per week.

## Patentansprüche

1. Zusammensetzung, umfassend eine therapeutisch wirksame Menge des Calcimimetikums Ac-c(C)arrrar-NH2 (SEQ ID NO:3) oder ein pharmazeutisch verträgliches Salz davon zur Verwendung bei der Behandlung einer Störung des Mineral- und Knochenstoffwechsels bei chronischer Nierenerkrankung (CKD-MBD), **gekennzeichnet durch** eine Verkalkung der Weichteile bei einem Individuum, wobei die therapeutisch wirksame Menge des Calcimimetikums den Umfang der Verkalkung der Weichteile bei dem Individuum verringert.

2. Zusammensetzung zur Verwendung nach Anspruch 1, wobei die Verkalkung der Weichteile eine Gefäßverkalkung ist.

3. Zusammensetzung zur Verwendung nach Anspruch 1, wobei die Zusammensetzung an CKD-MBD-Patienten mit einer Nierenerkrankung im Endstadium verabreicht wird, die eine Hämodialyse erhalten und wobei die Zusammensetzung dreimal pro Woche intravenös verabreicht wird.

## Revendications

1. Composition comprenant une quantité thérapeutiquement efficace du calcimimétique Ac-c(C)arrrar-NH2 (SEQ ID NO : 3) ou d'un sel pharmaceutiquement acceptable de celui-ci pour son utilisation dans le traitement des troubles minéraux et osseux de la maladie rénale chronique (TMO-MRC) **caractérisés par** une calcification des tissus mous chez un sujet, dans laquelle la quantité thérapeutiquement efficace du calcimimétique réduit la quantité de calcification des tissus mous chez le sujet.

2. Composition pour son utilisation selon la revendication 1, dans laquelle la calcification des tissus mous est une calcification vasculaire.

3. Composition pour son utilisation selon la revendication 1, dans laquelle la composition est administrée à des patients atteints de TMO-MRC souffrant d'une maladie rénale en phase terminale et recevant une hémodialyse, et dans laquelle la composition est administrée par voie intraveineuse trois fois par semaine.


### SEQUENCE LISTING

<110> Walter, Sarah Tomlinson, James E. Bell, Gregory
<120> Therapeutic Agents for Regulating Serum PHosphorus
<130> 63200.8022.wo00
<140> Not yet assigned
   <141> Filed herewith
<150> US 61/558,389
   <151> 2011-11-10
<160> 183
<170> PatentIn version 3.4
<210> 1
   <211> 7
   <212> PRT
   <213> Artificial Sequence
<220>
   <223> Synthetic peptide
<220>
   <221> VARIANT
   <222> (1)...(7)
   <223> Xaa = any amino acid as defined in the specification filed herewith
<400> 1
   Xaa Xaa Xaa Xaa Xaa Xaa Xaa
   1 5
<210> 2
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> VARIANT
   <222> (2), (6)
   <223> Xaa = D-Ala
<220>
   <221> VARIANT
   <222> (3), (4), (5), (7)
   <223> Xaa = D-Arg
<400> 2
   Xaa Xaa Xaa Xaa Xaa Xaa Xaa
   1 5
<210> 3
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Cys-Cys disulfide bond to Cys
<220>
   <221> VARIANT
   <222> (2), (6)
   <223> Xaa = D-Ala
<220>
   <221> VARIANT
   <222> (3), (4), (5), (7)
   <223> Xaa = D-Arg
<220>
   <221> MOD_RES
   <222> (7)
   <223> C-terminal amidation
<400> 3
   Xaa Xaa Xaa Xaa Xaa Xaa Xaa
   1               5
<210> 4
   <211> 5
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2)...(5)
   <223> Xaa = D-Arg
<220>
   <221> MOD_RES
   <222> (5)
   <223> C-terminal amidation
<400> 4
   Xaa Xaa Xaa Xaa Xaa
   1               5
<210> 5
   <211> 6
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2)..(6)
   <223> Xaa = D-Arg
<220>
   <221> MOD_RES
   <222> (6)
   <223> C-terminal amidation
<400> 5
   Xaa Xaa Xaa Xaa Xaa Xaa
   1               5
<210> 6
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2)..(7)
   <223> Xaa = D-Arg
<220>
   <221> MOD_RES
   <222> (7)
   <223> C-terminal amidation
<400> 6
   Xaa Xaa Xaa Xaa Xaa Xaa Xaa
   1               5
<210> 7
   <211> 8
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2)..(8)
   <223> Xaa = D-Arg
<220>
   <221> MOD_RES
   <222> (8)
   <223> C-terminal amidation
<400> 7
   Xaa Xaa Xaa Xaa Xaa Xaa Xaa Xaa
   1               5
<210> 8
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2)..(2)
   <223> Xaa = D-Ala
<220>
   <221> VARIANT
   <222> (3)...(7)
   <223> Xaa = D-Arg
<220>
   <221> MOD_RES
   <222> (7)
   <223> C-terminal amidation
<400> 8
   Xaa Xaa Xaa Xaa Xaa Xaa Xaa
   1               5
<210> 9
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2)..(2)
   <223> Xaa = D-Arg
<220>
   <221> VARIANT
   <222> (3)..(3)
   <223> Xaa = D-Ala
<220>
   <221> VARIANT
   <222> (4)...(7)
   <223> Xaa = D-Arg
<220>
   <221> MOD_RES
   <222> (7)
   <223> C-terminal amidation
<400> 9
   Xaa Xaa Xaa Xaa Xaa Xaa Xaa
   1               5
<210> 10
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2), (3), (5)...(7)
   <223> Xaa = D-Arg
<220>
   <221> VARIANT
   <222> (4)..(4)
   <223> Xaa = D-Ala
<220>
   <221> MOD_RES
   <222> (7)
   <223> C-terminal amidation
<400> 10
   Xaa Xaa Xaa Xaa Xaa Xaa Xaa
   1               5
<210> 11
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2)...(4), (6)...(7)
   <223> Xaa = D-Arg
<220>
   <221> VARIANT
   <222> (5)..(5)
   <223> Xaa = D-Ala
<220>
   <221> MOD_RES
   <222> (7)
   <223> C-terminal amidation
<400> 11
   Xaa Xaa Xaa Xaa Xaa Xaa Xaa
   1 5
<210> 12
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2)...(5), (7)
   <223> Xaa = D-Arg
<220>
   <221> VARIANT
   <222> (6)..(6)
   <223> Xaa = D-Ala
<220>
   <221> MOD_RES
   <222> (7)
   <223> C-terminal amidation
<400> 12
   Xaa Xaa Xaa Xaa Xaa Xaa Xaa
   1 5
<210> 13
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2)...(6)
   <223> Xaa = D-Arg
<220>
   <221> VARIANT
   <222> (7)..(7)
   <223> Xaa = D-Ala
<220>
   <221> MOD_RES
   <222> (7)
   <223> C-terminal amidation
<400> 13
   Xaa Xaa Xaa Xaa Xaa Xaa Xaa
    1               5
<210> 14
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2), (3), (5), (6)
   <223> Xaa = D-Arg
<220>
   <221> VARIANT
   <222> (4), (7)
   <223> Xaa = D-Ala
<220>
   <221> MOD_RES
   <222> (7)
   <223> C-terminal amidation
<400> 14
   Xaa Xaa Xaa Xaa Xaa Xaa Xaa
   1 5
<210> 15
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2), (4)
   <223> Xaa = D-Ala
<220>
   <221> VARIANT
   <222> (3), (5) (6), (7)
   <223> Xaa = D-Arg
<220>
   <221> MOD_RES
   <222> (7)
   <223> C-terminal amidation
<400> 15
   Xaa Xaa Xaa Xaa Xaa Xaa Xaa
   1 5
<210> 16
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2), (5)
   <223> Xaa = D-Ala
<220>
   <221> VARIANT
   <222> (3), (4), (6), (7)
   <223> Xaa = D-Arg
<220>
   <221> MOD_RES
   <222> (7)
   <223> C-terminal amidation
<400> 16
   Xaa Xaa Xaa Xaa Xaa Xaa Xaa
   1               5
<210> 17
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2), (3), (6), (7)
   <223> Xaa = D-Arg
<220>
   <221> VARIANT
   <222> (4), (5)
   <223> Xaa = D-Ala
<220>
   <221> MOD_RES
   <222> (7)
   <223> C-terminal amidation
<400> 17
   Xaa Xaa Xaa Xaa Xaa Xaa Xaa
   1               5
<210> 18
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2), (4), (6), (7)
   <223> Xaa = D-Arg
<220>
   <221> VARIANT
   <222> (3), (5)
   <223> Xaa = D-Ala
<220>
   <221> MOD_RES
   <222> (7)
   <223> C-terminal amidation
<400> 18
   Xaa Xaa Xaa Xaa Xaa Xaa Xaa
   1               5
<210> 19
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2), (7)
   <223> Xaa = D-Ala
<220>
   <221> VARIANT
   <222> (3)...(6)
   <223> Xaa = D-Arg
<220>
   <221> MOD_RES
   <222> (7)
   <223> C-terminal amidation
<400> 19
   Xaa Xaa Xaa Xaa Xaa Xaa Xaa
   1 5
<210> 20
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2), (4), (5), (6)
   <223> Xaa = D-Arg
<220>
   <221> VARIANT
   <222> (3), (7)
   <223> Xaa = D-Ala
<220>
   <221> MOD_RES
   <222> (7)
   <223> C-terminal amidation
<400> 20
   Xaa Xaa Xaa Xaa Xaa Xaa Xaa
   1               5
<210> 21
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2), (3), (4), (7)
   <223> Xaa = D-Arg
<220>
   <221> VARIANT
   <222> (5), (6)
   <223> Xaa = D-Ala
<220>
   <221> MOD_RES
   <222> (7)
   <223> C-terminal amidation
<400> 21
   Xaa Xaa Xaa Xaa Xaa Xaa Xaa
   1               5
<210> 22
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2)...(3)
   <223> Xaa = D-Ala
<220>
   <221> VARIANT
   <222> (4)...(7)
   <223> Xaa = D-Arg
<220>
   <221> MOD_RES
   <222> (7)
   <223> C-terminal amidation
<400> 22
   Xaa Xaa Xaa Xaa Xaa Xaa Xaa
    1               5
<210> 23
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2), (4), (5), (7)
   <223> Xaa = D-Arg
<220>
   <221> VARIANT
   <222> (3), (6)
   <223> Xaa = D-Ala
<220>
   <221> MOD_RES
   <222> (7)
   <223> C-terminal amidation
<400> 23
   Xaa Xaa Xaa Xaa Xaa Xaa Xaa
   1               5
<210> 24
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2), (5), (6), (7)
   <223> Xaa = D-Arg
<220>
   <221> VARIANT
   <222> (3), (4)
   <223> Xaa = D-Ala
<220>
   <221> MOD_RES
   <222> (7)
   <223> C-terminal amidation
<400> 24
   Xaa Xaa Xaa Xaa Xaa Xaa Xaa
   1               5
<210> 25
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4), (6)
   <223> Xaa = D-Ala
<220>
   <221> MOD_RES
   <222> (7)
   <223> C-terminal amidation
<400> 25
   Xaa Xaa Xaa Xaa Xaa Xaa Xaa
   1               5
<210> 26
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> VARIANT
   <222> (2), (6)
   <223> Xaa = D-Ala
<220>
   <221> VARIANT
   <222> (3), (4), (5), (7)
   <223> Xaa = D-Arg
<220>
   <221> MOD_RES
   <222> (7)
   <223> C-terminal amidation
<400> 26
   Xaa Xaa Xaa Xaa Xaa Xaa Xaa
   1 5
<210> 27
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Cys-Cys disulfide bond to Cys
<220>
   <221> VARIANT
   <222> (2), (6)
   <223> Xaa = D-Ala
<220>
   <221> VARIANT
   <222> (3), (4), (5), (7)
   <223> Xaa = D-Arg
<220>
   <221> MOD_RES
   <222> (7)
   <223> C-terminal amidation
<400> 27
   Xaa Xaa Xaa Xaa Xaa Xaa Xaa
   1 5
<210> 28
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Cys-Cys disulfide bond to Cys
<220>
   <221> VARIANT
   <222> (2), (3), (5), (7)
   <223> Xaa = D-Arg
<220>
   <221> VARIANT
   <222> (4), (6)
   <223> Xaa = D-Ala
<220>
   <221> MOD_RES
   <222> (7)
   <223> C-terminal amidation
<400> 28
   Xaa Xaa Xaa Xaa Xaa Xaa Xaa
    1               5
<210> 29
   <211> 6
   <212> PRT
   <213> Artificial Sequence
<220>
   <223> Synthetic peptide
<220>
   <221> VARIANT
   <222> (1), (5)
   <223> Xaa = D-Ala
<220>
   <221> MOD_RES
   <222> (1)..(1)
   <223> N-terminal acetylation
<220>
   <221> VARIANT
   <222> (2), (3), (4), (6)
   <223> Xaa = D-Arg
<220>
   <221> MOD_RES
   <222> (7)
   <223> C-terminal amidation
<400> 29
   Xaa Xaa Xaa Xaa Xaa Xaa
   1 5
<210> 30
   <211> 8
   <212> PRT
   <213> Artificial Sequence
<220>
   <223> Synthetic peptide
<220>
   <221> VARIANT
   <222> (1)..(1)
   <223> Xaa = bAla
<220>
   <221> MOD_RES
   <222> (1)..(1)
   <223> N-terminal acetylation
<220>
   <221> VARIANT
   <222> (2)
   <223> Xaa = D-Cys
<220>
   <221> VARIANT
   <222> (3)...(8)
   <223> Xaa = D-Arg
<220>
   <221> MOD_RES
   <222> (8)
   <223> C-terminal amidation
<400> 30
   Xaa Xaa Xaa Xaa Xaa Xaa Xaa Xaa
   1 5
<210> 31
   <211> 7
   <212> PRT
   <213> Artificial Sequence
<220>
   <223> Synthetic peptide
<220>
   <221> VARIANT
   <222> (1)..(1)
   <223> Xaa = 3-Mercaptopropionic acid
<220>
   <221> VARIANT
   <222> (2)...(7)
   <223> Xaa = D-Arg
<220>
   <221> MOD_RES
   <222> (7)
   <223> C-terminal amidation
<400> 31
   Xaa Xaa Xaa Xaa Xaa Xaa Xaa
   1 5
<210> 32
   <211> 7
   <212> PRT
   <213> Artificial Sequence
<220>
   <223> Synthetic peptide
<220>
   <221> VARIANT
   <222> (1)..(1)
   <223> Xaa = D-Homocysteine
<220>
   <221> MOD_RES
   <222> (1)..(1)
   <223> N-terminal acetylation
<220>
   <221> VARIANT
   <222> (2)...(7)
   <223> Xaa = D-Arg
<220>
   <221> MOD_RES
   <222> (7)
   <223> C-terminal amidation
<400> 32
   Xaa Xaa Xaa Xaa Xaa Xaa Xaa
   1 5
<210> 33
   <211> 7
   <212> PRT
   <213> Artificial Sequence
<220>
   <223> Synthetic peptide
<220>
   <221> VARIANT
   <222> (1)..(1)
   <223> Xaa = D-penicillamine
<220>
   <221> MOD_RES
   <222> (1)..(1)
   <223> N-terminal acetylation
<220>
   <221> VARIANT
   <222> (2)...(7)
   <223> Xaa = D-Arg
<220>
   <221> MOD_RES
   <222> (7)
   <223> C-terminal amidation
<400> 33
   Xaa Xaa Xaa Xaa Xaa Xaa Xaa
   1               5
<210> 34
   <211> 7
   <212> PRT
   <213> Artificial Sequence
<220>
   <223> Synthetic peptide
<220>
   <221> VARIANT
   <222> (1)..(1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Cys-Cys interchain disulfide bond
<220>
   <221> VARIANT
   <222> (2), (6)
   <223> Xaa = D-Ala
<220>
   <221> VARIANT
   <222> (3), (4), (5), (7)
   <223> Xaa = D-Arg
<220>
   <221> MOD_RES
   <222> (7)
   <223> C-terminal amidation
<400> 34
   Xaa Xaa Xaa Xaa Xaa Xaa Xaa
   1 5
<210> 35
   <211> 7
   <212> PRT
   <213> Artificial Sequence
<220>
   <223> Synthetic peptide
<220>
   <221> MOD_RES
   <222> (1)..(1)
   <223> N-terminal acetylation
<220>
   <221> DISULFID
   <222> (1)..(1)
   <223> Cys-Cys disulfide bond to Cys
<220>
   <221> VARIANT
   <222> (2), (6)
   <223> Xaa = D-Ala
<220>
   <221> VARIANT
   <222> (3), (4), (5), (7)
   <223> Xaa = D-Arg
<220>
   <221> MOD_RES
   <222> (7)
   <223> C-terminal amidation
<400> 35
   Cys Xaa Xaa Xaa Xaa Xaa Xaa
   1 5
<210> 36
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Cys-Cys disulfide bond to Cys
<220>
   <221> VARIANT
   <222> (2), (6)
   <223> Xaa = D-Ala
<220>
   <221> VARIANT
   <222> (4), (5), (7)
   <223> Xaa = D-Arg
<220>
   <221> MOD_RES
   <222> (7)
   <223> C-terminal amidation
<400> 36
   Xaa Xaa Arg Xaa Xaa Xaa Xaa
   1               5
<210> 37
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Cys-Cys disulfide bond to Cys
<220>
   <221> VARIANT
   <222> (2), (6)
   <223> Xaa = D-Ala
<220>
   <221> VARIANT
   <222> (3), (5), (7)
   <223> Xaa = D-Arg
<220>
   <221> MOD_RES
   <222> (7)
   <223> C-terminal amidation
<400> 37
   Xaa Xaa Xaa Arg Xaa Xaa Xaa
   1               5
<210> 38
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Cys-Cys disulfide bond to Cys
<220>
   <221> VARIANT
   <222> (2), (6)
   <223> Xaa = D-Ala
<220>
   <221> VARIANT
   <222> (3), (4), (7)
   <223> Xaa = D-Arg
<220>
   <221> MOD_RES
   <222> (7)
   <223> C-terminal amidation
<400> 38
   Xaa Xaa Xaa Xaa Arg Xaa Xaa
   1               5
<210> 39
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Cys-Cys disulfide bond to Cys
<220>
   <221> VARIANT
   <222> (2)
   <223> Xaa = D-Ala
<220>
   <221> VARIANT
   <222> (3), (4), (5), (7)
   <223> Xaa = D-Arg
<220>
   <221> MOD_RES
   <222> (7)
   <223> C-terminal amidation
<400> 39
   Xaa Xaa Xaa Xaa Xaa Ala Xaa
   1               5
<210> 40
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Cys-Cys disulfide bond to Cys
<220>
   <221> VARIANT
   <222> (2)
   <223> Xaa = D-Ala
<220>
   <221> VARIANT
   <222> (3), (4), (5), (6)
   <223> Xaa = D-Arg
<220>
   <221> MOD_RES
   <222> (7)
   <223> C-terminal amidation
<400> 40
   Xaa Xaa Xaa Xaa Xaa Xaa Arg
   1               5
<210> 41
   <211> 9
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)...(9)
   <223> Xaa = D-Arg
<220>
   <221> MOD_RES
   <222> (7)
   <223> C-terminal amidation
<400> 41
   Xaa Xaa Xaa Xaa Xaa Xaa Xaa Xaa Xaa
   1 5
<210> 42
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (3), (4), (5), (7)
   <223> Xaa = D-Arg
<220>
   <221> MOD_RES
   <222> (7)
   <223> C-terminal amidation
<400> 42
   Xaa Gly Xaa Xaa Xaa Gly Xaa
   1 5
<210> 43
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (3), (4), (5), (7)
   <223> Xaa = D-Arg
<220>
   <221> MOD_RES
   <222> (7)
   <223> C-terminal amidation
<400> 43
   Xaa Ala Xaa Xaa Xaa Ala Xaa
   1 5
<210> 44
   <211> 7
   <212> PRT
   <213> Artificial Sequence
<220>
   <223> Synthetic peptide
<220>
   <221> MOD_RES
   <222> (1)..(1)
   <223> N-terminal acetylation
<220>
   <221> VARIANT
   <222> (4)
   <223> Xaa = D-Arg
<220>
   <221> VARIANT
   <222> (2), (6)
   <223> Xaa = D-Ala
<220>
   <221> MOD_RES
   <222> (7)
   <223> C-terminal amidation
<400> 44
   Cys Xaa Arg Xaa Arg Xaa Arg
   1 5
<210> 45
   <211> 9
   <212> PRT
   <213> Artificial Sequence
<220>
   <223> Synthetic peptide
<400> 45
   Cys His Asp Ala Pro Ile Gly Tyr Asp
   1 5
<210> 46
   <211> 9
   <212> PRT
   <213> Artificial Sequence
<220>
   <223> Synthetic peptide
<400> 46
   Cys Pro Asp Tyr His Asp Ala Gly Ile
   1 5
<210> 47
   <211> 12
   <212> PRT
   <213> Artificial Sequence
<220>
   <223> Synthetic peptide
<220>
   <221> MOD_RES
   <222> (1)..(1)
   <223> N-terminal acetylation
<220>
   <221> MOD_RES
   <222> (12)..(12)
   <223> C-terminal amidation
<400> 47
   Cys Tyr Gly Arg Lys Lys Arg Arg Gln Arg Arg Arg
   1 5 10
<210> 48
   <211> 11
   <212> PRT
   <213> Artificial Sequence
<220>
   <223> Synthetic peptide
<220>
   <221> MOD_RES
   <222> (1)..(1)
   <223> N-terminal acetylation
<220>
   <221> MOD_RES
   <222> (11)..(11)
   <223> C-terminal amidation
<400> 48
   Tyr Gly Arg Lys Lys Arg Arg Gln Arg Arg Arg
   1 5 10
<210> 49
   <211> 8
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (4), (5)
   <223> Xaa = D-Ala
<220>
   <221> VARIANT
   <222> (3), (6), (7), (8)
   <223> Xaa = D-Arg
<220>
   <221> MOD_RES
   <222> (8)
   <223> C-terminal amidation
<400> 49
   Xaa Xaa Xaa Xaa Xaa Xaa Xaa Xaa
    1               5
<210> 50
   <211> 12
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Tyr
<220>
   <221> VARIANT
   <222> (3)
   <223> Xaa = D-Gly
<220>
   <221> VARIANT
   <222> (4), (7), (8), (10), (11), (12)
   <223> Xaa = D-Arg
<220>
   <221> VARIANT
   <222> (5), (6)
   <223> Xaa = D-Lys
<220>
   <221> VARIANT
   <222> (9)
   <223> Xaa = D-Gln
<220>
   <221> MOD_RES
   <222> (12)
   <223> C-terminal amidation
<400> 50
   Xaa Xaa Xaa Xaa Xaa Xaa Xaa Xaa Xaa Xaa Xaa Xaa
    1               5                   10
<210> 51
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)...(7)
   <223> Xaa = D-Arg
<220>
   <221> MOD_RES
   <222> (7)
   <223> C-terminal amidation
<400> 51
   Xaa Xaa Xaa Xaa Xaa Xaa Xaa
   1 5
<210> 52
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (6)
   <223> Xaa = D-Ala
<220>
   <221> VARIANT
   <222> (3), (4), (5), (7)
   <223> Xaa = D-Arg
<220>
   <221> MOD_RES
   <222> (7)
   <223> C-terminal amidation
<400> 52
   Xaa Xaa Xaa Xaa Xaa Xaa Xaa
   1 5
<210> 53
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Cys-Cys disulfide bonded to Glutathione
<220>
   <221> VARIANT
   <222> (2)...(7)
   <223> Xaa = D-Arg
<220>
   <221> MOD_RES
   <222> (7)
   <223> C-terminal amidation
<400> 53
   Xaa Xaa Xaa Xaa Xaa Xaa Xaa
   1               5
<210> 54
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> DISULFID
   <222> (1)..(1)
   <223> Cys-Cys disulfide bonded to Glutathione
<220>
   <221> VARIANT
   <222> (2)...(7)
   <223> Xaa = D-Arg
<400> 54
   Xaa Xaa Xaa Xaa Xaa Xaa Xaa
   1               5
<210> 55
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Cys-Cys disulfide bonded to acetylated Glutathione
<220>
   <221> VARIANT
   <222> (2), (6)
   <223> Xaa = D-Ala
<220>
   <221> VARIANT
   <222> (3), (4), (5), (7)
   <223> Xaa = D-Arg
<220>
   <221> MOD_RES
   <222> (7)
   <223> C-terminal amidation
<400> 55
   Xaa Xaa Xaa Xaa Xaa Xaa Xaa
   1               5
<210> 56
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Cys-Cys disulfide bonded to 3-Mercaptopropionic acid
<220>
   <221> VARIANT
   <222> (2), (6)
   <223> Xaa = D-Ala
<220>
   <221> VARIANT
   <222> (3), (4), (5), (7)
   <223> Xaa = D-Arg
<220>
   <221> MOD_RES
   <222> (7)
   <223> C-terminal amidation
<400> 56
   Xaa Xaa Xaa Xaa Xaa Xaa Xaa
   1 5
<210> 57
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Cys-Cys disulfide bonded to Polyethylene glycol
<220>
   <221> VARIANT
   <222> (2), (6)
   <223> Xaa = D-Ala
<220>
   <221> VARIANT
   <222> (3), (4), (5), (7)
   <223> Xaa = D-Arg
<220>
   <221> MOD_RES
   <222> (7)
   <223> C-terminal amidation
<400> 57
   Xaa Xaa Xaa Xaa Xaa Xaa Xaa
   1 5
<210> 58
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Cys-Cys disulfide bonded to Polyethylene glycol
<220>
   <221> VARIANT
   <222> (2), (6)
   <223> Xaa = D-Ala
<220>
   <221> VARIANT
   <222> (2)...(7)
   <223> Xaa = D-Arg
<220>
   <221> MOD_RES
   <222> (7)
   <223> C-terminal amidation
<400> 58
   Xaa Xaa Xaa Xaa Xaa Xaa Xaa
   1 5
<210> 59
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Cys-Cys disulfide bonded to Polyethylene glycol
<220>
   <221> VARIANT
   <222> (2)...(7)
   <223> Xaa = D-Arg
<220>
   <221> MOD_RES
   <222> (7)
   <223> C-terminal amidation
<400> 59
   Xaa Xaa Xaa Xaa Xaa Xaa Xaa
    1               5
<210> 60
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Cys-Cys disulfide bonded to Cys
<220>
   <221> VARIANT
   <222> (2), (6)
   <223> Xaa = D-Ala
<220>
   <221> VARIANT
   <222> (3), (4), (5), (7)
   <223> Xaa = D-Arg
<220>
   <221> MOD_RES
   <222> (7)
   <223> C-terminal amidation
<400> 60
   Xaa Xaa Xaa Xaa Xaa Xaa Xaa
   1               5
<210> 61
   <211> 8
   <212> PRT
   <213> Artificial Sequence
<220>
   <223> Synthetic peptide
<220>
   <221> VARIANT
   <222> (1)
   <223> Xaa = bAla
<220>
   <221> MOD_RES
   <222> (1)..(1)
   <223> N-terminal acetylation
<220>
   <221> VARIANT
   <222> (2)
   <223> Xaa = D-Cys
<220>
   <221> DISULFID
   <222> (2)..(2)
   <223> Cys-Cys disulfide bonded to Cys
<220>
   <221> VARIANT
   <222> (3), (7)
   <223> Xaa = D-Ala
<220>
   <221> VARIANT
   <222> (4), (5), (6), (8)
   <223> Xaa = D-Arg
<220>
   <221> MOD_RES
   <222> (7)
   <223> C-terminal amidation
<400> 61
   Xaa Xaa Xaa Xaa Xaa Xaa Xaa Xaa
   1               5
<210> 62
   <211> 8
   <212> PRT
   <213> Artificial Sequence
<220>
   <223> Synthetic peptide
<220>
   <221> VARIANT
   <222> (1)
   <223> Xaa = bAla
<220>
   <221> VARIANT
   <222> (2)
   <223> Xaa = D-Cys
<220>
   <221> DISULFID
   <222> (2)..(2)
   <223> Cys-Cys disulfide bonded to Cys
<220>
   <221> VARIANT
   <222> (3), (7)
   <223> Xaa = D-Ala
<220>
   <221> VARIANT
   <222> (4), (5), (6), (8)
   <223> Xaa = D-Arg
<400> 62
   Xaa Xaa Xaa Xaa Xaa Xaa Xaa Xaa
   1               5
<210> 63
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (3), (4), (5), (7)
   <223> Xaa = D-Arg
<400> 63
   Xaa Gly Xaa Xaa Xaa Gly Xaa
   1               5
<210> 64
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (3), (4), (5), (7)
   <223> Xaa = D-Arg
<400> 64
   Xaa Ala Xaa Xaa Xaa Ala Xaa
   1               5
<210> 65
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (6)
   <223> Xaa = D-Val
<220>
   <221> VARIANT
   <222> (3), (4), (5), (7)
   <223> Xaa = D-Arg
<220>
   <221> MOD_RES
   <222> (7)
   <223> C-terminal amidation
<400> 65
   Xaa Xaa Xaa Xaa Xaa Xaa Xaa
   1               5
<210> 66
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (6)
   <223> Xaa = D-Val
<220>
   <221> VARIANT
   <222> (3), (4), (5), (7)
   <223> Xaa = D-Arg
<400> 66
   Xaa Xaa Xaa Xaa Xaa Xaa Xaa
   1               5
<210> 67
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)...(7)
   <223> Xaa = D-Arg
<220>
   <221> MOD_RES
   <222> (7)
   <223> C-terminal amidation
<400> 67
   Xaa Xaa Xaa Xaa Xaa Xaa Xaa
    1               5
<210> 68
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Ala
<220>
   <221> VARIANT
   <222> (3), (4), (5), (7)
   <223> Xaa = D-Arg
<220>
   <221> VARIANT
   <222> (6)
   <223> Xaa = D-Glu
<220>
   <221> MOD_RES
   <222> (7)
   <223> C-terminal amidation
<400> 68
   Xaa Xaa Xaa Xaa Xaa Xaa Xaa
   1               5
<210> 69
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Glu
<220>
   <221> VARIANT
   <222> (3), (4), (5), (7)
   <223> Xaa = D-Arg
<220>
   <221> VARIANT
   <222> (6)
   <223> Xaa = D-Ala
<220>
   <221> MOD_RES
   <222> (7)
   <223> C-terminal amidation
<400> 69
   Xaa Xaa Xaa Xaa Xaa Xaa Xaa
   1 5
<210> 70
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), 6)
   <223> Xaa = D-Ala
<220>
   <221> VARIANT
   <222> (3), (4), (5)
   <223> Xaa = D-Arg
<220>
   <221> VARIANT
   <222> (7)
   <223> Xaa = D-Lys
<220>
   <221> MOD_RES
   <222> (7)
   <223> C-terminal amidation
<400> 70
   Xaa Xaa Xaa Xaa Xaa Xaa Xaa
   1 5
<210> 71
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Glu
<220>
   <221> VARIANT
   <222> (3), (4), (5), (7)
   <223> Xaa = D-Arg
<220>
   <221> VARIANT
   <222> (6)
   <223> Xaa = D-Ala
<220>
   <221> MOD_RES
   <222> (7)
   <223> C-terminal amidation
<400> 71
   Xaa Xaa Xaa Xaa Xaa Xaa Xaa
    1               5
<210> 72
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (6)
   <223> Xaa = D-Ala
<220>
   <221> VARIANT
   <222> (3)
   <223> Xaa = D-Lys
<220>
   <221> VARIANT
   <222> (4), (5), (7)
   <223> Xaa = D-Arg
<220>
   <221> MOD_RES
   <222> (7)
   <223> C-terminal amidation
<400> 72
   Xaa Xaa Xaa Xaa Xaa Xaa Xaa
   1               5
<210> 73
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (6)
   <223> Xaa = D-Ala
<220>
   <221> VARIANT
   <222> (4)
   <223> Xaa = D-Lys
<220>
   <221> VARIANT
   <222> (3), (5), (7)
   <223> Xaa = D-Arg
<220>
   <221> MOD_RES
   <222> (7)
   <223> C-terminal amidation
<400> 73
   Xaa Xaa Xaa Xaa Xaa Xaa Xaa
   1               5
<210> 74
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (6)
   <223> Xaa = D-Ala
<220>
   <221> VARIANT
   <222> (3), (4), (5), (7)
   <223> Xaa = D-Arg
<220>
   <221> MOD_RES
   <222> (7)
   <223> C-terminal amidation
<400> 74
   Xaa Xaa Xaa Xaa Xaa Xaa Xaa
   1               5
<210> 75
   <211> 7
   <212> PRT
   <213> Artificial Sequence
<220>
   <223> Synthetic peptide
<220>
   <221> MOD_RES
   <222> (1)..(1)
   <223> N-terminal acetylation
<220>
   <221> MOD_RES
   <222> (7)
   <223> C-terminal amidation
<400> 75
   Cys Ala Arg Arg Arg Ala Arg
   1 5
<210> 76
   <211> 9
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3)
   <223> Xaa = D-Ala
<220>
   <221> VARIANT
   <222> (4)...(9)
   <223> Xaa = D-Arg
<220>
   <221> MOD_RES
   <222> (9)
   <223> C-terminal amidation
<400> 76
   Xaa Xaa Xaa Xaa Xaa Xaa Xaa Xaa Xaa
   1 5
<210> 77
   <211> 10
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (4)
   <223> Xaa = D-Ala
<220>
   <221> VARIANT
   <222> (5)...(10)
   <223> Xaa = D-Arg
<220>
   <221> MOD_RES
   <222> (10)
   <223> C-terminal amidation
<400> 77
   Xaa Xaa Xaa Xaa Xaa Xaa Xaa Xaa Xaa Xaa
    1               5                   10
<210> 78
   <211> 9
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (4), (6), (8)
   <223> Xaa = D-Ala
<220>
   <221> VARIANT
   <222> (3), (5), (7), (9)
   <223> Xaa = D-Arg
<220>
   <221> MOD_RES
   <222> (9)
   <223> C-terminal amidation
<400> 78
   Xaa Xaa Xaa Xaa Xaa Xaa Xaa Xaa Xaa
    1               5
<210> 79
   <211> 9
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (6), (8)
   <223> Xaa = D-Ala
<220>
   <221> VARIANT
   <222> (3), (4), (5), (7), (9)
   <223> Xaa = D-Arg
<220>
   <221> MOD_RES
   <222> (9)
   <223> C-terminal amidation
<400> 79
   Xaa Xaa Xaa Xaa Xaa Xaa Xaa Xaa Xaa
   1               5
<210> 80
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> VARIANT
   <222> (2)...(7)
   <223> Xaa = D-Arg
<220>
   <221> MOD_RES
   <222> (7)
   <223> C-terminal amidation
<400> 80
   Xaa Xaa Xaa Xaa Xaa Xaa Xaa
   1               5
<210> 81
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Cys-Cys interchain bond with benzoyl
<220>
   <221> VARIANT
   <222> (2)...(7)
   <223> Xaa = D-Arg
<220>
   <221> MOD_RES
   <222> (7)
   <223> C-terminal amidation
<400> 81
   Xaa Xaa Xaa Xaa Xaa Xaa Xaa
   1               5
<210> 82
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Cys-Cys interchain bond with acetyl
<220>
   <221> VARIANT
   <222> (2)...(7)
   <223> Xaa = D-Arg
<220>
   <221> MOD_RES
   <222> (7)
   <223> C-terminal amidation
<400> 82
   Xaa Xaa Xaa Xaa Xaa Xaa Xaa
   1               5
<210> 83
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Ala
<220>
   <221> VARIANT
   <222> (3), (4), (5), (7)
   <223> Xaa = D-Arg
<220>
   <221> VARIANT
   <222> (6)
   <223> Xaa = D-Phe
<220>
   <221> MOD_RES
   <222> (7)
   <223> C-terminal amidation
<400> 83
   Xaa Xaa Xaa Xaa Xaa Xaa Xaa
   1               5
<210> 84
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Ala
<220>
   <221> VARIANT
   <222> (3), (4), (5), (7)
   <223> Xaa = D-Arg
<220>
   <221> VARIANT
   <222> (6)
   <223> Xaa = D-Ile
<220>
   <221> MOD_RES
   <222> (7)
   <223> C-terminal amidation
<400> 84
   Xaa Xaa Xaa Xaa Xaa Xaa Xaa
   1               5
<210> 85
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Ala
<220>
   <221> VARIANT
   <222> (3), (4), (5), (7)
   <223> Xaa = D-Arg
<220>
   <221> VARIANT
   <222> (6)
   <223> Xaa = D-Leu
<220>
   <221> MOD_RES
   <222> (7)
   <223> C-terminal amidation
<400> 85
   Xaa Xaa Xaa Xaa Xaa Xaa Xaa
   1               5
<210> 86
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (6)
   <223> Xaa = D-Ala
<220>
   <221> VARIANT
   <222> (3), (4), (7)
   <223> Xaa = D-Arg
<220>
   <221> VARIANT
   <222> (5)
   <223> Xaa = D-Lys
<220>
   <221> MOD_RES
   <222> (7)
   <223> C-terminal amidation
<400> 86
   Xaa Xaa Xaa Xaa Xaa Xaa Xaa
   1               5
<210> 87
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Ala
<220>
   <221> VARIANT
   <222> (3), (4), (5), (7)
   <223> Xaa = D-Arg
<220>
   <221> VARIANT
   <222> (6)
   <223> Xaa = D-Val
<220>
   <221> MOD_RES
   <222> (7)
   <223> C-terminal amidation
<400> 87
   Xaa Xaa Xaa Xaa Xaa Xaa Xaa
   1               5
<210> 88
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Ala
<220>
   <221> VARIANT
   <222> (3), (4), (5), (7)
   <223> Xaa = D-Arg
<220>
   <221> VARIANT
   <222> (6)
   <223> Xaa = D-Pro
<220>
   <221> MOD_RES
   <222> (7)
   <223> C-terminal amidation
<400> 88
   Xaa Xaa Xaa Xaa Xaa Xaa Xaa
   1               5
<210> 89
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Ala
<220>
   <221> VARIANT
   <222> (3), (4), (5), (7)
   <223> Xaa = D-Arg
<220>
   <221> VARIANT
   <222> (6)
   <223> Xaa = D-His
<220>
   <221> MOD_RES
   <222> (7)
   <223> C-terminal amidation
<400> 89
   Xaa Xaa Xaa Xaa Xaa Xaa Xaa
   1               5
<210> 90
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Ala
<220>
   <221> VARIANT
   <222> (3), (4), (5), (7)
   <223> Xaa = D-Arg
<220>
   <221> VARIANT
   <222> (6)
   <223> Xaa = D-Gln
<220>
   <221> MOD_RES
   <222> (7)
   <223> C-terminal amidation
<400> 90
   Xaa Xaa Xaa Xaa Xaa Xaa Xaa
   1               5
<210> 91
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Ala
<220>
   <221> VARIANT
   <222> (3), (4), (5), (7)
   <223> Xaa = D-Arg
<220>
   <221> VARIANT
   <222> (6)
   <223> Xaa = D-Thr
<220>
   <221> MOD_RES
   <222> (7)
   <223> C-terminal amidation
<400> 91
   Xaa Xaa Xaa Xaa Xaa Xaa Xaa
   1               5
<210> 92
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Ala
<220>
   <221> VARIANT
   <222> (3), (4), (5), (7)
   <223> Xaa = D-Arg
<220>
   <221> VARIANT
   <222> (6)
   <223> Xaa = D-Ser
<220>
   <221> MOD_RES
   <222> (7)
   <223> C-terminal amidation
<400> 92
   Xaa Xaa Xaa Xaa Xaa Xaa Xaa
   1               5
<210> 93
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Ala
<220>
   <221> VARIANT
   <222> (3), (4), (5), (7)
   <223> Xaa = D-Arg
<220>
   <221> MOD_RES
   <222> (7)
   <223> C-terminal amidation
<400> 93
   Xaa Xaa Xaa Xaa Xaa Gly Xaa
   1               5
<210> 94
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Gle
<220>
   <221> VARIANT
   <222> (3), (4), (5), (7)
   <223> Xaa = D-Arg
<220>
   <221> VARIANT
   <222> (6)
   <223> Xaa = D-Ala
<220>
   <221> MOD_RES
   <222> (7)
   <223> C-terminal amidation
<400> 94
   Xaa Xaa Xaa Xaa Xaa Xaa Xaa
   1 5
<210> 95
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (3), (4), (5), (7)
   <223> Xaa = D-Arg
<220>
   <221> VARIANT
   <222> (6)
   <223> Xaa = D-Ala
<220>
   <221> MOD_RES
   <222> (7)
   <223> C-terminal amidation
<400> 95
   Xaa Gly Xaa Xaa Xaa Xaa Xaa
   1 5
<210> 96
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Ile
<220>
   <221> VARIANT
   <222> (3), (4), (5), (7)
   <223> Xaa = D-Arg
<220>
   <221> VARIANT
   <222> (6)
   <223> Xaa = D-Ala
<220>
   <221> MOD_RES
   <222> (7)
   <223> C-terminal amidation
<400> 96
   Xaa Xaa Xaa Xaa Xaa Xaa Xaa
   1               5
<210> 97
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Pro
<220>
   <221> VARIANT
   <222> (3), (4), (5), (7)
   <223> Xaa = D-Arg
<220>
   <221> VARIANT
   <222> (6)
   <223> Xaa = D-Ala
<220>
   <221> MOD_RES
   <222> (7)
   <223> C-terminal amidation
<400> 97
   Xaa Xaa Xaa Xaa Xaa Xaa Xaa
   1               5
<210> 98
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Leu
<220>
   <221> VARIANT
   <222> (3), (4), (5), (7)
   <223> Xaa = D-Arg
<220>
   <221> VARIANT
   <222> (6)
   <223> Xaa = D-Ala
<220>
   <221> MOD_RES
   <222> (7)
   <223> C-terminal amidation
<400> 98
   Xaa Xaa Xaa Xaa Xaa Xaa Xaa
   1               5
<210> 99
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Gln
<220>
   <221> VARIANT
   <222> (3), (4), (5), (7)
   <223> Xaa = D-Arg
<220>
   <221> VARIANT
   <222> (6)
   <223> Xaa = D-Ala
<220>
   <221> MOD_RES
   <222> (7)
   <223> C-terminal amidation
<400> 99
   Xaa Xaa Xaa Xaa Xaa Xaa Xaa
   1               5
<210> 100
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Thr
<220>
   <221> VARIANT
   <222> (3), (4), (5), (7)
   <223> Xaa = D-Arg
<220>
   <221> VARIANT
   <222> (6)
   <223> Xaa = D-Ala
<220>
   <221> MOD_RES
   <222> (7)
   <223> C-terminal amidation
<400> 100
   Xaa Xaa Xaa Xaa Xaa Xaa Xaa
   1 5
<210> 101
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Val
<220>
   <221> VARIANT
   <222> (3), (4), (5), (7)
   <223> Xaa = D-Arg
<220>
   <221> VARIANT
   <222> (6)
   <223> Xaa = D-Ala
<220>
   <221> MOD_RES
   <222> (7)
   <223> C-terminal amidation
<400> 101
   Xaa Xaa Xaa Xaa Xaa Xaa Xaa
   1               5
<210> 102
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Ser
<220>
   <221> VARIANT
   <222> (3), (4), (5), (7)
   <223> Xaa = D-Arg
<220>
   <221> VARIANT
   <222> (6)
   <223> Xaa = D-Ala
<220>
   <221> MOD_RES
   <222> (7)
   <223> C-terminal amidation
<400> 102
   Xaa Xaa Xaa Xaa Xaa Xaa Xaa
   1 5
<210> 103
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-His
<220>
   <221> VARIANT
   <222> (3), (4), (5), (7)
   <223> Xaa = D-Arg
<220>
   <221> VARIANT
   <222> (6)
   <223> Xaa = D-Ala
<220>
   <221> MOD_RES
   <222> (7)
   <223> C-terminal amidation
<400> 103
   Xaa Xaa Xaa Xaa Xaa Xaa Xaa
   1 5
<210> 104
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Phe
<220>
   <221> VARIANT
   <222> (3), (4), (5), (7)
   <223> Xaa = D-Arg
<220>
   <221> VARIANT
   <222> (6)
   <223> Xaa = D-Ala
<220>
   <221> MOD_RES
   <222> (7)
   <223> C-terminal amidation
<400> 104
   Xaa Xaa Xaa Xaa Xaa Xaa Xaa
   1               5
<210> 105
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (6)
   <223> Xaa = D-Ala
<220>
   <221> MOD_RES
   <222> (7)
   <223> C-terminal amidation
<400> 105
   Xaa Xaa Xaa Gly Xaa Xaa Xaa
   1               5
<210> 106
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4)
   <223> Xaa = D-Pro
<220>
   <221> VARIANT
   <222> (6)
   <223> Xaa = D-Ala
<220>
   <221> MOD_RES
   <222> (7)
   <223> C-terminal amidation
<400> 106
   Xaa Xaa Xaa Xaa Xaa Xaa Xaa
   1               5
<210> 107
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4)
   <223> Xaa = D-Glu
<220>
   <221> VARIANT
   <222> (6)
   <223> Xaa = D-Ala
<220>
   <221> MOD_RES
   <222> (7)
   <223> C-terminal amidation
<400> 107
   Xaa Xaa Xaa Xaa Xaa Xaa Xaa
   1               5
<210> 108
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4)
   <223> Xaa = D-Thr
<220>
   <221> VARIANT
   <222> (6)
   <223> Xaa = D-Ala
<220>
   <221> MOD_RES
   <222> (7)
   <223> C-terminal amidation
<400> 108
   Xaa Xaa Xaa Xaa Xaa Xaa Xaa
    1               5
<210> 109
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4)
   <223> Xaa = D-His
<220>
   <221> VARIANT
   <222> (6)
   <223> Xaa = D-Ala
<220>
   <221> MOD_RES
   <222> (7)
   <223> C-terminal amidation
<400> 109
   Xaa Xaa Xaa Xaa Xaa Xaa Xaa
   1 5
<210> 110
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4)
   <223> Xaa = D-Phe
<220>
   <221> VARIANT
   <222> (6)
   <223> Xaa = D-Ala
<220>
   <221> MOD_RES
   <222> (7)
   <223> C-terminal amidation
<400> 110
   Xaa Xaa Xaa Xaa Xaa Xaa Xaa
   1 5
<210> 111
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4)
   <223> Xaa = D-Ser
<220>
   <221> VARIANT
   <222> (6)
   <223> Xaa = D-Ala
<220>
   <221> MOD_RES
   <222> (7)
   <223> C-terminal amidation
<400> 111
   Xaa Xaa Xaa Xaa Xaa Xaa Xaa
    1               5
<210> 112
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4)
   <223> Xaa = D-Gln
<220>
   <221> VARIANT
   <222> (6)
   <223> Xaa = D-Ala
<220>
   <221> MOD_RES
   <222> (7)
   <223> C-terminal amidation
<400> 112
   Xaa Xaa Xaa Xaa Xaa Xaa Xaa
   1               5
<210> 113
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4)
   <223> Xaa = D-Val
<220>
   <221> VARIANT
   <222> (6)
   <223> Xaa = D-Ala
<220>
   <221> MOD_RES
   <222> (7)
   <223> C-terminal amidation
<400> 113
   Xaa Xaa Xaa Xaa Xaa Xaa Xaa
   1               5
<210> 114
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4)
   <223> Xaa = D-Leu
<220>
   <221> VARIANT
   <222> (6)
   <223> Xaa = D-Ala
<220>
   <221> MOD_RES
   <222> (7)
   <223> C-terminal amidation
<400> 114
   Xaa Xaa Xaa Xaa Xaa Xaa Xaa
   1               5
<210> 115
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4)
   <223> Xaa = D-Ile
<220>
   <221> VARIANT
   <222> (6)
   <223> Xaa = D-Ala
<220>
   <221> MOD_RES
   <222> (7)
   <223> C-terminal amidation
<400> 115
   Xaa Xaa Xaa Xaa Xaa Xaa Xaa
   1               5
<210> 116
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4)
   <223> Xaa = N-methylglycine (sarcosine)
<220>
   <221> VARIANT
   <222> (6)
   <223> Xaa = D-Ala
<220>
   <221> MOD_RES
   <222> (7)
   <223> C-terminal amidation
<400> 116
   Xaa Xaa Xaa Xaa Xaa Xaa Xaa
   1               5
<210> 117
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Ala
<220>
   <221> VARIANT
   <222> (3), (4), (5), (7)
   <223> Xaa = D-Arg
<220>
   <221> VARIANT
   <222> (6)
   <223> Xaa = N-methylglycine (sarcosine)
<220>
   <221> MOD_RES
   <222> (7)
   <223> C-terminal amidation
<400> 117
   Xaa Xaa Xaa Xaa Xaa Xaa Xaa
   1 5
<210> 118
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (6)
   <223> Xaa = N-methylalanine (Nma)
<220>
   <221> VARIANT
   <222> (3), (4), (5), (7)
   <223> Xaa = D-Arg
<220>
   <221> MOD_RES
   <222> (7)
   <223> C-terminal amidation
<400> 118
   Xaa Xaa Xaa Xaa Xaa Xaa Xaa
   1 5
<210> 119
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4)
   <223> Xaa = D-Ala
<220>
   <221> VARIANT
   <222> (6)
   <223> Xaa = N-methylalanine (Nma)
<220>
   <221> MOD_RES
   <222> (7)
   <223> C-terminal amidation
<400> 119
   Xaa Xaa Xaa Xaa Xaa Xaa Xaa
   1               5
<210> 120
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (6)
   <223> Xaa = 2-aminoisobutyric acid (Aib)
<220>
   <221> VARIANT
   <222> (3), (4), (5), (7)
   <223> Xaa = D-Arg
<220>
   <221> MOD_RES
   <222> (7)
   <223> C-terminal amidation
<400> 120
   Xaa Xaa Xaa Xaa Xaa Xaa Xaa
   1 5
<210> 121
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4)
   <223> Xaa = N-methylalanine (Nma)
<220>
   <221> VARIANT
   <222> (6)
   <223> Xaa = D-Ala
<220>
   <221> MOD_RES
   <222> (7)
   <223> C-terminal amidation
<400> 121
   Xaa Xaa Xaa Xaa Xaa Xaa Xaa
   1 5
<210> 122
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Ala
<220>
   <221> VARIANT
   <222> (3), (4), (5), (7)
   <223> Xaa = D-Arg
<220>
   <221> VARIANT
   <222> (6)
   <223> Xaa = N-methylalanine (Nma)
<220>
   <221> MOD_RES
   <222> (7)
   <223> C-terminal amidation
<400> 122
   Xaa Xaa Xaa Xaa Xaa Xaa Xaa
   1               5
<210> 123
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = 2-aminoisobutyric acid (Aib)
<220>
   <221> VARIANT
   <222> (3), (4), (5), (7)
   <223> Xaa = D-Arg
<220>
   <221> VARIANT
   <222> (6)
   <223> Xaa = D-Ala
<220>
   <221> MOD_RES
   <222> (7)
   <223> C-terminal amidation
<400> 123
   Xaa Xaa Xaa Xaa Xaa Xaa Xaa
   1               5
<210> 124
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Ala
<220>
   <221> VARIANT
   <222> (3), (4), (5), (7)
   <223> Xaa = D-Arg
<220>
   <221> VARIANT
   <222> (6)
   <223> Xaa = 2-aminoisobutyric acid (Aib)
<220>
   <221> MOD_RES
   <222> (7)
   <223> C-terminal amidation
<400> 124
   Xaa Xaa Xaa Xaa Xaa Xaa Xaa
   1 5
<210> 125
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (6)
   <223> Xaa = N-methylglycine (sarcosine)
<220>
   <221> VARIANT
   <222> (3), (4), (5), (7)
   <223> Xaa = D-Arg
<220>
   <221> MOD_RES
   <222> (7)
   <223> C-terminal amidation
<400> 125
   Xaa Xaa Xaa Xaa Xaa Xaa Xaa
   1 5
<210> 126 <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4)
   <223> Xaa = D-Ala
<220>
   <221> VARIANT
   <222> (6)
   <223> Xaa = N-methylglycine (sarcosine)
<220>
   <221> MOD_RES
   <222> (7)
   <223> C-terminal amidation
<400> 126
   Xaa Xaa Xaa Xaa Xaa Xaa Xaa
   1               5
<210> 127
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = N-methylalanine (Nma)
<220>
   <221> VARIANT
   <222> (3), (4), (5), (7)
   <223> Xaa = D-Arg
<220>
   <221> VARIANT
   <222> (6)
   <223> Xaa = D-Ala
<220>
   <221> MOD_RES
   <222> (7)
   <223> C-terminal amidation
<400> 127
   Xaa Xaa Xaa Xaa Xaa Xaa Xaa
   1 5
<210> 128
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = N-methylglycine (sarcosine)
<220>
   <221> VARIANT
   <222> (3), (4), (5), (7)
   <223> Xaa = D-Arg
<220>
   <221> VARIANT
   <222> (6)
   <223> Xaa = D-Ala
<220>
   <221> MOD_RES
   <222> (7)
   <223> C-terminal amidation
<400> 128
   Xaa Xaa Xaa Xaa Xaa Xaa Xaa
   1 5
<210> 129
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Ala
<220>
   <221> VARIANT
   <222> (3), (4), (5), (7)
   <223> Xaa = D-Arg
<220>
   <221> VARIANT
   <222> (6)
   <223> Xaa = Norleucine (NLeu)
<220>
   <221> MOD_RES
   <222> (7)
   <223> C-terminal amidation
<400> 129
   Xaa Xaa Xaa Xaa Xaa Xaa Xaa
   1               5
<210> 130
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (6)
   <223> Xaa = D-Norleucine (dNLeu)
<220>
   <221> VARIANT
   <222> (3), (4), (5), (7)
   <223> Xaa = D-Arg
<220>
   <221> MOD_RES
   <222> (7)
   <223> C-terminal amidation
<400> 130
   Xaa Xaa Xaa Xaa Xaa Xaa Xaa
   1               5
<210> 131
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Ala
<220>
   <221> VARIANT
   <222> (3), (4), (5), (7)
   <223> Xaa = D-Arg
<220>
   <221> VARIANT
   <222> (6)
   <223> Xaa = D-Norvaline (dNval)
<220>
   <221> MOD_RES
   <222> (7)
   <223> C-terminal amidation
<400> 131
   Xaa Xaa Xaa Xaa Xaa Xaa Xaa
   1 5
<210> 132
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (6)
   <223> Xaa = D-Norvaline (dNval)
<220>
   <221> VARIANT
   <222> (3), (4), (5), (7)
   <223> Xaa = D-Arg
<220>
   <221> MOD_RES
   <222> (7)
   <223> C-terminal amidation
<400> 132
   Xaa Xaa Xaa Xaa Xaa Xaa Xaa
   1 5
<210> 133
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (4)
   <223> Xaa = D-Ala
<220>
   <221> VARIANT
   <222> (2), (3), (5), (7)
   <223> Xaa = D-Arg
<220>
   <221> VARIANT
   <222> (6)
   <223> Xaa = D-Norleucine (dNLeu)
<220>
   <221> MOD_RES
   <222> (7)
   <223> C-terminal amidation
<400> 133
   Xaa Xaa Xaa Xaa Xaa Xaa Xaa
   1               5
<210> 134
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Norleucine (dNLeu)
<220>
   <221> VARIANT
   <222> (3), (4), (5), (7)
   <223> Xaa = D-Arg
<220>
   <221> VARIANT
   <222> (6)
   <223> Xaa = D-Ala
<220>
   <221> MOD_RES
   <222> (7)
   <223> C-terminal amidation
<400> 134
   Xaa Xaa Xaa Xaa Xaa Xaa Xaa
   1 5
<210> 135
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4)
   <223> Xaa = D-Ala
<220>
   <221> VARIANT
   <222> (6)
   <223> Xaa = D-Norvaline (dNVal)
<220>
   <221> MOD_RES
   <222> (7)
   <223> C-terminal amidation
<400> 135
   Xaa Xaa Xaa Xaa Xaa Xaa Xaa
   1 5
<210> 136
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)
   <223> Xaa = D-Norvaline (dNVal)
<220>
   <221> VARIANT
   <222> (3), (4), (5), (7)
   <223> Xaa = D-Arg
<220>
   <221> VARIANT
   <222> (6)
   <223> Xaa = D-Ala
<220>
   <221> MOD_RES
   <222> (7)
   <223> C-terminal amidation
<400> 136
   Xaa Xaa Xaa Xaa Xaa Xaa Xaa
    1               5
<210> 137
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4)
   <223> Xaa = D-Norvaline (dNVal)
<220>
   <221> VARIANT
   <222> (6)
   <223> Xaa = D-Ala
<220>
   <221> MOD_RES
   <222> (7)
   <223> C-terminal amidation
<400> 137
   Xaa Xaa Xaa Xaa Xaa Xaa Xaa
    1               5
<210> 138
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4)
   <223> Xaa = D-Norleucine (dNLeu)
<220>
   <221> VARIANT
   <222> (6)
   <223> Xaa = D-Ala
<220>
   <221> MOD_RES
   <222> (7)
   <223> C-terminal amidation
<400> 138
   Xaa Xaa Xaa Xaa Xaa Xaa Xaa
   1 5
<210> 139
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Bond with D-homocysteine (dHcy)
<220>
   <221> VARIANT
   <222> (2), (6)
   <223> Xaa = D-Ala
<220>
   <221> VARIANT
   <222> (3), (4), (5), (7)
   <223> Xaa = D-Arg
<220>
   <221> MOD_RES
   <222> (7)
   <223> C-terminal amidation
<400> 139
   Xaa Xaa Xaa Xaa Xaa Xaa Xaa
   1 5
<210> 140
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Bond with 3-mercaptopropionic acid (Mpa)
<220>
   <221> VARIANT
   <222> (2), (6)
   <223> Xaa = D-Ala
<220>
   <221> VARIANT
   <222> (3), (4), (5), (7)
   <223> Xaa = D-Arg
<220>
   <221> MOD_RES
   <222> (7)
   <223> C-terminal amidation
<400> 140
   Xaa Xaa Xaa Xaa Xaa Xaa Xaa
   1               5
<210> 141
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Bond with acetylated L-Cysteine
<220>
   <221> VARIANT
   <222> (2), (6)
   <223> Xaa = D-Ala
<220>
   <221> VARIANT
   <222> (3), (4), (5), (7)
   <223> Xaa = D-Arg
<220>
   <221> MOD_RES
   <222> (7)
   <223> C-terminal amidation
<400> 141
   Xaa Xaa Xaa Xaa Xaa Xaa Xaa
   1               5
<210> 142
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Bond with D-Cysteine
<220>
   <221> VARIANT
   <222> (2), (6)
   <223> Xaa = D-Ala
<220>
   <221> VARIANT
   <222> (3), (4), (5), (7)
   <223> Xaa = D-Arg
<220>
   <221> MOD_RES
   <222> (7)
   <223> C-terminal amidation
<400> 142
   Xaa Xaa Xaa Xaa Xaa Xaa Xaa
   1               5
<210> 143
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Bond with Polyethylene glycol
<220>
   <221> VARIANT
   <222> (2)...(7)
   <223> Xaa = D-Arg
<220>
   <221> MOD_RES
   <222> (7)
   <223> C-terminal amidation
<400> 143
   Xaa Xaa Xaa Xaa Xaa Xaa Xaa
   1               5
<210> 144
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Bond with Polyethylene glycol
<220>
   <221> VARIANT
   <222> (2)...(7)
   <223> Xaa = D-Arg
<220>
   <221> MOD_RES
   <222> (7)
   <223> C-terminal amidation
<400> 144
   Xaa Xaa Xaa Xaa Xaa Xaa Xaa
   1 5
<210> 145
   <211> 7
   <212> PRT
   <213> Artificial Sequence
<220>
   <223> Synthetic peptide
<400> 145
   Cys Glu Glu Glu Glu Glu Glu
   1 5
<210> 146
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2)...(5)
   <223> Xaa = D-Arg
<220>
   <221> VARIANT
   <222> (6), (7)
   <223> Xaa = D-Ala
<220>
   <221> MOD_RES
   <222> (7)
   <223> C-terminal amidation
<400> 146
   Xaa Xaa Xaa Xaa Xaa Xaa Xaa
   1               5
<210> 147
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (6)
   <223> Xaa = D-Ala
<220>
   <221> VARIANT
   <222> (3), (4), (5), (7)
   <223> Xaa = D-Lys
<220>
   <221> MOD_RES
   <222> (7)
   <223> C-terminal amidation
<400> 147
   Xaa Xaa Xaa Xaa Xaa Xaa Xaa
   1 5
<210> 148
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (4), (6)
   <223> Xaa = D-Ala
<220>
   <221> VARIANT
   <222> (3), (5), (7)
   <223> Xaa = D-Arg
<220>
   <221> MOD_RES
   <222> (7)
   <223> C-terminal amidation
<400> 148
   Xaa Xaa Xaa Xaa Xaa Xaa Xaa
   1 5
<210> 149
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4)
   <223> Xaa = D-Ala
<220>
   <221> MOD_RES
   <222> (7)
   <223> C-terminal amidation
<400> 149
   Xaa Xaa Xaa Xaa Xaa Gly Xaa
   1               5
<210> 150
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4)
   <223> Xaa = D-Ala
<220>
   <221> VARIANT
   <222> (6)
   <223> Xaa = D-Gln
<220>
   <221> MOD_RES
   <222> (7)
   <223> C-terminal amidation
<400> 150
   Xaa Xaa Xaa Xaa Xaa Xaa Xaa
   1 5
<210> 151
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4)
   <223> Xaa = D-Ala
<220>
   <221> VARIANT
   <222> (6)
   <223> Xaa = D-His
<220>
   <221> MOD_RES
   <222> (7)
   <223> C-terminal amidation
<400> 151
   Xaa Xaa Xaa Xaa Xaa Xaa Xaa
   1 5
<210> 152
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (3), (5), (7)
   <223> Xaa = D-Arg
<220>
   <221> VARIANT
   <222> (4)
   <223> Xaa = D-Ala
<220>
   <221> VARIANT
   <222> (6)
   <223> Xaa = D-Ile
<220>
   <221> MOD_RES
   <222> (7)
   <223> C-terminal amidation
<400> 152
   Xaa Xaa Xaa Xaa Xaa Xaa Xaa
   1               5
<210> 153
   <211> 6
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (5)
   <223> Xaa = D-Ala
<220>
   <221> DISULFID
   <222> (2)..(2)
   <223> Bond with 1,3-diaminopropionic acid (DAP)
<220>
   <221> VARIANT
   <222> (3), (4), (6)
   <223> Xaa = D-Arg
<220>
   <221> MOD_RES
   <222> (6)
   <223> C-terminal amidation
<400> 153
   Xaa Xaa Xaa Xaa Xaa Xaa
   1               5
<210> 154
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> VARIANT
   <222> (2), (6)
   <223> Xaa = D-Ala
<220>
   <221> VARIANT
   <222> (3), (4), (5)
   <223> Xaa = D-HOmoarginine
<220>
   <221> VARIANT
   <222> (7)
   <223> Xaa = D-Arg
<220>
   <221> MOD_RES
   <222> (7)
   <223> C-terminal amidation
<400> 154
   Xaa Xaa Xaa Xaa Xaa Xaa Xaa
   1 5
<210> 155
   <211> 7
   <212> PRT
   <213> Artificial Sequence
<220>
   <223> Synthetic peptide
<220>
   <221> VARIANT
   <222> (1)
   <223> Xaa = a thiol-containing amino acid residue
<220>
   <221> VARIANT
   <222> (2)
   <223> Xaa = a non-cationic amino acid residue
<220>
   <221> VARIANT
   <222> (3), (4), (7)
   <223> Xaa = any amino acid residue
<220>
   <221> VARIANT
   <222> (5)
   <223> Xaa = a cationic amino acid residue
<220>
   <221> VARIANT
   <222> (6)
   <223> Xaa = a non-cationic amino acid residue
<400> 155
   Xaa Xaa Xaa Xaa Xaa Xaa Xaa
   1 5
<210> 156
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> VARIANT
   <222> (2)
   <223> Xaa = a non-cationic amino acid residue
<220>
   <221> VARIANT
   <222> (3), (4), (7)
   <223> Xaa = any amino acid residue
<220>
   <221> VARIANT
   <222> (5)
   <223> Xaa = D-Arg, L-Arg, D-Lys or L-Lys
<220>
   <221> VARIANT
   <222> (6)
   <223> Xaa = a non-cationic amino acid residue
<400> 156
   Xaa Xaa Xaa Xaa Xaa Xaa Xaa
   1               5
<210> 157
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> VARIANT
   <222> (2), (3), (4)
   <223> Xaa = any amino acid residue
<220>
   <221> VARIANT
   <222> (5), (7)
   <223> Xaa = any cationic amino acid residue
<220>
   <221> VARIANT
   <222> (6)
   <223> Xaa = a non-cationic amino acid residue
<400> 157
   Xaa Xaa Xaa Xaa Xaa Xaa Xaa
   1               5
<210> 158
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> VARIANT
   <222> (2), (6)
   <223> Xaa = a non-cationic amino acid residue
<220>
   <221> VARIANT
   <222> (3), (4), (7)
   <223> Xaa = any amino acid residue
<220>
   <221> VARIANT
   <222> (5)
   <223> Xaa = D-Arg, L-Arg, D-Lys or L-Lys
<400> 158
   Xaa Xaa Xaa Xaa Xaa Xaa Xaa
   1               5
<210> 159
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> VARIANT
   <222> (2)
   <223> Xaa = D-Ala
<220>
   <221> VARIANT
   <222> (3), (4), (7)
   <223> Xaa = any cationic amino acid residue
<220>
   <221> VARIANT
   <222> (5)
   <223> Xaa = D-Arg
<220>
   <221> VARIANT
   <222> (6)
   <223> Xaa = D-Ala
<400> 159
   Xaa Xaa Xaa Xaa Xaa Xaa Xaa
   1 5
<210> 160
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> VARIANT
   <222> (2), (3), (7)
   <223> Xaa = any cationic amino acid residue
<220>
   <221> VARIANT
   <222> (4)
   <223> Xaa = D-Ala
<220>
   <221> VARIANT
   <222> (5)
   <223> Xaa = D-Arg
<220>
   <221> VARIANT
   <222> (6)
   <223> Xaa = D-Ala
<400> 160
   Xaa Xaa Xaa Xaa Xaa Xaa Xaa
   1 5
<210> 161
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> VARIANT
   <222> (2)
   <223> Xaa = D-Ala
<220>
   <221> VARIANT
   <222> (3), (4), (7)
   <223> Xaa = any amino acid
<220>
   <221> VARIANT
   <222> (5)
   <223> Xaa = D-Arg
<220>
   <221> VARIANT
   <222> (6)
   <223> Xaa = D-Ala
<400> 161
   Xaa Xaa Xaa Xaa Xaa Xaa Xaa
   1               5
<210> 162
   <211> 4
   <212> PRT
   <213> Artificial Sequence
<220>
   <223> Synthetic peptide
<400> 162
   Cys Arg Arg Arg
   1
<210> 163
   <211> 5
   <212> PRT
   <213> Artificial Sequence
<220>
   <223> Synthetic peptide
<400> 163
   Cys Arg Arg Arg Arg
   1               5
<210> 164
   <211> 8
   <212> PRT
   <213> Artificial Sequence
<220>
   <223> Synthetic peptide
<400> 164
   Cys Arg Arg Arg Arg Arg Arg Arg
   1 5
<210> 165
   <211> 9
   <212> PRT
   <213> Artificial Sequence
<220>
   <223> Synthetic peptide
<400> 165
   Cys Arg Arg Arg Arg Arg Arg Arg Arg
   1 5
<210> 166
   <211> 10
   <212> PRT
   <213> Artificial Sequence
<220>
   <223> Synthetic peptide
<400> 166
   Cys Arg Arg Arg Arg Arg Arg Arg Arg Arg
   1 5 10
<210> 167
   <211> 11
   <212> PRT
   <213> Artificial Sequence
<220>
   <223> Synthetic peptide
<400> 167
   Cys Arg Arg Arg Arg Arg Arg Arg Arg Arg Arg
   1 5 10
<210> 168
   <211> 12
   <212> PRT
   <213> Artificial Sequence
<220>
   <223> Synthetic peptide
<400> 168
   Cys Arg Arg Arg Arg Arg Arg Arg Arg Arg Arg Arg
   1 5 10
<210> 169
   <211> 13
   <212> PRT
   <213> Artificial Sequence
<220>
   <223> Synthetic peptide
<400> 169
   Cys Arg Arg Arg Arg Arg Arg Arg Arg Arg Arg Arg Arg
   1 5 10
<210> 170
   <211> 7
   <212> PRT
   <213> Artificial Sequence
<220>
   <223> Synthetic peptide
<220>
   <221> VARIANT
   <222> (1)
   <223> Xaa = D-Cys
<220>
   <221> MOD_RES
   <222> (1)..(1)
   <223> N-terminal acetylation
<220>
   <221> DISULFID
   <222> (1)..(1)
   <223> Cys-Cys disulfide bonded to acetylated Glutathione
<220>
   <221> VARIANT
   <222> (4), (6)
   <223> Xaa = D-Ala
<220>
   <221> VARIANT
   <222> (2), (3), (5), (7)
   <223> Xaa = D-Arg
<220>
   <221> MOD_RES
   <222> (7)
   <223> C-terminal amidation
<400> 170
   Xaa Xaa Xaa Xaa Xaa Xaa Xaa
   1 5